(19) Europäisches Patentamt
European Patent Office
Office européen des brevets

(11) **EP 3 919 072 A1**

(12) **EUROPEAN PATENT APPLICATION**
published in accordance with Art. 153(4) EPC

(43) Date of publication:
**08.12.2021 Bulletin 2021/49**

(21) Application number: **20748243.1**

(22) Date of filing: **29.01.2020**

(51) Int Cl.:
***A61K 39/00*** *(2006.01)* ***A61K 39/39*** *(2006.01)*
***A61P 35/00*** *(2006.01)* ***A61P 37/04*** *(2006.01)*
***A61K 47/69*** *(2017.01)*

(86) International application number:
**PCT/JP2020/003076**

(87) International publication number:
**WO 2020/158771 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2019 JP 2019013556**

(71) Applicants:
- **Mie University**
  **Tsu-shi, Mie 514-8507 (JP)**
- **Kyoto University**
  **Kyoto-shi, Kyoto 606-8501 (JP)**
- **CHUGAI SEIYAKU KABUSHIKI KAISHA**
  **Tokyo 115-8543 (JP)**

(72) Inventors:
- **SHIKU, Hiroshi**
  **Tsu-shi, Mie 514-8507 (JP)**
- **AKIYOSHI, Kazunari**
  **Kyoto-shi, Kyoto 606-8501 (JP)**
- **HIRAKURA, Tai**
  **Gotemba-shi, Shizuoka 412-8513 (JP)**
- **SHIMOBOJI, Tsuyoshi**
  **Gotemba-shi, Shizuoka 412-8513 (JP)**
- **NAKAI, Takashi**
  **Gotemba-shi, Shizuoka 412-8513 (JP)**
- **CHUANOI, Sayan**
  **Gotemba-shi, Shizuoka 412-8513 (JP)**
- **TOGAWA, Hideyuki**
  **Gotemba-shi, Shizuoka 412-8513 (JP)**

(74) Representative: **Vossius & Partner Patentanwälte Rechtsanwälte mbB**
**Siebertstraße 3**
**81675 München (DE)**

(54) **CANCER VACCINE PREPARATION**

(57) The present invention provides a vaccine formulation for use in the prevention and/or treatment of a cancer, comprising a complex of a hyaluronic acid derivative having an introduced hydrophobic group, and an antigen.

Figure 12-1

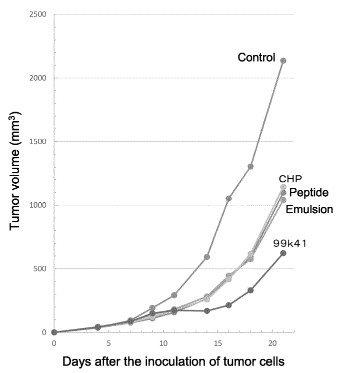


EP 3 919 072 A1

SEQUENCE LISTING

<110> MIE UNIVERSITY
KYOTO UNIVERSITY
CHUGAI SEIYAKU KABUSHIKI KAISHA

<120> Cancer vaccine formulations

<130> FA0001-19324

<150> JP 2019-013556
<151> 2019-01-29

<160> 23

<170> PatentIn version 3.5

<210> 1
<211> 37
<212> PRT
<213> Artificial Sequence

<220>
<223> mERK2 p121

<400> 1

```
Asn Asp His Ile Ala Tyr Phe Leu Tyr Gln Ile Leu Arg Gly Leu Gln
1               5                   10                  15


Tyr Ile His Ser Ala Asn Val Leu His Arg Asp Leu Lys Pro Ser Asn
            20                  25                  30


Leu Leu Leu Asn Thr
        35
```

<210> 2
<211> 9
<212> PRT
<213> Artificial Sequence

<220>
<223> mERK2 p121 killer T cell epitope

<400> 2

```
Gln Tyr Ile His Ser Ala Asn Val Leu
1               5
```

<210> 3
<211> 17
<212> PRT
<213> Artificial Sequence

<220>
<223> mERK2 p121 helper T cell epitope

<400> 3

```
Arg Gly Leu Gln Tyr Ile His Ser Ala Asn Val Leu His Arg Asp Leu
1               5                   10                  15

Lys


<210>  4
<211>  40
<212>  PRT
<213>  Artificial Sequence

<220>
<223>  MAGE-A4 p264

<400>  4

Gly Ser Asn Pro Ala Arg Tyr Glu Phe Leu Trp Gly Pro Arg Ala Leu
1               5                   10                  15

Ala Glu Thr Ser Tyr Val Lys Val Leu Glu His Val Val Arg Val Asn
            20                  25                  30

Ala Arg Val Arg Ile Ala Tyr Pro
        35                  40


<210>  5
<211>  9
<212>  PRT
<213>  Artificial Sequence

<220>
<223>  MAGE-A4 p264 killer T cell epitope

<400>  5

Ser Asn Pro Ala Arg Tyr Glu Phe Leu
1               5


<210>  6
<211>  16
<212>  PRT
<213>  Artificial Sequence

<220>
<223>  MAGE-A4 p264 helper T cell epitope

<400>  6

Val Lys Val Leu Glu His Val Val Arg Val Asn Ala Arg Val Arg Ile
1               5                   10                  15


<210>  7
<211>  9
<212>  PRT
<213>  Artificial Sequence
```

<220>
<223> MAGE-A4 p265

<400> 7

Ser Asn Pro Ala Arg Tyr Glu Phe Leu
1               5

<210> 8
<211> 19
<212> PRT
<213> Artificial Sequence

<220>
<223> MAGE-A4 p285

<400> 8

Val Lys Val Leu Glu His Val Val Arg Val Asn Ala Arg Val Arg Ile
1               5                   10                  15

Ala Tyr Pro

<210> 9
<211> 38
<212> PRT
<213> Artificial Sequence

<220>
<223> TRP2TRP1gp100-6G

<400> 9

Ser Val Tyr Asp Phe Phe Val Trp Leu Gly Gly Gly Gly Gly Gly Thr
1               5                   10                  15

Trp His Arg Tyr His Leu Leu Gly Gly Gly Gly Gly Gly Glu Gly Ser
            20                  25                  30

Arg Asn Gln Asp Trp Leu
        35

<210> 10
<211> 38
<212> PRT
<213> Artificial Sequence

<220>
<223> TRP2TRP1gp100-6Y

<400> 10

Ser Val Tyr Asp Phe Phe Val Trp Leu Tyr Tyr Tyr Tyr Tyr Tyr Thr
1               5                   10                  15

Trp His Arg Tyr His Leu Leu Tyr Tyr Tyr Tyr Tyr Tyr Glu Gly Ser
20 25 30

Arg Asn Gln Asp Trp Leu
35

<210> 11
<211> 34
<212> PRT
<213> Artificial Sequence

<220>
<223> TRP2TRP1gp100-4L

<400> 11

Ser Val Tyr Asp Phe Phe Val Trp Leu Leu Leu Leu Leu Thr Trp His
1 5 10 15

Arg Tyr His Leu Leu Leu Leu Leu Leu Glu Gly Ser Arg Asn Gln Asp
20 25 30

Trp Leu

<210> 12
<211> 38
<212> PRT
<213> Artificial Sequence

<220>
<223> TRP2TRP1gp100-6L

<400> 12

Ser Val Tyr Asp Phe Phe Val Trp Leu Leu Leu Leu Leu Leu Leu Thr
1 5 10 15

Trp His Arg Tyr His Leu Leu Leu Leu Leu Leu Leu Leu Glu Gly Ser
20 25 30

Arg Asn Gln Asp Trp Leu
35

<210> 13
<211> 9
<212> PRT
<213> Artificial Sequence

<220>
<223> TRP2

<400> 13

Ser Val Tyr Asp Phe Phe Val Trp Leu

1 5

<210> 14
<211> 8
<212> PRT
<213> Artificial Sequence

<220>
<223> TRP1

<400> 14

Thr Trp His Arg Tyr His Leu Leu
1 5

<210> 15
<211> 9
<212> PRT
<213> Artificial Sequence

<220>
<223> gp100

<400> 15

Glu Gly Ser Arg Asn Gln Asp Trp Leu
1 5

<210> 16
<211> 29
<212> PRT
<213> Artificial Sequence

<220>
<223> AH1gp70-6G

<400> 16

Leu Val Gln Phe Ile Lys Asp Arg Ile Ser Val Val Gln Ala Gly Gly
1 5 10 15

Gly Gly Gly Gly Ser Pro Ser Tyr Val Tyr His Gln Phe
20 25

<210> 17
<211> 29
<212> PRT
<213> Artificial Sequence

<220>
<223> AH1gp70-6Y

<400> 17

Leu Val Gln Phe Ile Lys Asp Arg Ile Ser Val Val Gln Ala Tyr Tyr
1 5 10 15

Tyr Tyr Tyr Tyr Ser Pro Ser Tyr Val Tyr His Gln Phe
20 25

<210> 18
<211> 27
<212> PRT
<213> Artificial Sequence

<220>
<223> AH1gp70-4L

<400> 18

Leu Val Gln Phe Ile Lys Asp Arg Ile Ser Val Val Gln Ala Leu Leu
1 5 10 15

Leu Leu Ser Pro Ser Tyr Val Tyr His Gln Phe
20 25

<210> 19
<211> 29
<212> PRT
<213> Artificial Sequence

<220>
<223> AH1gp70-6L

<400> 19

Leu Val Gln Phe Ile Lys Asp Arg Ile Ser Val Val Gln Ala Leu Leu
1 5 10 15

Leu Leu Leu Leu Ser Pro Ser Tyr Val Tyr His Gln Phe
20 25

<210> 20
<211> 9
<212> PRT
<213> Artificial Sequence

<220>
<223> AH1

<400> 20

Ser Pro Ser Tyr Val Tyr His Gln Phe
1 5

<210> 21
<211> 14
<212> PRT
<213> Artificial Sequence

<220>
<223> gp70

<400> 21

Leu Val Gln Phe Ile Lys Asp Arg Ile Ser Val Val Gln Ala
1               5                   10

<210> 22
<211> 40
<212> PRT
<213> Artificial Sequence

<220>
<223> MAGE-A4 p264-4L

<400> 22

Gly Ser Asn Pro Ala Arg Tyr Glu Phe Leu Trp Gly Pro Arg Ala Leu
1               5                   10                  15

Leu Leu Leu Leu Tyr Val Lys Val Leu Glu His Val Val Arg Val Asn
            20                  25                  30

Ala Arg Val Arg Ile Ala Tyr Pro
        35                  40

<210> 23
<211> 40
<212> PRT
<213> Artificial Sequence

<220>
<223> MAGE-A4 p264-4W

<400> 23

Gly Ser Asn Pro Ala Arg Tyr Glu Phe Leu Trp Gly Pro Arg Ala Leu
1               5                   10                  15

Trp Trp Trp Trp Tyr Val Lys Val Leu Glu His Val Val Arg Val Asn
            20                  25                  30

Ala Arg Val Arg Ile Ala Tyr Pro
        35                  40

**Description**

TECHNICAL FIELD

The present invention relates to a vaccine formulation for use in the prevention and/or treatment of a cancer, comprising a complex of a hyaluronic acid derivative having an introduced hydrophobic group, and an antigen.

BACKGROUND ART

A vaccine formulation for use in the prevention and/or treatment of a cancer has been reported which comprises a hydrophobized polysaccharide such as cholesterol-bearing pullulan (CHP) or cholesterol-bearing mannan (CMP), and an antigen (Patent Literature 1). Also, a vaccine formulation for cancer treatment has been reported which comprises a complex of a hydrophobized polysaccharide such as CHP, and a synthesized long-chain peptide antigen having a plurality of T cell recognition epitopes, and an immunopotentiating agent (Patent Literatures 2 and 3).

These vaccine formulations are based on the principles that antigens phagocytized by antigen-presenting cells are cleaved into peptides of various lengths by intracellular proteasome, protease, or peptidase and loaded as antigenic epitope peptides onto major histocompatibility complex (MHC) class I molecules or MHC class II molecules on the cell surface to form complexes, which are in turn specifically recognized by cytotoxic T cells (CTL, $CD8^+$) or helper T cells ($CD4^+$) so that these cells are activated (Non Patent Literature 1).

Meanwhile, it has been reported that a hyaluronic acid derivative obtained by introducing a group having a cholesteryl group as a hydrophobic group to hyaluronic acid forms fine particles by association in water and forms a complex with a drug (Patent Literature 4). It has also been reported that a hyaluronic acid derivative obtained by converting a carboxyl group of the glucuronic acid moiety of hyaluronic acid into an amide group through reaction with a particular amino acid, and further introducing a steryl group which is a hydrophobic group to remaining carboxyl groups has both characteristics of biodegradability and retention in blood, and a complex of the hyaluronic acid derivative and a drug has characteristics favorable as a pharmaceutical composition (Patent Literature 5).

CITATION LIST

PATENT LITERATURE

[Patent Literature 1] International Publication No. WO 1998/009650
[Patent Literature 2] International Publication No. WO 2013/031882
[Patent Literature 3] International Publication No. WO 2015/050158
[Patent Literature 4] International Publication No. WO 2010/053140
[Patent Literature 5] International Publication No. WO 2014/038641

NON PATENT LITERATURE

[Non Patent Literature 1] ACS Nano Vol. 8, p. 9209-9218, 2014

SUMMARY OF INVENTION

TECHNICAL PROBLEM

There is a demand for a vaccine formulation having a higher function than that of already reported vaccine formulations containing CHP. Particularly, it is desirable to develop a cancer vaccine formulation having characteristics of markedly inducing antigen-specific cytotoxic T cells (CTL) or a high antitumor effect.

SOLUTION TO PROBLEM

The present inventor has conducted diligent studies to solve these problems and consequently completed the present invention by finding that a vaccine formulation comprising a complex of a hyaluronic acid derivative having an introduced hydrophobic group, and an antigen markedly induces antigen-specific CTL and has high antitumor activity.

Specifically, the present invention relates to a complex that is formed by enclosing an antigen through spontaneous association in an aqueous solution, improves the accumulation of the antigen to lymph node, and markedly

induces antigen-specific CTL, a complex having high antitumor activity, a vaccine formulation comprising the complex, and production methods therefor. The present invention also relates to a complex that is formed by enclosing an antigen through better dispersion in water, improves the accumulation of the antigen to lymph node, and markedly induces antigen-specific CTL, a vaccine formulation comprising the complex, and production methods therefor.

In one aspect, the present invention provides a vaccine formulation for use in the prevention and/or treatment of a cancer according to any of [1] to [13] and [15] to [27], a complex for use in the vaccine formulation according to any of [14] and [28], and a method for producing the complex according to [29] as follows.

[1] A vaccine formulation for use in the prevention or treatment of a cancer, comprising a hyaluronic acid derivative having an introduced hydrophobic group, and an antigen, wherein

the hyaluronic acid derivative having an introduced hydrophobic group is the following:
a hyaluronic acid derivative comprising at least one repeating unit represented by the formula (I):

[Formula 1]

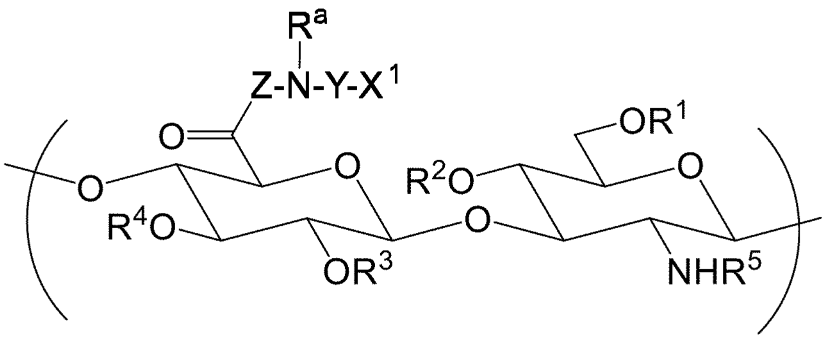

(I)

wherein $R^1$, $R^2$, $R^3$, and $R^4$ are each independently selected from a hydrogen atom, $C_{1-6}$ alkyl, formyl and $C_{1-6}$ alkylcarbonyl;

$R^5$ is a hydrogen atom, formyl, or $C_{1-6}$ alkylcarbonyl;
Z represents a direct bond, or a peptide linker having 2 to 30 arbitrary amino acid residues;
$X^1$ is a hydrophobic group selected from groups represented by the following formulas:

$-NR^b-R$,

$-NR^b-COO-R$,

$-NR^b-CO-R$,

$-NR^b-CO-NR^c-R$,

$-COO-R$,

$-O-COO-R$,

$-S-R$,

$-CO-Y^a-S-R$,

$-O-CO-Y^b-S-R$,

$-NR^b-CO-Y^b-S-R$,

and

$-S-S-R$;

$R^a$, $R^b$ and $R^c$ are each independently selected from a hydrogen atom, $C_{1-20}$ alkyl, amino-$C_{2-20}$ alkyl and hydroxy-$C_{2-20}$ alkyl, wherein in the alkyl moiety of each of the groups, 1 to 3 groups each independently selected from -O- and -$NR^f$- are optionally inserted;
$R^f$ is selected from a hydrogen atom, $C_{1-12}$ alkyl, amino-$C_{2-12}$ alkyl and hydroxy-$C_{2-12}$ alkyl, wherein in the alkyl moiety of each of the groups, 1 or 2 groups each independently selected from -O- and -NH- are optionally inserted;
R is a steryl group;
Y is $C_{2-30}$ alkylene, or $-(CH_2CH_2O)_m-CH_2CH_2-$, wherein in the alkylene, 1 to 5 groups each independently selected from -O-, -$NR^g$- and -S-S- are optionally inserted;
$R^g$ is selected from a hydrogen atom, $C_{1-20}$ alkyl, amino-$C_{2-20}$ alkyl or hydroxy-$C_{2-20}$ alkyl, wherein in the alkyl moiety of each of the groups, 1 to 3 groups each independently selected from -O- and -NH- are optionally inserted;
$Y^a$ is $C_{1-5}$ alkylene;
$Y^b$ is $C_{2-8}$ alkylene or $C_{2-8}$ alkenylene; and
m is an integer selected from 1 to 100; or

a hyaluronic acid derivative comprising a repeating unit represented by the formula (II):

[Formula 2]

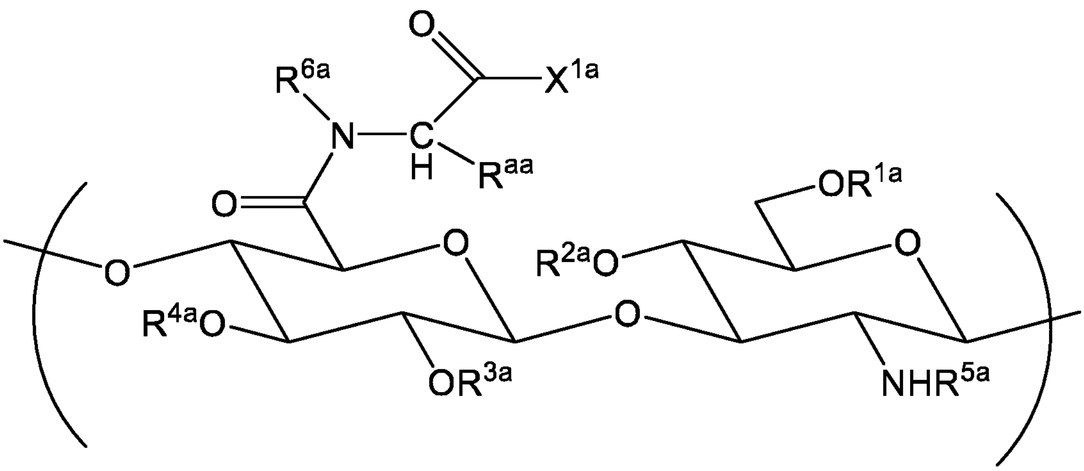

(II)

wherein $R^{1a}$, $R^{2a}$, $R^{3a}$, and $R^{4a}$ are each independently selected from a hydrogen atom, $C_{1-6}$ alkyl, formyl, and $C_{1-6}$ alkylcarbonyl;

$R^{5a}$ is a hydrogen atom, formyl, or $C_{1-6}$ alkylcarbonyl;
$X^{1a}$ is hydroxy, $-O^-Q^+$, $C_{1-6}$ alkoxy, $-NR^7R^8$, or $-NR^9-Z^1-Z^2$;
$Q^+$ represents a counter cation;
$R^{6a}$, $R^7$, $R^8$, and $R^9$ are each independently selected from a hydrogen atom, and $C_{1-6}$ alkyl;
$R^{aa}$ is a hydrogen atom, or $C_{1-6}$ alkyl, wherein the alkyl is optionally substituted by one or more groups each independently selected from hydroxy, carboxy, carbamoyl, $C_{1-6}$ alkylthio, aryl, and heteroaryl, wherein the aryl is optionally substituted by one or more hydroxy groups;
$Z^1$ is $C_{2-30}$ alkylene, or $-(CH_2CH_2O)_{ma}-CH_2CH_2-$, wherein in the alkylene, 1 to 5 groups each independently selected from-O-, -$NR^{ga}$- and -S-S- are optionally inserted, and ma is an integer selected from 1 to 100;
$Z^2$ is selected from groups represented by the following formulas:

$-NR^{ba}-Z^3,$

$-NR^{ba}-COO-Z^3,$

$-NR^{ba}-CO-Z^3,$

$-NR^{ba}-CO-NR^{ca}-Z^3,$

-COO-$Z^3$,

-CO-$NR^{ca}$-$Z^3$,

-O-C

-O-COO-$Z^3$,

-S-$Z^3$,

-CO-$Z^a$-S-$Z^3$,

-O-CO-$Z^b$-S-$Z^3$,

-$NR^{ba}$-CO-$Z^b$-S-$Z^3$,

and

-S-S-$Z^3$;

$R^{ba}$ and $R^{ca}$ are each independently selected from a hydrogen atom, $C_{1-20}$ alkyl, amino-$C_{2-2}$o alkyl and hydroxy-$C_{2-20}$ alkyl, wherein in the alkyl moiety of each of the groups, 1 to 3 groups each independently selected from -O- and -$NR^{fa}$- are optionally inserted;
$R^{fa}$ is independently selected from a hydrogen atom, $C_{1-12}$ alkyl, amino-$C_{2-12}$ alkyl and hydroxy-$C_{2-12}$ alkyl, wherein in the alkyl moiety of each of the groups, 1 or 2 groups each independently selected from -O- and -NH- are optionally inserted;
$R^{ga}$ is independently selected from a hydrogen atom, $C_{1-20}$ alkyl, amino-$C_{2-20}$ alkyl and hydroxy-$C_{2-20}$ alkyl, wherein in the alkyl moiety of each of the groups, 1 to 3 groups each independently selected from -O- and -NH- are optionally inserted;
$Z^3$ is a steryl group;
$Z^a$ is $C_{1-5}$ alkylene; and
$Z^b$ is $C_{2-8}$ alkylene or $C_{2-8}$ alkenylene,

the hyaluronic acid derivative, when not comprising a repeating unit represented by the formula (II) wherein $X^{1a}$ is -$NR^9$-$Z^1$-$Z^2$, further comprising a repeating unit represented by the formula (III): [0014]

[Formula 3]

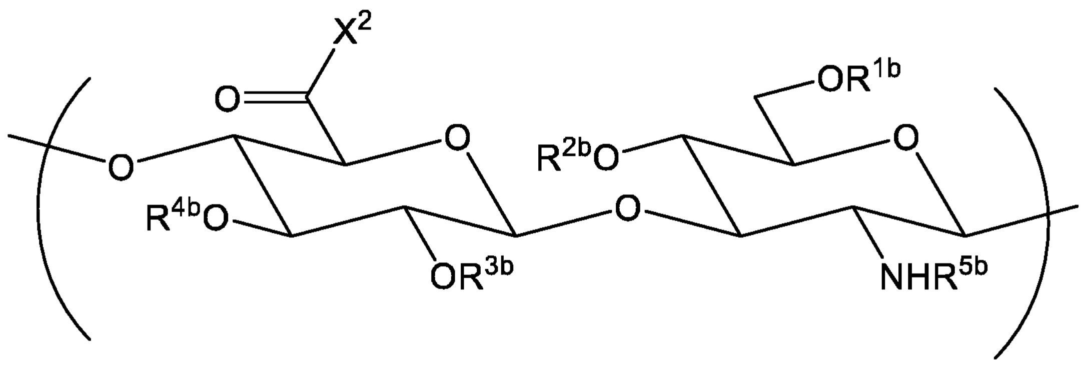

(III)

wherein $R^{1b}$, $R^{2b}$, $R^{3b}$ and $R^{4b}$ are each independently selected from a hydrogen atom, $C_{1-6}$ alkyl, formyl, and $C_{1-6}$ alkylcarbonyl;

$R^{5b}$ is a hydrogen atom, formyl, or $C_{1-6}$ alkylcarbonyl; and
$X^2$ is -$NR^9$-$Z^1$-$Z^2$, wherein $R^9$, $Z^1$, and $Z^2$ are as already defined.

[2] The vaccine formulation according to [1], wherein the hyaluronic acid derivative having an introduced hydrophobic

group further comprises a repeating unit represented by the formula (IIIc):

[Formula 4]

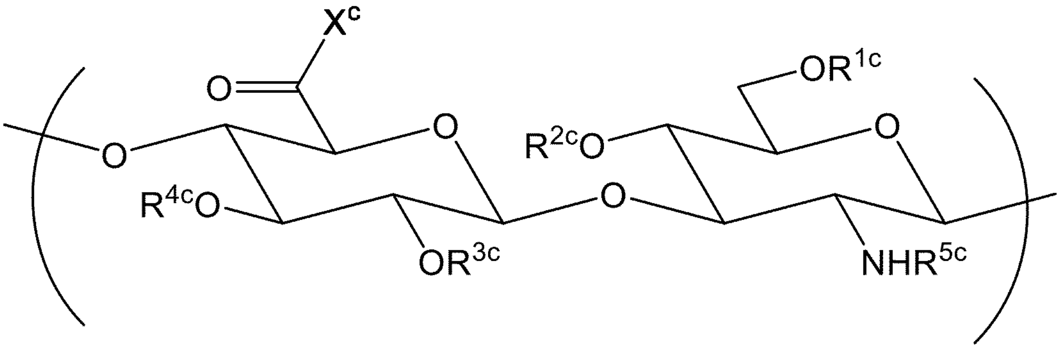

(IIIc)

wherein $R^{1c}$, $R^{2c}$, $R^{3c}$ and $R^{4c}$ are each independently selected from a hydrogen atom, $C_{1-6}$ alkyl, formyl and $C_{1-6}$ alkylcarbonyl;

$R^{5c}$ is selected from a hydrogen atom, formyl and $C_{1-6}$ alkylcarbonyl; and
$X^c$ is selected from hydroxy and $-O^-Q^+$, wherein $Q^+$ represents a counter cation.

[3] The vaccine formulation according to [1] or [2], wherein the vaccine formulation comprises a hyaluronic acid derivative comprising a repeating unit represented by the formula (I), wherein a ratio of the repeating unit represented by the formula (I) to disaccharide repeating units present is 5 to 50%.
[4] The vaccine formulation according to [1] or [2], wherein the vaccine formulation comprises a hyaluronic acid derivative comprising a repeating unit represented by the formula (II), wherein a ratio of a disaccharide unit comprising the group $-NR^9-Z^1-Z^2$ to disaccharide repeating units present is 5 to 50%.
[5] The vaccine formulation according to any of [1] to [3], wherein the vaccine formulation comprises a hyaluronic acid derivative comprising a repeating unit represented by the formula (I), wherein Z is a direct bond, Y is $C_{2-10}$ alkylene, $X^1$ is -NH-COO-R, and R is a cholesteryl group.
[6] The vaccine formulation according to any of [1], [2] and [4], wherein the vaccine formulation comprises a hyaluronic acid derivative comprising a repeating unit represented by the formula (II), wherein $Z^1$ is $C_{2-10}$ alkylene, $Z^2$ is -NH-COO-$Z^3$, and $Z^3$ is a cholesteryl group.
[7] The vaccine formulation according to any of [1] to [6], wherein the hyaluronic acid derivative is produced using hyaluronic acid composed only of a disaccharide unit represented by the formula (IIIc) defined in claim 2, wherein when all of $R^{1c}$, $R^{2c}$, $R^{3c}$, and $R^{4c}$ are hydrogen atoms, $R^{5c}$ is acetyl, and $X^c$ is $-O^-Na^+$, a weight-average molecular weight is 5 kilodaltons to 200 kilodaltons.
[8] The vaccine formulation according to any of [1] to [7], wherein the hyaluronic acid derivative and the antigen form a complex.
[9] The vaccine formulation according to any of [1] to [8] for administration in combination with at least one type of adjuvant.
[10] The vaccine formulation according to any of [1] to [9], wherein the antigen is an antigenic peptide or an antigenic protein.
[11] The vaccine formulation according to [10], wherein the antigenic peptide comprises two or more CD8-positive cytotoxic T cell recognition epitopes or CD4-positive helper T cell recognition epitopes.
[12] The vaccine formulation according to [11], wherein the antigenic peptide has an amino acid linker between the epitopes.
[13] The vaccine formulation according to any of [1] to [12] for administration in combination with at least one type of antibody for use in cancer treatment.
[14] A complex formed from a hyaluronic acid derivative comprising a repeating unit represented by the formula (I) or the formula (II) according to any of [1] to [7], and an antigen that is used in a vaccine for use in the prevention or treatment of a cancer.
[15] The vaccine formulation according to any of [1] to [3], [5] and [7] to [13], wherein the vaccine formulation comprises a hyaluronic acid derivative comprising at least one repeating unit represented by the formula (I), wherein Y is $-(CH_2)_{n1}-$ or $-(CH_2CH_2O)_{m1}-CH_2CH_2-$, n1 is an integer of 2 to 15, and m1 is an integer of 1 to 4.
[16] The vaccine formulation according to any of [1] to [3], [5], [7] to [13] and [15], wherein the vaccine formulation comprises a hyaluronic acid derivative comprising at least one repeating unit represented by the formula (I), wherein

Y is $-(CH_2)_{n1}-$.

[17] The vaccine formulation according to any of [1] to [3], [5], [7] to [13], [15], and [16], wherein the vaccine formulation comprises a hyaluronic acid derivative comprising at least one repeating unit represented by the formula (I), wherein Y is $-(CH_2)_{n1}$, and n1 is 2, 6, 8, or 12.

[18] The vaccine formulation according to any of [1] to [13] and [15] to [17] for administration in combination with at least one type of adjuvant, wherein the adjuvant is i) a substance activating an innate immune receptor (pattern recognition receptor), ii) an antigen-presenting cell-stimulating substance, or iii) a substance having an effect of inhibiting the acquirement of immunosuppressive activity by antigen-presenting cells.

[19] The vaccine formulation according to any of [1] to [13] and [15] to [18] for administration in combination with at least one type of adjuvant, wherein the adjuvant is CpG oligo DNA, polyIC, QuilA, QS21, Sting, monophosphoryl lipid, R848, imiquimod, or MPL.

[20] The vaccine formulation according to any of [1] to [13] and [15] to [19], wherein the antigen is an antigenic peptide, and the antigenic peptide comprises one or more each of CD8-positive cytotoxic T cell recognition epitopes and CD4-positive helper T cell recognition epitopes.

[21] The vaccine formulation according to any of [1] to [13] and [15] to [20], wherein the antigen is an antigenic peptide, and the number of amino acid residues in the antigenic peptide is 8 to 120.

[22] The vaccine formulation according to any of [1] to [13] and [15] to [21], wherein the antigen is an antigenic peptide, and the number of amino acid residues in the antigenic peptide is 8 to 50.

[23] The vaccine formulation according to any of [1] to [13] and [15] to [22], wherein the antigen is an antigenic peptide, and the number of amino acid residues in the antigenic peptide is 16 to 80.

[24] The vaccine formulation according to any of [1] to [13] and [15] to [23], wherein the antigen is an antigenic peptide, and the number of amino acid residues in the antigenic peptide is 23 to 60.

[25] The vaccine formulation according to any of [1] to [13] and [15] to [24] for administration in combination with at least one type of antibody for use in cancer treatment, wherein the antibody is an antibody inhibiting an immunosuppressive signal from tumor, or at least one type of antibody activating a costimulatory signal of immunocytes.

[26] The vaccine formulation according to any of [1] to [13] and [15] to [25] for administration in combination with at least one type of antibody for use in cancer treatment, wherein the antibody is an anti-CTLA4 antibody, an anti-PDI antibody, an anti-PDLI antibody, an anti-OX40 antibody, or an anti-4-1BB antibody.

[27] The vaccine formulation according to any of [1] to [13] and [15] to [26] for administration in combination with at least one type of antibody for use in cancer treatment, wherein the antibody is an anti-PDLI antibody.

[28] A complex formed from a hyaluronic acid derivative comprising a repeating unit represented by the formula (I) or the formula (II) according to any of [15] to [17], and an antigen that is used in a vaccine for use in the prevention or treatment of a cancer.

[29] A method for producing a complex of a hyaluronic acid derivative having an introduced hydrophobic group, and an antigen, comprising the step of mixing a hyaluronic acid derivative comprising a repeating unit represented by the formula (I) or the formula (II) according to any of [1] to [7] and [15] to [17] with an antigenic peptide that is used in a vaccine for use in the prevention or treatment of a cancer in a solution.

In another aspect, the present invention provides a method for preventing and/or treating a cancer according to any of [30] to [46], and use according to any of [47] to [63] as follows.

[30] A method for preventing and/or treating a cancer, comprising administering a vaccine formulation comprising a hyaluronic acid derivative comprising a repeating unit represented by the formula (I) or the formula (II), and an antigen according to any of [1] to [7] and [15] to [17] to a subject.

[31] The method according to [30], wherein the hyaluronic acid derivative and the antigen form a complex.

[32] The method according to [30], wherein the vaccine formulation is administered in combination with at least one type of adjuvant.

[33] The method according to [30], wherein the antigen is an antigenic peptide or an antigenic protein.

[34] The method according to [33], wherein the antigenic peptide comprises two or more CD8-positive cytotoxic T cell recognition epitopes or CD4-positive helper T cell recognition epitopes.

[35] The method according to [34], wherein the antigenic peptide has an amino acid linker between epitopes.

[36] The method according to [30], wherein the vaccine formulation is administered in combination with at least one type of antibody for use in cancer treatment.

[37] The method according to [30], wherein the vaccine formulation is administered in combination with at least one type of adjuvant, and the adjuvant is i) a substance activating an innate immune receptor (pattern recognition receptor), ii) an antigen-presenting cell-stimulating substance, or iii) a substance having an effect of inhibiting the acquirement of immunosuppressive activity by antigen-presenting cells.

[38] The method according to [30], wherein the vaccine formulation is administered in combination with at least one type of adjuvant, and the adjuvant is CpG oligo DNA, polyIC, QuilA, QS21, Sting, monophosphoryl lipid, R848, imiquimod, or MPL.

[39] The method according to [30], wherein the antigen is an antigenic peptide, and the antigenic peptide comprises one or more each of CD8-positive cytotoxic T cell recognition epitopes and CD4-positive helper T cell recognition epitopes.

[40] The method according to [30], wherein the antigen is an antigenic peptide, and the number of amino acid residues in the antigenic peptide is 8 to 120.

[41] The method according to [30], wherein the antigen is an antigenic peptide, and the number of amino acid residues in the antigenic peptide is 8 to 50.

[42] The method according to [30], wherein the antigen is an antigenic peptide, and the number of amino acid residues in the antigenic peptide is 16 to 80.

[43] The method according to [30], wherein the antigen is an antigenic peptide, and the number of amino acid residues in the antigenic peptide is 23 to 60.

[44] The method according to [30], wherein the vaccine formulation is administered in combination with at least one type of antibody for use in cancer treatment, and the antibody is an antibody inhibiting an immunosuppressive signal from tumor, or at least one type of antibody activating a costimulatory signal of immunocytes.

[45] The method according to [30], wherein the vaccine formulation is administered in combination with at least one type of antibody for use in cancer treatment, and the antibody is an anti-CTLA4 antibody, an anti-PDl antibody, an anti-PDLl antibody, an anti-OX40 antibody, or an anti-4-1BB antibody.

[46] The method according to [30], wherein the vaccine formulation is administered in combination with at least one type of antibody for use in cancer treatment, and the antibody is an anti-PDLl antibody.

[47] Use of a hyaluronic acid derivative comprising a repeating unit represented by the formula (I) or the formula (II), and an antigen according to any of [1] to [7] and [15] to [17] in the production of a vaccine formulation for use in the prevention or treatment of a cancer.

[48] Use according to [47], wherein the hyaluronic acid derivative and the antigen form a complex.

[49] Use according to [47], wherein the vaccine formulation is administered in combination with at least one type of adjuvant.

[50] Use according to [47], wherein the antigen is an antigenic peptide or an antigenic protein.

[51] Use according to [50], wherein the antigenic peptide comprises two or more CD8-positive cytotoxic T cell recognition epitopes or CD4-positive helper T cell recognition epitopes.

[52] Use according to [51], wherein the antigenic peptide has an amino acid linker between epitopes.

[53] Use according to [47], wherein the vaccine formulation is administered in combination with at least one type of antibody for use in cancer treatment.

[54] Use according to [47], wherein the vaccine formulation is administered in combination with at least one type of adjuvant, and the adjuvant is i) a substance activating an innate immune receptor (pattern recognition receptor), ii) an antigen-presenting cell-stimulating substance, or iii) a substance having an effect of inhibiting the acquirement of immunosuppressive activity by antigen-presenting cells.

[55] Use according to [47], wherein the vaccine formulation is administered in combination with at least one type of adjuvant, and the adjuvant is CpG oligo DNA, polyIC, QuilA, QS21, Sting, monophosphoryl lipid, R848, imiquimod, or MPL.

[56] Use according to [47], wherein the antigen is an antigenic peptide, and the antigenic peptide comprises one or more each of CD8-positive cytotoxic T cell recognition epitopes and CD4-positive helper T cell recognition epitopes.

[57] Use according to [47], wherein the antigen is an antigenic peptide, and the number of amino acid residues in the antigenic peptide is 8 to 120.

[58] Use according to [47], wherein the antigen is an antigenic peptide, and the number of amino acid residues in the antigenic peptide is 8 to 50.

[59] Use according to [47], wherein the antigen is an antigenic peptide, and the number of amino acid residues in the antigenic peptide is 16 to 80.

[60] Use according to [47], wherein the antigen is an antigenic peptide, and the number of amino acid residues in the antigenic peptide is 23 to 60.

[61] Use according to [47], wherein the vaccine formulation is administered in combination with at least one type of antibody for use in cancer treatment, and the antibody is an antibody inhibiting an immunosuppressive signal from tumor, or at least one type of antibody activating a costimulatory signal of immunocytes.

[62] Use according to [47], wherein the vaccine formulation is administered in combination with at least one type of antibody for use in cancer treatment, and the antibody is an anti-CTLA4 antibody, an anti-PDl antibody, an anti-PDLl antibody, an anti-OX40 antibody, or an anti-4-1BB antibody.

[63] Use according to [47], wherein the vaccine formulation is administered in combination with at least one type of antibody for use in cancer treatment, and the antibody is an anti-PDLl antibody.

ADVANTAGEOUS EFFECTS OF INVENTION

Use of the vaccine formulation comprising a complex of a hyaluronic acid derivative having an introduced hydrophobic group, and an antigen according to the present invention improves the accumulation of the antigen to lymph node, enables marked induction of antigen-specific CTL, and enables potentiation of an anti-cancer effect by immunity. The complex and the vaccine formulation of the present invention also have characteristics excellent in safety, particularly, safety in long-term administration, because the hyaluronic acid derivative used is also excellent in safety.

BRIEF DESCRIPTION OF DRAWINGS

Figure 1 is a graph showing the ratio of the number of interferon gamma-producing $CD8^+$ cells to the number of all $CD8^+$ cells in BALB/c mouse-derived spleen cells (ordinate) when mERK2 p121 was used as a peptide for $CD8^+$ T cell stimulation. Each sample is as follows: NT: spleen cells derived from an untreated mouse, CpG: spleen cells derived from a mouse given an antigenic peptide (mERK2 p121) and CpG oligo DNA, CHP+CpG: spleen cells derived from a mouse given a complex of CHP (CHP-80T; 80 k) and the antigenic peptide, and CpG oligo DNA, 99k41+CpG: spleen cells derived from a mouse given a complex of the antigenic peptide and a HA derivative (99k HA-$C_6$-Chol-41%), and CpG oligo DNA.

Figure 2-1 is a graph showing the ratio of the number of interferon gamma-producing $CD8^+$ cells to the number of all $CD8^+$ cells in BALB/c mouse-derived spleen cells (ordinate) when mERK2 p121 was used as a peptide for $CD8^+$ T cell stimulation. Each sample is as follows: NT: spleen cells derived from an untreated mouse, 99k41: spleen cells derived from a mouse given a complex of an antigenic peptide (mERK2 p121) and a HA derivative (99k HA-$C_6$-Chol-41%), 99k41+CpG: spleen cells derived from a mouse given a complex of the antigenic peptide and the HA derivative, and CpG oligo DNA, 99k41+polyIC: spleen cells derived from a mouse given a complex of the antigenic peptide and the HA derivative, and polyIC, 99k41+QuilA: spleen cells derived from a mouse given a complex of the antigenic peptide and the HA derivative, and QuilA, 99k41+sting: spleen cells derived from a mouse given a complex of the antigenic peptide and the HA derivative, and Sting.

Figure 2-2 is a graph showing the ratio of the number of interferon gamma-producing $CD4^+$ cells to the number of all $CD4^+$ cells in BALB/c mouse-derived spleen cells (ordinate) when mERK2 p121 was used as a peptide for $CD4^+$ T cell stimulation. Each sample is the same as in Figure 2-1.

Figure 3-1 is a graph showing the ratio of the number of interferon gamma-producing $CD8^+$ cells to the number of all $CD8^+$ cells in BALB/c mouse-derived spleen cells (ordinate) when mERK2 p121 was used as a peptide for $CD8^+$ T cell stimulation. Each sample is as follows: NT: spleen cells derived from an untreated mouse, 99k41+CpG: spleen cells derived from a mouse given a complex of an antigenic peptide (mERK2 p121) and a HA derivative (99k HA-$C_6$-Chol-41%), and CpG oligo DNA, 99k41+R848: spleen cells derived from a mouse given a complex of the antigenic peptide and the HA derivative, and R848, 99k41+MPL: spleen cells derived from a mouse given a complex of the antigenic peptide and the HA derivative, and MPL.

Figure 3-2 is a graph showing the ratio of the number of interferon gamma-producing $CD4^+$ cells to the number of all $CD4^+$ cells in BALB/c mouse-derived spleen cells (ordinate) when mERK2 p121 was used as a peptide for $CD4^+$ T cell stimulation. Each sample is the same as in Figure 3-1.

Figure 4 is a graph showing the ratio of the number of interferon gamma-producing $CD8^+$ cells to the number of all $CD8^+$ cells in BALB/c mouse-derived spleen cells (ordinate) when MAGE-A4 p265 was used as a peptide for $CD8^+$ T cell stimulation. Each sample is as follows: NT: spleen cells derived from an untreated mouse, peptide+CpG: spleen cells derived from a mouse given an antigenic peptide (MAGE-A4 p264) and CpG oligo DNA, CHP/Wt+CpG: spleen cells derived from a mouse given a complex of CHP (80 k) and the antigenic peptide, and CpG oligo DNA, 99k41/Wt+CpG: spleen cells derived from a mouse given a complex of the antigenic peptide and a HA derivative (99k HA-$C_6$-Chol-41%), and CpG oligo DNA.

Figure 5 is a graph showing the ratio of the number of interferon gamma-producing $CD8^+$ cells to the number of all $CD8^+$ cells in BALB/c mouse-derived spleen cells (ordinate) when MAGE-A4 p265 was used as a peptide for $CD8^+$ T cell stimulation. Each sample is as follows: NT: spleen cells derived from an untreated mouse, CHP/Wt: spleen cells derived from a mouse given a complex of CHP (80 k) and an antigenic peptide (MAGE-A4 p264), 99k41/Wt: spleen cells derived from a mouse given a complex of the antigenic peptide and a HA derivative (99k HA-$C_6$-Chol-41%).

Figure 6 is a graph showing the ratio of the number of interferon gamma-producing $CD8^+$ cells to the number of all $CD8^+$ cells in BALB/c mouse-derived spleen cells (ordinate) when MAGE-A4 p265 was used as a peptide for $CD8^+$ T cell stimulation. Each sample is as follows: NT: spleen cells derived from an untreated mouse, 99k41/Wt+CpG: spleen cells derived from a mouse given a complex of an antigenic peptide (MAGE-A4 p264) and a HA derivative

(99k HA-$C_6$-Chol-41%), and CpG oligo DNA, 99k41/LLLL+CpG: spleen cells derived from a mouse given a complex of an antigenic peptide (MAGE-A4 p264-4L) and the HA derivative, and CpG oligo DNA, 99k41/WWWW+CpG: spleen cells derived from a mouse given a complex of an antigenic peptide (MAGE-A4 p264-4W) and the HA derivative, and CpG oligo DNA.

Figure 7-1 is a graph showing the ratio of the number of interferon gamma-producing $CD8^+$ cells to the number of all $CD8^+$ cells in C57BL/6 mouse-derived spleen cells (ordinate) when TRP1 was used as a peptide for $CD8^+$ T cell stimulation. Each sample is as follows: NT: spleen cells derived from an untreated mouse, CHP/6Y+CpG: spleen cells derived from a mouse given a complex of CHP and an antigenic peptide (TRP2TRP1gp100-6Y), and CpG oligo DNA, 99k43/6Y+CpG: spleen cells derived from a mouse given a complex of the antigenic peptide and a HA derivative (99k HA-$C_6$-Chol-43%), and CpG oligo DNA.

Figure 7-2 is a graph showing the ratio of the number of interferon gamma-producing $CD8^+$ cells to the number of all $CD8^+$ cells in C57BL/6 mouse-derived spleen cells (ordinate) when TRP2 was used as a peptide for $CD8^+$ T cell stimulation. Each sample is the same as in Figure 7-1.

Figure 7-3 is a graph showing the ratio of the number of interferon gamma-producing $CD8^+$ cells to the number of all $CD8^+$ cells in C57BL/6 mouse-derived spleen cells (ordinate) when gp100 was used as a peptide for $CD8^+$ T cell stimulation. Each sample is the same as in Figure 7-1.

Figure 8-1 is a graph showing the ratio of the number of interferon gamma-producing $CD8^+$ cells to the number of all $CD8^+$ cells in C57BL/6 mouse-derived spleen cells (ordinate) when TRP1 was used as a peptide for $CD8^+$ T cell stimulation. Each sample is as follows: NT: spleen cells derived from an untreated mouse, 99k43/6G+CpG: spleen cells derived from a mouse given a complex of an antigenic peptide (TRP2TRP1gp100-6G) and a HA derivative (99k HA-$C_6$-Chol-43%), and CpG oligo DNA, 99k43/6Y+CpG: spleen cells derived from a mouse given a complex of an antigenic peptide (TRP2TRP1gp100-6Y) and the HA derivative, and CpG oligo DNA, 99k43/4L+CpG: spleen cells derived from a mouse given a complex of an antigenic peptide (TRP2TRP1gp100-4L) and the HA derivative, and CpG oligo DNA, 99k43/6L+CpG: spleen cells derived from a mouse given a complex of an antigenic peptide (TRP2TRP1gp100-6L) and the HA derivative, and CpG oligo DNA.

Figure 8-2 is a graph showing the ratio of the number of interferon gamma-producing $CD8^+$ cells to the number of all $CD8^+$ cells in C57BL/6 mouse-derived spleen cells (ordinate) when TRP2 was used as a peptide for $CD8^+$ T cell stimulation. Each sample is the same as in Figure 8-1.

Figure 8-3 is a graph showing the ratio of the number of interferon gamma-producing $CD8^+$ cells to the number of all $CD8^+$ cells in C57BL/6 mouse-derived spleen cells (ordinate) when gp100 was used as a peptide for $CD8^+$ T cell stimulation. Each sample is the same as in Figure 8-1.

Figure 9-1 is a graph showing the ratio of the number of interferon gamma-producing $CD8^+$ cells to the number of all $CD8^+$ cells in BALB/c mouse-derived spleen cells (ordinate) when AH1 was used as a peptide for $CD8^+$ T cell stimulation. Each sample is as follows: NT: spleen cells derived from an untreated mouse, CHP/6Y+CpG: spleen cells derived from a mouse given a complex of CHP and an antigenic peptide (AH1gp70-6Y), and CpG oligo DNA, 99k43/6Y+CpG: spleen cells derived from a mouse given a complex of the antigenic peptide and a HA derivative (99k HA-$C_6$-Chol-43%), and CpG oligo DNA.

Figure 9-2 is a graph showing the ratio of the number of interferon gamma-producing $CD4^+$ cells to the number of all $CD4^+$ cells in BALB/c mouse-derived spleen cells (ordinate) when gp70 was used as a peptide for $CD4^+$ T cell stimulation. Each sample is the same as in Figure 9-1.

Figure 10-1 is a graph showing the ratio of the number of interferon gamma-producing $CD8^+$ cells to the number of all $CD8^+$ cells in BALB/c mouse-derived spleen cells (ordinate) when AH1 was used as a peptide for $CD8^+$ T cell stimulation. Each sample is as follows: NT: spleen cells derived from an untreated mouse, CHP/6L+CpG: spleen cells derived from a mouse given a complex of CHP and an antigenic peptide (AH1gp70-6L), and CpG oligo DNA, 99k43/6L+CpG: spleen cells derived from a mouse given a complex of the antigenic peptide and a HA derivative (99k HA-$C_6$-Chol-43%), and CpG oligo DNA.

Figure 10-2 is a graph showing the ratio of the number of interferon gamma-producing $CD4^+$ cells to the number of all $CD4^+$ cells in BALB/c mouse-derived spleen cells (ordinate) when gp70 was used as a peptide for $CD4^+$ T cell stimulation. Each sample is the same as in Figure 10-1.

Figure 11-1 is a graph showing the ratio of the number of interferon gamma-producing $CD8^+$ cells to the number of all $CD8^+$ cells in BALB/c mouse-derived spleen cells (ordinate) when AH1 was used as a peptide for $CD8^+$ T cell stimulation. Each sample is as follows: NT: spleen cells derived from an untreated mouse, 99k43/6G+CpG: spleen cells derived from a mouse given a complex of an antigenic peptide (AH1gp70-6G) and a HA derivative (99k HA-$C_6$-Chol-43%), and CpG oligo DNA, 99k43/6Y+CpG: spleen cells derived from a mouse given a complex of an antigenic peptide (AH1gp70-6Y) and the HA derivative, and CpG oligo DNA, 99k43/4L+CpG: spleen cells derived from a mouse given a complex of an antigenic peptide (AH1gp70-4L) and the HA derivative, and CpG oligo DNA, 99k43/6L+CpG: spleen cells derived from a mouse given a complex of an antigenic peptide (AH1gp70-6L) and the HA derivative, and CpG oligo DNA.

Figure 11-2 is a graph showing the ratio of the number of interferon gamma-producing $CD4^+$ cells to the number of all $CD4^+$ cells in BALB/c mouse-derived spleen cells (ordinate) when gp70 was used as a peptide for $CD4^+$ T cell stimulation. Each sample is the same as in Figure 11-1.

Figure 12-1 is a graph showing changes in the average tumor volume of a mouse fibrosarcoma CMS5a-bearing BALB/c mouse group (ordinate). Each group is as follows: control: a mouse group given no sample, peptide: a mouse group given an antigenic peptide (mERK2 p121) and CpG oligo DNA, emulsion: a mouse group given an emulsion formulation produced from the antigenic peptide, and CpG oligo DNA, CHP: a mouse group given a complex of CHP and the antigenic peptide, and CpG oligo DNA, 99k41: a mouse group given a complex of the antigenic peptide and a HA derivative (99k HA-$C_6$-Chol-41%), and CpG oligo DNA.

Figure 12-2 is a graph showing changes in the tumor volume of each individual of a mouse fibrosarcoma CMS5a-bearing BALB/c mouse group (ordinate) on a group basis. Each group is the same as in Figure 12-1.

Figure 13-1 is a graph showing changes in the average tumor volume of a mouse fibrosarcoma CMS5a-bearing BALB/c mouse group (n = 5) (ordinate). Each group is as follows: control: a mouse group given no sample, CpG: a mouse group given CpG oligo DNA, CHP: a mouse group given a complex of CHP and an antigenic peptide (mERK2 p121), and CpG oligo DNA, 99k41: a mouse group given a complex of the antigenic peptide and a HA derivative (99k HA-$C_6$-Chol-41%), and CpG oligo DNA.

Figure 13-2 is a graph showing changes in the tumor volume of each individual of a mouse fibrosarcoma CMS5a-bearing BALB/c mouse group (n = 5) (ordinate) on a group basis. Each group is the same as in Figure 13-1.

Figure 14-1 is a graph showing changes in the average tumor volume of a mouse fibrosarcoma CMS5a-bearing BALB/c mouse group (n = 5) (ordinate). Each group is as follows: control: a mouse group given no sample, aCTLA4/aPD1/aPDL1: a mouse group given an anti-CTLA4 antibody, an anti-PD1 antibody, and an anti-PDL1 antibody, 99k41: a mouse group given a complex of an antigenic peptide (mERK2 p121) and a HA derivative (99k HA-$C_6$-Chol-41%), and CpG oligo DNA.

Figure 14-2 is a graph showing changes in the tumor volume of each individual of a mouse fibrosarcoma CMS5a-bearing BALB/c mouse group (n = 5) (ordinate) on a group basis. Each group is the same as in Figure 14-1.

Figure 15-1 is a graph showing changes in the average tumor volume of a mouse fibrosarcoma CMS5a-bearing BALB/c mouse group (n = 5) (ordinate). Each group is as follows: control: a mouse group given no sample, aCTLA4/aPDL1/aOX40/a4-1BB: a mouse group given an anti-CTLA4 antibody, an anti-PDL1 antibody, an anti-OX40 antibody, and an anti-4-1BB antibody, 99k41: a mouse group given a complex of an antigenic peptide (mERK2 p121) and a HA derivative (99k HA-$C_6$-Chol-41%), and CpG oligo DNA, 99k41+aCTLA4/aPDL1/aOX40/a4-1BB: a mouse group given a complex of the antigenic peptide and the HA derivative, CpG oligo DNA, and an anti-CTLA4 antibody, an anti-PDL1 antibody, an anti-OX40 antibody, and an anti-4-1BB antibody.

Figure 15-2 is a graph showing changes in the tumor volume of each individual of a mouse fibrosarcoma CMS5a-bearing BALB/c mouse group (n = 5) (ordinate) on a group basis. Each group is the same as in Figure 15-1.

Figure 16-1 is a graph showing changes in the average tumor volume of a mouse colorectal cancer cell CT26-bearing BALB/c mouse group (ordinate). Each group is as follows: control: a mouse group given no sample, emulsion: a mouse group given an emulsion formulation produced from an antigenic peptide (AH1gp70-6L), and CpG oligo DNA, CHP: a mouse group given a complex of CHP and the antigenic peptide, and CpG oligo DNA, 99k43: a mouse group given a complex of the antigenic peptide and a HA derivative (99k HA-$C_6$-Chol-43%), and CpG oligo DNA.

Figure 16-2 is a graph showing changes in the tumor volume of each individual of a mouse colorectal cancer cell CT26-bearing BALB/c mouse group (n = 5) (ordinate) on a group basis. Each group is the same as in Figure 16-1.

Figure 17-1 is a graph showing changes in the average tumor volume of a mouse melanoma B16F10-bearing C57BL/6 mouse group (ordinate). Each group is as follows: control: a mouse group given no sample, CHP: a mouse group given a complex of CHP and an antigenic peptide (TRP2TRP1gp100-6Y), and CpG oligo DNA, 99k43: a mouse group given a complex of the antigenic peptide and a HA derivative (99k HA-$C_6$-Chol-43%), and CpG oligo DNA.

Figure 17-2 is a graph showing changes in the tumor volume of each individual of a mouse melanoma B16F10-bearing C57BL/6 mouse group (n = 5) (ordinate) on a group basis. Each group is the same as in Figure 17-1.

Figure 18-1 is a graph showing fluorescence intensity per cell of BALB/c mouse lymph node 24 hours after sample administration in a lymph node migration test. Each administration sample is as follows: NT: no sample added, LPA: an antigenic peptide (fluorescently labeled mERK2 p121 (fluorescein-conjugated)), CHP: a complex of CHP and the antigenic peptide, 10k17: a complex of the antigenic peptide and a HA derivative (10k HA-$C_6$-Chol-17%), 10k25: a complex of the antigenic peptide and a HA derivative(10k HA-$C_6$-Chol-25%), 10k42: a complex of the antigenic peptide and a HA derivative (10k HA-$C_6$-Chol-42%), 50k23: a complex of the antigenic peptide and a HA derivative (50k HA-$C_6$-Chol-23%), 50k43: a complex of the antigenic peptide and a HA derivative (50k HA-$C_6$-Chol-43%), 99k24: a complex of the antigenic peptide and a HA derivative (99k HA-$C_6$-Chol-24%), 99k43: a complex of the antigenic peptide and a HA derivative (99k HA-$C_6$-Chol-43%).

Figure 18-2 is a graph showing fluorescence intensity per cell of BALB/c mouse lymph node 72 hours after sample

administration in a lymph node migration test. Each administration sample is the same as in Figure 18-1.

Figure 19 is a graph showing fluorescence intensity per cell of BALB/c mouse lymph node 24 hours after sample administration in a lymph node migration test. Each administration sample is as follows: NT: no sample added, peptide: an antigenic peptide (fluorescently labeled mERK2 p121 (fluorescein-conjugated)), 99k41: a complex of the antigenic peptide and a HA derivative (99k HA-$C_6$-Chol-41%), 10kHA-Ala-Chol30: a complex of the antigenic peptide and a HA derivative (10k HA-Ala-$C_6$-Chol-30%), 10kHA-Ser-Chol28: a complex of the antigenic peptide and a HA derivative (10k HA-Ser-$C_6$-Chol-28%), 10kHA-Gly-Chol32: a complex of the antigenic peptide and a HA derivative (10k HA-Gly-$C_6$-Chol-32%), 10kHA-Thr-Chol32: a complex of the antigenic peptide and a HA derivative (10k HA-Thr-$C_6$-Chol-32%), 10kHA-Asn-Chol20: a complex of the antigenic peptide and a HA derivative (10k HA-Asn-$C_6$-Chol-20%), 10kHA-Asp-Chol32: a complex of the antigenic peptide and a HA derivative (10k HA-Asp-$C_6$-Chol-32%), 10kHA-Phe-Chol31: a complex of the antigenic peptide and a HA derivative (10k HA-Phe-$C_6$-Chol-31%), 10kHA-Tyr-Chol32: a complex of the antigenic peptide and a HA derivative (10k HA-Tyr-$C_6$-Chol-32%), 10kHA-Ile-Chol28: a complex of the antigenic peptide and a HA derivative (10kHA-Ile-$C_6$-Chol-28%), 10kHA-Leu-Chol31: a complex of the antigenic peptide and a HA derivative (10kHA-Leu-$C_6$-Chol-31%), 10kHA-Val-Chol32: a complex of the antigenic peptide and a HA derivative (10k HA-Val-$C_6$-Chol-32%), 10kHA-Trp-Chol24: a complex of the antigenic peptide and a HA derivative (10k HA-Trp-$C_6$-Chol-24%), 10kHA-Gln-Chol32: a complex of the antigenic peptide and a HA derivative (10k HA-Gln-$C_6$-Chol-32%), 10kHA-Glu-Chol32: a complex of the antigenic peptide and a HA derivative (10k HA-Glu-$C_6$-Chol-32%).

Figure 20-1 is a graph showing changes in the average tumor volume of a mouse melanoma B16F10-bearing C57BL/6 mouse group (ordinate). Each group is as follows: control: a mouse group given no sample, 99k42: a mouse group given a complex of an antigenic peptide and a HA derivative (99k HA-$C_6$-Chol-42%), and CpG oligo DNA, 50k42: a mouse group given a complex of the antigenic peptide and a HA derivative (50k HA-$C_6$-Chol-42%), and CpG oligo DNA, 10k43: a mouse group given a complex of the antigenic peptide and a HA derivative (10k HA-$C_6$-Chol-43%), and CpG oligo DNA.

Figure 20-2 is a graph showing changes in the tumor volume of each individual of a mouse melanoma B16F10-bearing C57BL/6 mouse group (n = 5) (ordinate) on a group basis. Each group is the same as in Figure 20-1.

Figure 21-1 is a graph showing changes in the average tumor volume of a mouse melanoma B16F10-bearing C57BL/6 mouse group (ordinate). Each group is as follows: control: a mouse group given no sample, 10kHA-Ala-Chol30: a mouse group given a complex of an antigenic peptide and an amino acid-modified HA derivative (10k HA-Ala-$C_6$-Chol-30%), and CpG oligo DNA, 10kHA-Gln-Chol32: a mouse group given a complex of the antigenic peptide and an amino acid-modified HA derivative (10k HA-Gln-$C_6$-Chol-32%), and CpG oligo DNA.

Figure 21-2 is a graph showing changes in the tumor volume of each individual of a mouse melanoma B16F10-bearing C57BL/6 mouse group (n = 5) (ordinate) on a group basis. Each group is the same as in Figure 21-1.

Figure 22-1 is a graph showing changes in the average tumor volume of a mouse melanoma B16F10-bearing C57BL/6 mouse group (ordinate). Each group is as follows: control: a mouse group given no sample, 99k42+PolyIC: a mouse group given a complex of an antigenic peptide and a HA derivative (99k HA-$C_6$-Chol-42%), and polyIC.

Figure 22-2 is a graph showing changes in the tumor volume of each individual of a mouse melanoma B16F10-bearing C57BL/6 mouse group (n = 5) (ordinate) on a group basis. Each group is the same as in Figure 22-1.

Figure 23-1 is a graph showing changes in the average tumor volume of a mouse melanoma B16F10-bearing C57BL/6 mouse group (ordinate). Each group is as follows: control: a mouse group given no sample, 99k42+Sting: a mouse group given a complex of an antigenic peptide and a HA derivative (99k HA-$C_6$-Chol-42%), and a Sting agonist, 99k42+R848: a mouse group given a complex of the antigenic peptide and a HA derivative (99k HA-$C_6$-Chol-42%), and R848.

Figure 23-2 is a graph showing changes in the tumor volume of each individual of a mouse melanoma B16F10-bearing C57BL/6 mouse group (n = 5) (ordinate) on a group basis. Each group is the same as in Figure 23-1.

Figure 24-1 is a graph showing the ratio of the number of TRP2-specific $CD8^+$ cells to the number of all $CD8^+$ cells in mouse melanoma B16F10-bearing mouse-derived tumor or lymph node (ordinate) when H-$2K^b$ TRP-2 Tetramer-SVYDFFVWL-APC was used as a tetramer. Each sample is as follows: NT: cells derived from an untreated cancer-bearing mouse, 99k41: cells derived from a cancer-bearing mouse given a complex of an antigenic peptide (TRP2TRP1gp100-6Y) and a HA derivative (99k HA-$C_6$-Chol-41%), and CpG oligo DNA.

Figure 24-2 is a graph showing the ratio of the number of gp100-specific $CD8^+$ cells to the number of all $CD8^+$ cells in mouse melanoma B16F10-bearing mouse-derived tumor or lymph node (ordinate) when H-$2D^b$ gp100 Tetramer-EGSRNQDWL-PE was used as a tetramer. Each sample is as follows: NT: cells derived from an untreated cancer-bearing mouse, 99k41: cells derived from a cancer-bearing mouse given a complex of an antigenic peptide (TRP2TRP1gp100-6Y) and a HA derivative (99k HA-$C_6$-Chol-41%), and CpG oligo DNA.

DESCRIPTION OF EMBODIMENTS

Hereinafter, the present invention will be described further specifically.

The complex of a hyaluronic acid derivative having an introduced hydrophobic group, and an antigen according to the present invention is a complex of a hyaluronic acid derivative comprising at least one disaccharide unit (which is also a repeating unit) represented by the formula (I), or a hyaluronic acid derivative comprising at least one disaccharide unit (which is also a repeating unit) represented by the formula (II), and an antigen. The complex can be used to produce the vaccine formulation of the present invention. The present specification also includes the disclosure of a method for producing the complex of a hyaluronic acid derivative having an introduced hydrophobic group, and an antigen according to the present invention.

Definition

The term "steryl group" described herein is not particularly limited as long as the group has a steroid skeleton. In this context, examples of the steroid specifically include cholesterol, dehydrocholesterol, coprostanol, coprosterol, cholestanol, campestanol, ergostanol, stigmastanol, coprostanol, stigmasterol, sitosterol, lanosterol, ergosterol, simiarenol, bile acid (cholanic acid, lithocholic acid, hyodeoxycholic acid, chenodeoxycholic acid, ursodeoxycholic acid, deoxycholic acid, apocholic acid, cholic acid, dehydrocholic acid, glycocholic acid, and taurocholic acid), testosterone, estradiol, progesterone, cortisol, cortisone, aldosterone, corticosterone, and deoxycorticosterone. Examples of the steryl group include a cholesteryl group, a stigmasteryl group, a lanosteryl group, an ergosteryl group, a cholanoyl group, and a choloyl group and preferably include a cholesteryl group (particularly, a cholest-5-en-3β-yl group represented by the following formula) and a cholanoyl group (particularly, a 5β-cholan-24-oyl group represented by the following formula).

[Formula 5]

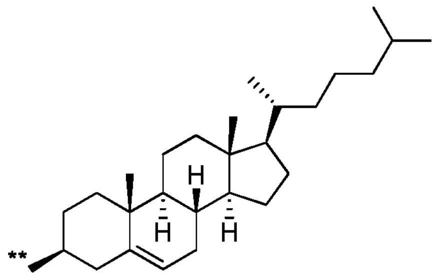

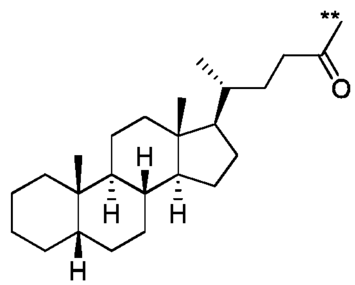

In this context, two asterisks represent a binding position.

The term "$C_{1-20}$ alkyl" described herein means a linear or branched alkyl group having 1 to 20 carbon atoms. Examples thereof include "$C_{1-4}$ alkyl" such as methyl, ethyl, n-propyl, i-propyl, n-butyl, s-butyl, i-butyl, and t-butyl and further include n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, and 2-ethylbutyl. The $C_{1-20}$ alkyl also includes "$C_{1-12}$ alkyl" having 1 to 12 carbon atoms and "$C_{1-6}$ alkyl" having 1 to 6 carbon atoms.

The term "$C_{1-6}$ alkyl" described herein means a linear or branched alkyl group having 1 to 6 carbon atoms. Examples thereof include "$C_{1-4}$ alkyl" such as methyl, ethyl, n-propyl, i-propyl, n-butyl, s-butyl, i-butyl, and t-butyl.

The term "$C_{1-6}$ alkylcarbonyl" described herein means an alkylcarbonyl group whose alkyl moiety is already mentioned $C_{1-6}$ alkyl. Examples thereof include "$C_{1-4}$ alkylcarbonyl" such as acetyl, propionyl, n-propylcarbonyl, i-propylcarbonyl, n-butylcarbonyl, s-butylcarbonyl, i-butylcarbonyl, and t-butylcarbonyl.

The term "$C_{1-6}$ alkoxy" described herein means an alkyloxy group whose alkyl moiety is already mentioned $C_{1-6}$ alkyl. Examples thereof include "$C_{1-4}$ alkoxy" such as methoxy ($H_3C$-O-), ethoxy, n-propoxy, i-propoxy, n-butoxy, s-butoxy, i-butoxy, and t-butoxy.

The term "$C_{1-6}$ alkylthio" described herein means an alkylthio group whose alkyl moiety is already mentioned $C_{1-6}$ alkyl. Examples thereof include methylthio ($H_3C$-S-), ethylthio, n-propylthio, i-propylthio, n-butylthio, s-butylthio, i-butylthio, and t-butylthio and preferably include methylthio.

The term "amino-$C_{2-20}$ alkyl" described herein means a linear or branched alkyl group having 2 to 20 carbon atoms and having an amino group as a substituent. The amino group may be positioned, for example, on a terminal carbon atom of the alkyl group. The amino-$C_{2-20}$ alkyl also includes "amino-$C_{2-12}$ alkyl" having 2 to 12 carbon atoms.

The term "hydroxy-$C_{2-20}$ alkyl" described herein means a linear or branched alkyl group having 2 to 20 carbon atoms and having a hydroxy group as a substituent. The hydroxy group may be positioned, for example, on a terminal carbon atom of the alkyl group. The hydroxy-$C_{2-20}$ alkyl also includes "hydroxy-$C_{2-12}$ alkyl" having 2 to 12 carbon atoms.

The term "$C_{2-30}$ alkylene" described herein means a linear or branched divalent saturated hydrocarbon group having 2 to 30 carbon atoms. Examples thereof include ethylene and propylene and include $C_{2-20}$ alkylene, $C_{2-8}$ alkylene, and a group-$(CH_2)_n$-(wherein n is 2 to 30, preferably 2 to 20, more preferably 2 to 15).

The term "$C_{1-5}$ alkylene" described herein means a linear or branched divalent saturated hydrocarbon group having 1 to 5 carbon atoms. Examples thereof include methylene, ethylene (ethane-1,2-diyl and ethane-1,1-diyl), propylene (propane-1,1-diyl and propane-1,2-diyl), butane-1,4-diyl, and pentane-1,5-diyl.

The term "$C_{2-10}$ alkylene" described herein means a linear or branched divalent saturated hydrocarbon group having 2 to 10 carbon atoms. Examples thereof include ethylene (ethane-1,2-diyl and ethane-1,1-diyl), propylene (propane-1,1-diyl, propane-1,2-diyl, and propane-1,3-diyl), butane-1,4-diyl, pentane-1,5-diyl, hexane-1,6-diyl, heptane-1,7-diyl, and octane-1,8-diyl. The "$C_{2-10}$ alkylene" includes "$C_{2-8}$ alkylene" having 2 to 8 carbon atoms and "$C_{2-6}$ alkylene" having 2 to 6 carbon atoms.

The term "$C_{2-8}$ alkylene" described herein means a linear or branched divalent saturated hydrocarbon group having 2 to 8 carbon atoms. Examples thereof include ethylene (ethane-1,2-diyl and ethane-1,1-diyl), propylene (propane-1,1-diyl, propane-1,2-diyl, and propane-1,3-diyl), butane-1,4-diyl, pentane-1,5-diyl, hexane-1,6-diyl, heptane-1,7-diyl, and octane-1,8-diyl.

The term "$C_{2-8}$ alkenylene" described herein means a linear or branched divalent saturated hydrocarbon group having 2 to 8 carbon atoms and containing one or more double bonds. Examples thereof include -CH=CH-, -$C(CH_3)$=CH-, 2-butene-1,4-diyl, hepta-2,4-diene-1,6-diyl and octa-2,4,6-triene-1,8-diyl. If geometric isomerism is present, respective isomers and their mixtures are also included therein.

In the present invention, the "aryl" means an aromatic carbocyclic group, for example, an aromatic carbocyclic group having 6 to 14 carbon atoms. Examples of the aryl include phenyl and naphthyl (1-naphthyl and 2-naphthyl). Examples of the aryl substituted by one or more hydroxy groups include 4-hydroxyphenyl.

In the present invention, the "heteroaryl" means an aromatic cyclic group containing one or more heteroatoms selected from a nitrogen atom, an oxygen atom and a sulfur atom as ring-constituting atoms, and may be partially saturated. The ring may be monocyclic, or bicyclic heteroaryl condensed with a benzene ring or a monocyclic heteroaryl ring. The number of ring-constituting atoms is, for example, 4 to 15, preferably 5 to 14, more preferably 6 to 10. Examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, imidazopyridyl and preferably indol-2-yl.

Examples of the "divalent $C_{2-50}$ hydrocarbon group" described herein include, but are not particularly limited to, linear, branched, cyclic and partially cyclic alkylene groups, alkenylene groups and alkynylene groups having 2 to 50 carbon atoms. Each of these groups may be a divalent aromatic ring or may contain an aromatic ring as a portion of the structure.

The "divalent $C_{2-50}$ polyalkyleneoxy" described herein is not particularly limited, and an alkylene group in a repeating unit may be linear or branched. Examples of the "divalent $C_{2-50}$ polyalkyleneoxy" include a divalent $C_{2-50}$ polyethyleneoxy group, a $C_{3-48}$ polypropyleneoxy group, and a $C_{3-48}$ polybutyleneoxy group. Each of these groups may be linked to another group via an oxygen atom or a carbon atom. Examples of the $C_{2-50}$ polyethyleneoxy group include -$O(CH_2CH_2O)_{1-25}$-, -$(CH_2CH_2O)_{1-25}$-, -$(OCH_2CH_2)_{1-25}$-, and -$(CH_2CH_2O)_{1-24}$-$(CH_2CH_2)$-.

The term "salt substance" described herein is not particularly limited as long as the salt substance is inorganic matter soluble in water. Examples thereof include: calcium salts such as calcium chloride and calcium phosphate; magnesium salts such as magnesium sulfate and magnesium chloride; aluminum salts such as aluminum sulfate and aluminum chloride; potassium salts such as potassium sulfate, potassium carbonate, potassium nitrate, potassium chloride, potassium bromide, and potassium iodide; sodium salts such as sodium bicarbonate, sodium carbonate, sodium sulfate, sodium nitrate, sodium chloride, sodium bromide, sodium iodide, sodium silicate, trisodium phosphate, disodium phosphate, sodium borate, sodium acetate, and sodium citrate; and lithium salts such as lithium chloride, lithium bromide, lithium iodide, and lithium carbonate, and preferably include sodium chloride, trisodium phosphate, disodium phosphate, potassium chloride, calcium chloride, and magnesium chloride.

The term "antigen" described herein refers to a substance that initiates immunity. The antigen is, for example, a substance that can induce the activation of lymphocytes such as T cells or B cells through presentation by antigen-presenting cells in lymph node or the spleen. The term "antigenic protein" refers to an antigen which is a protein. The "antigenic peptide" refers to an antigen which is a peptide. For example, the antigenic peptide is a portion of an amino acid sequence contained in the antigenic protein and comprises a T cell recognition epitope. A plurality of T cell recognition epitopes may be combined.

The term "adjuvant" described herein refers to a substance that potentiates immune reaction in a subject by administration to the subject in combination with a vaccine containing an antigen.

Hyaluronic acid derivative comprising repeating unit represented by formula (I)

In one aspect, the hyaluronic acid derivative comprising a repeating unit represented by the formula (I) is composed substantially of (1) a repeating unit of the formula (I); or (2) repeating units of the formula (I) and the formula (IIIc).

When the hyaluronic acid derivative comprising a repeating unit represented by the formula (I) comprises two or more repeating units of the formula (I), the repeating units may be the same or different. The hyaluronic acid derivative may be modified at a position other than the repeating unit of the formula (I). For example, a hydroxy group may be converted to -O($C_{1-6}$ alkyl), -O(formyl) or -O($C_{1-6}$ alkylcarbonyl), etc., and a carboxyl group may be converted to an amide group or an ester group or may form a salt.

According to one aspect, the group -Z-N($R^a$)Y-$X^1$ in the formula (I) is selected from groups represented by the following formulas:

-NH-$(CH_2)_{mz}$-NH-R;

-NH-$(CH_2)_{mz}$-NH-COO-R;

-NH-$(CH_2CH_2O)_m$-$CH_2CH_2$-NH-COO-R;

-NH-$(CH_2)_{mz}$-COO-R;

-NH-$(CH_2CH_2O)_m$-$CH_2CH_2$-COO-R;

-NH-$(CH_2)_{mz}$-O-COO-R;

-NH-$(CH_2CH_2O)_m$-$CH_2CH_2$-O-COO-R;

-NH-$(CH_2)_{mz}$-S-R;

-NH-$(CH_2CH_2O)_m$-$CH_2CH_2$-S-R;

-NH-$(CH_2)_{mz}$-O-CO-CH($R^{10}$)-$CH_2$-S-R;

-NH-$(CH_2)_{mz}$-NHCO-CH($R^{10}$)-$CH_2$-S-R;

-NH-$(CH_2CH_2O)_m$-$CH_2CH_2$-NHCO-CH($R^{10}$)-$CH_2$-S-R;

-NH-$(CH_2CH_2O)_m$-$CH_2CH_2$-O-CO-CH($R^{10}$)-$CH_2$-S-R;

-NH-$(CH_2)_{mz}$-S-S-R;

and

-Z-N$R^a$-Y-N$R^b$-COO-R

wherein mz is an integer of 2 to 30, $R^{10}$ is a hydrogen atom or a methyl group, and R and m are as already defined herein.

The group is preferably a group selected from

-NH-$(CH_2)_{mz}$-NH-COO-R;

-NH-$(CH_2CH_2O)_m$-$CH_2CH_2$-NH-COO-R;

and

-NH-$(CH_2)_{mz}$-S-S-R

wherein mz, R, and m are as already defined herein.

In a preferred aspect, Z in the formula (I) is a direct bond. In one aspect, when Z in the formula (I) is a peptide linker, $X^1$ is -N$R^b$-COO-R.

Specific examples of Y in the formula (I) include $-CH_2CH_2O-CH_2CH_2-S-S-CH_2CH_2O-CH_2CH_2-$, $-(CH_2CH_2O)_2-CH_2CH_2-S-S-CH_2CH_2O-CH_2CH_2-$, $-CH_2CH_2O-CH_2CH_2-S-S-(CH_2CH_2O)_2-CH_2CH_2-$ and $-(CH_2CH_2O)_2-CH_2CH_2-S-S-(CH_2CH_2O)_2-CH_2CH_2-$.

$Y^a$ in the formula (I) is preferably $-CH_2-$ and $-CH_2-CH_2-$.

$Y^b$ in the formula (I) is preferably $-CH_2-CH_2-$, $-CH(CH_3)CH_2-$, 2-butene-1,4-diyl, hepta-2,4-diene-1,6-diyl or octa-2,4,6-triene-1,8-diyl, more preferably $-CH_2-CH_2-$ or $-CH(CH_3)CH_2-$.

In one aspect, Z in the formula (I) is a peptide linker represented by $-NH-[CH(-Z^a)-CONH]_{n-1}-CH(-Z^a)-CO-$, wherein n is an integer of 2 to 30, and each $Z^a$ independently represents a substituent in an a-amino acid represented by $H_2N-CH(-Z^a)-COOH$. The peptide linker binds at its N terminus to a carboxyl group of the glucuronic acid moiety and binds at its C terminus to the group $-N(-R^a)-Y-X^1$. Examples of the amino acids that can be used as amino acid residues of the peptide linker include a-amino acids, for example, natural (L) amino acids such as alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine, and their D forms. Every a-amino acid including synthesized amino acids can be used. Specifically, examples of $Z^a$ include $-CH_3$, $H_2NC(NH)NH(CH_2)_3-$, and $H_2NCOCH_2-$. n Z may be the same or different. n is an integer of 2 to 30, preferably 2 to 10, more preferably 2 to 4. Preferred examples of the peptide linker include -Gly-Phe-Leu-Gly-, -Asn-Phe-Phe-, -Phe-Phe-, and -Phe-Gly-.

Specific examples of the group $-Z-N(R^a)Y-X^1$ in the formula (I) include $-NH-(CH_2)_2-NH-CO-$cholesteryl, $-NH-(CH_2)_4-NH-(CH_2)_3-NH-(CH_2)_3-NH-COO-$cholesteryl, $-NH-(CH_2)_3-NH-(CH_2)_4-NH-(CH_2)_3-NH-COO-$cholesteryl, $-NH-(CH_2)_4-NH-(CH_2)_3-NH-COO-$cholesteryl, $-NH-(CH_2)_4-N(-(CH_2)_3-NH_2)-COO-$cholesteryl, $-NH-(CH_2)_3-NH-(CH_2)_4-N(-(CH_2)_3-NH_2)-COO-$cholesteryl, $-NH-(CH_2)_3-NH-(CH_2)_4-N(-(CH_2)_3-NH-(CH_2)_3-NH_2)-COO-$cholesteryl, $-NH-(CH_2)_3-NH-(CH_2)_4-N(-(CH_2)_3-NH_2)-CO-NH-$cholesteryl, $-NH-(CH_2)_3-NH-(CH_2)_4-N(-(CH_2)_3-NH_2)-CO-$cholesteryl, and-$NH-(CH_2)_3-NH-(CH_2)_4-N(-(CH_2)_3-NH_2)-$cholesteryl. In a preferred aspect of the group $-Z-N(R^a)Y-X^1$, each of $R^a$, $R^b$ and $R^c$ is a hydrogen atom, Y is linear $C_{2-30}$ alkylene or $-(CH_2CH_2O)_m-CH_2CH_2-$, and $Y^a$ is linear $C_{1-5}$ alkylene, or $Y^b$ is linear $C_{2-8}$ alkylene or linear $C_{2-8}$ alkenylene.

In one aspect, in the group $Z-N(R^a)Y-X^1$, Z is a direct bond, $R^a$ is a hydrogen atom, Y is, for example, $C_{2-12}$ alkylene, preferably $C_{2-6}$ alkylene, more preferably $C_6$ alkylene, $X^1$ is $-NR^b-COO-R$, $R^b$ is a hydrogen atom, and R is a cholesteryl group.

In one aspect, the hyaluronic acid derivative comprising a repeating unit represented by the formula (I) further comprises a repeating unit represented by the formula (IIIc). When two or more repeating units represented by the formula (IIIc) are contained, the repeating units may be the same or different.

$Q^+$ in the formula (IIIc) is not particularly limited as long as $Q^+$ is a counter cation that forms a salt with a carboxyl group in water. Divalent or higher valent $Q^+$ forms a salt with a plurality of carboxyl groups according to the valence. Examples of the counter cation include: metal ions such as lithium ions, sodium ions, rubidium ions, cesium ions, magnesium ions, and calcium ions; and ammonium ions represented by the formula: $N^+R^jR^kR^1R^m$ (wherein $R^j$, $R^k$, $R^1$ and $R^m$ are each independently selected from a hydrogen atom and $C_{1-6}$ alkyl), and preferably include sodium ions, potassium ions, and tetraalkylammonium ions (e.g., tetra-n-butylammonium ions). $R^j$, $R^k$, $R^1$ and $R^m$ are preferably the same groups selected from $C_{1-6}$ alkyl, and are preferably n-butyl groups.

All of the groups $R^1$, $R^2$, $R^3$, and $R^4$ in the formula (I), and $R^{1a}$, $R^{2a}$, $R^{3a}$ and $R^{4a}$ in the formula (IIIc) are preferably hydrogen atoms. Both $R^a$ and $R^b$ are preferably hydrogen atoms.

The group $R^5$ in the formula (I) is preferably acetyl.

In one aspect, the hyaluronic acid derivative comprising a repeating unit represented by the formula (I) is composed substantially of repeating units of the formulas (I) and (IIIc). In the hyaluronic acid derivative, for example, 80% or more, preferably 90% or more, more preferably 95% or more, of disaccharide repeating units consisting of D-glucuronic acid and N-acetylglucosamine contained in the derivative are repeating units of the formula (I) or (IIIc). In one aspect, the hyaluronic acid derivative is composed only of repeating units represented by the formula (I) and the formula (IIIc).

Y defined in the formula (I) may be, for example, $-(CH_2)_{na}-$ (wherein na is selected from integers of 2 to 20, preferably 2 to 15, more preferably 2 to 12) and is preferably $-(CH_2)_2-$, $-(CH_2)_6-$, $-(CH_2)_8-$ or $-(CH_2)_{12}-$, more preferably $-(CH_2)_6-$. Such Y is preferred from the viewpoint of precipitation formation and stable dispersion mentioned later.

According to one aspect, in the hyaluronic acid derivative comprising a repeating unit represented by the formula (I), the rate of introduction of the hydrophobic group to disaccharide repeating units present in the derivative is, for example, 1 to 50%, preferably 7 to 50%, more preferably 17 to 50%, still more preferably 20 to 45%. When the rate of introduction falls within the range described above, the hyaluronic acid derivative comprising a repeating unit represented by the formula (I) can efficiently form a complex with the antigen in a solution.

In this context, the rate of introduction of the hydrophobic group is calculated according to the following expression:

[Expression 1]

$$\text{(Rate of introduction of hydrophobic group)} = \frac{\text{(The number of disaccharide repeating units having an introduced hydrophobic group)}}{\text{(The number of disaccharide repeating units present)}} \times 100$$

In this context, the "disaccharide repeating units present in the derivative" include a repeating unit of the formula (I) wherein a hydrophobic group is introduced by the conversion of a carboxyl group to an amide group, and a repeating unit of the formula (IIIc) wherein no hydrophobic group is introduced. The rate of introduction can be controlled by reaction conditions, for example, the ratio of a reagent, and can be determined by, for example, NMR measurement.

The hyaluronic acid derivative comprising a repeating unit represented by the formula (I) is preferably synthesized by using hyaluronic acid composed only of a repeating unit represented by the formula (IIIc) or a derivative thereof as a starting material, wherein when all of $R^{1c}$, $R^{2c}$, $R^{3c}$, and $R^{4c}$ are hydrogen atoms, $R^{5c}$ is acetyl, and $X^c$ is $-O^-Na^+$, a weight-average molecular weight is, for example, 1 kDa to 500 kDa, preferably 3 kDa to 500 kDa, more preferably 5 kDa to 200 kDa. In one aspect, the weight-average molecular weight of the starting material is preferably 1 kDa to 1000 kDa, more preferably 3 kDa to 200 kDa, from the viewpoint of complex formation with the antigen. The weight-average molecular weight of the starting material is preferably 1 kDa to 1000 kDa, more preferably 3 kDa to 500 kDa, still more preferably 5 kDa to 200 kDa, even more preferably 5 kDa to 150 kDa, from the viewpoint of the transfer of the complex to lymph node.

In general, hyaluronic acid and a derivative thereof are difficult to obtain as single products. Therefore, their molecular weights are calculated as number-average molecular weights or weight-average molecular weights. In the present invention, a weight-average molecular weight is calculated. Various methods known in the art, for example, a light scattering method, an osmotic pressure method, and a viscosity method, described in Seiichi Nakahama, et al., "Essential Polymer Science" (published by Kodansha Ltd., ISBN 4-06-153310-X) can be used as methods for measuring the weight-average molecular weight. A viscosity-average molecular weight described herein can also be measured by a method usually used in the technical field to which the present invention belongs, such as use of a Ubbelohde viscometer. In the case of using a commercially available hyaluronic acid and derivative thereof having a clearly specified molecular weight, the clearly specific numeric value may be used as a molecular weight.

In the hyaluronic acid derivative comprising a repeating unit of the formula (I), the hydrophobic group is introduced by the conversion of a carboxyl group of glucuronic acid as one of the saccharides of the disaccharide constituting the repeating unit to an amide group. The *in vivo* kinetics of a formulation to be produced using the hyaluronic acid derivative may be controlled by adjusting the degree of modification of the hyaluronic acid derivative.

When the rate of carboxyl group modification of the glucuronic acid moiety of the hyaluronic acid derivative comprising a repeating unit of the formula (I) is high, binding to a hyaluronic acid receptor including CD44 is suppressed so that the hyaluronic acid derivative serves as a drug carrier (including a vaccine) that remains long in the body. Furthermore, each organ including lymph node, and cells can be targeted by introducing a targeting element to the hyaluronic acid derivative. Examples of the targeting element include target tissue-specific peptides, antibodies, antibody fragments, aptamers, RGD peptides against cancer cells, folic acid, anisamide, transferrin, galactose for the liver, and tocopherol.

The rate of carboxyl group modification with the hydrophobic group, i.e., the rate of introduction of the hydrophobic group, of the glucuronic acid moiety of the hyaluronic acid derivative comprising a repeating unit of the formula (I) is preferably 4 to 60%, more preferably 5 to 50%, still more preferably 7 to 50%, even more preferably 7 to 45%, from the viewpoint of complex formation with the antigen. The rate is preferably 6 to 60%, more preferably 17 to 50%, still more preferably 20 to 50%, even more preferably 20 to 45%, from the viewpoint of the transfer of the complex to lymph node.

The combination of the molecular weight of the starting material and the rate of introduction of the hydrophobic group for the hyaluronic acid derivative comprising a repeating unit represented by the formula (I) is preferably 3 kDa to 500 kDa and 4 to 60%, more preferably 3 kDa to 200 kDa and 6 to 50%, still more preferably 5 kDa to 200 kDa and 6 to 50%, even more preferably 5 kDa to 150 kDa and 6 to 45%, from the viewpoint of complex formation with the antigen. The combination is preferably 3 kDa to 500 kDa and 4 to 60%, more preferably 3 kDa to 200 kDa and 6 to 50%, still more preferably 5 kDa to 200 kDa and 6 to 50%, even more preferably 5 kDa to 150 kDa and 20 to 45%, from the viewpoint of the transfer of the complex to lymph node. The combination is preferably 3 kDa to 200 kDa and 4 to 60%, more preferably 3 kDa to 200 kDa and 6 to 50%, still more preferably 5 kDa to 200 kDa and 6 to 50%, even more preferably 5 kDa to 150 kDa and 17 to 45%, from the viewpoint of stable dispersion. The combination is preferably 5 kDa to 27 kDa and 2 to 50%, more preferably 5 kDa to 27 kDa and 8 to 35%, still more preferably 5 kDa to 18 kDa and 8 to 35%, even more preferably 5 kDa to 18 kDa and 8 to 35%, further preferably 5 kDa to 18 kDa and 15 to 22%, from the viewpoint of improvement in retention in blood. The combination is preferably 5 kDa to 300 kDa and 2 to 30%, more preferably 5 kDa to 50 kDa and 2 to 22%, still more preferably 5 kDa to 27 kDa and 2 to 22%, even more preferably 5

kDa to 27 kDa and 7 to 22%, from the viewpoint of gelation.

Hyaluronic acid derivative comprising repeating unit represented by formula (II)

In one aspect, the hyaluronic acid derivative comprising a repeating unit represented by the formula (II) is composed substantially of (1) a repeating unit of the formula (II); (2) repeating units of the formula (II) and the formula (III); (3) repeating units of the formula (II) and the formula (IIIc); or (4) repeating units of the formula (II), the formula (III), and the formula (IIIc). In the hyaluronic acid derivative, for example, 80% or more, preferably 90% or more, more preferably 95% or more, of disaccharide repeating units consisting of D-glucuronic acid and N-acetylglucosamine contained in the derivative are repeating units of the formula (II), (III), or (IIIc). In one aspect, the hyaluronic acid derivative is composed only of (1) a repeating unit of the formula (II); (2) repeating units of the formula (II) and the formula (III); (3) repeating units of the formula (II) and the formula (IIIc); or (4) repeating units of the formula (II), the formula (III), and the formula (IIIc).

The ratio of a particular disaccharide unit to disaccharide repeating units present in the hyaluronic acid derivative comprising a repeating unit represented by the formula (II) means the ratio of the particular disaccharide unit to all disaccharide units contained in a given amount of the hyaluronic acid derivative comprising a repeating unit represented by the formula (II), which is a polysaccharide having disaccharide units as repeating units.

In the formula (II) which represents a disaccharide unit contained in the hyaluronic acid derivative comprising a repeating unit represented by the formula (II), all of $R^{1a}$, $R^{2a}$, $R^{3a}$, and $R^{4a}$ are preferably hydrogen atoms. $R^{5a}$ is preferably a hydrogen atom or $C_{1-6}$ alkylcarbonyl, more preferably a hydrogen atom or acetyl, still more preferably acetyl. In the formulas (III) and (IIIc) which represent disaccharide units contained in the hyaluronic acid derivative comprising a repeating unit represented by the formula (II), all of $R^{1b}$, $R^{2b}$, $R^{3b}$ and $R^{4b}$, and $R^{1c}$, $R^{2c}$, $R^{3c}$ and $R^{4c}$ are preferably hydrogen atoms. Each of $R^{5b}$ and $R^{5c}$ is preferably a hydrogen atom or $C_{1-6}$ alkylcarbonyl, more preferably a hydrogen atom or acetyl. Still more preferably, both of these moieties are acetyl.

Specific examples of $R^{aa}$ in the formula (II) include a hydrogen atom, methyl, hydroxymethyl, 1-hydroxyethyl, carbamoylmethyl, carboxymethyl, 1-methylpropyl, 2-methylpropyl, isopropyl, 2-carboxyethyl, 2-methylthioethyl, 2-carbamoylethyl, phenylmethyl, (4-hydroxyphenyl)methyl, and indol-3-ylmethyl.

When the group $-CHR^{aa}-$ serves as asymmetric center, respective optical isomers and their mixtures are also included therein. $H_2N-CHR^{aa}-COOH$ (amino acid) preferably forms an L configuration (natural).

In the formula (II), $R^{6a}$, $R^7$, $R^8$, and $R^9$ are each independently, for example, a hydrogen atom or methyl. Preferably, all of these moieties are hydrogen atoms.

The form of the group $-CHR^{aa}-CO-X^{1a}$ in the formula (II) is, for example, a group $-CHR^{aa}-COOH$. Specific examples of the group include the following groups.

[Formula 6]

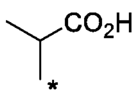
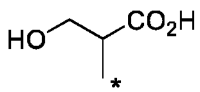
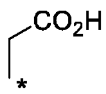
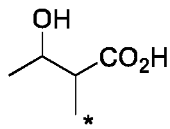
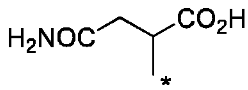
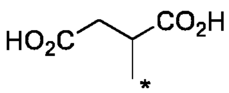
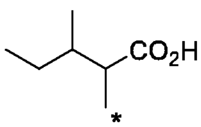
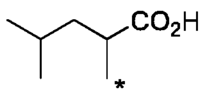
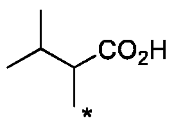
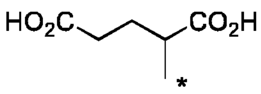
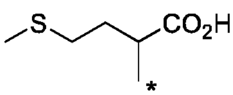
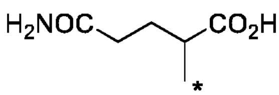
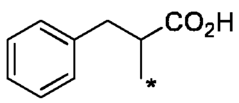
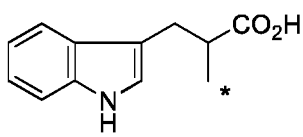

In this context, each asterisk represents a binding position to $-NR^{6a}-$ (the same holds true for the description below).

Preferred examples of the group -$CHR^{aa}$-COOH include the following groups.

[Formula 7]

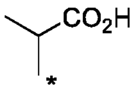
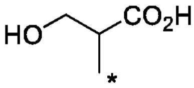
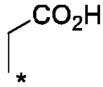
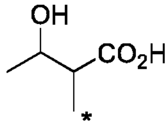
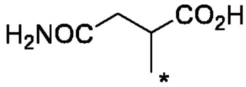
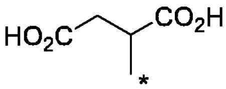
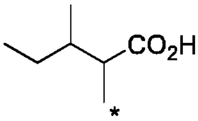
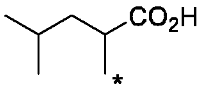
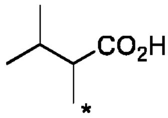
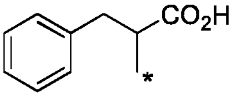
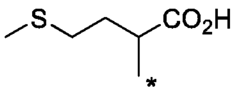
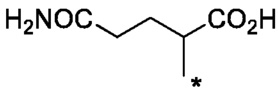

Preferred examples of the group -$CHR^{aa}$-COOH include the following groups.

[Formula 8]

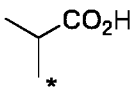
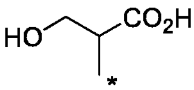
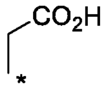
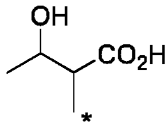
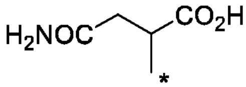
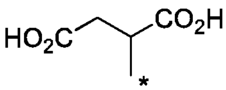
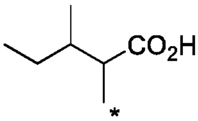
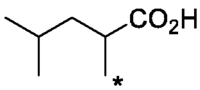
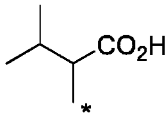
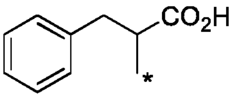

Preferred examples of the group -$CHR^{aa}$-COOH include the following groups.

[Formula 9]

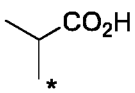
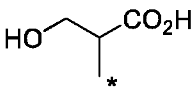
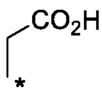
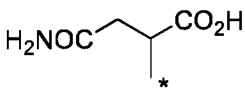
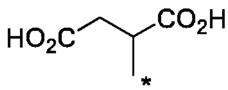
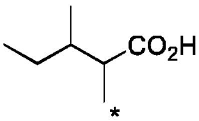
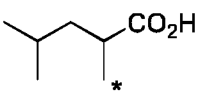
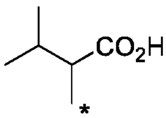
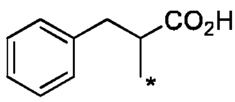
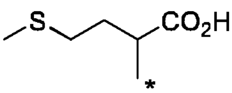
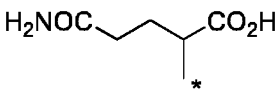

Preferred examples of the group -$CHR^{aa}$-COOH include the following groups.

[Formula 10]

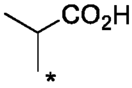 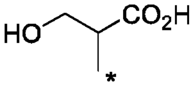 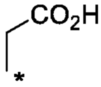 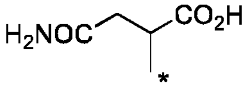 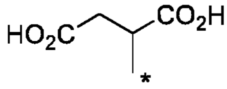

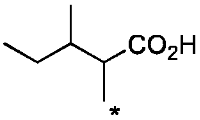 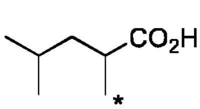 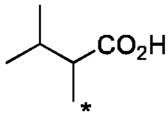 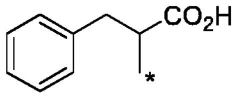

Preferred examples of the group -$CHR^{aa}$-COOH include the following groups from the viewpoint of the delivery of the complex to lymph node.

[Formula 11]

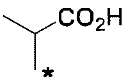 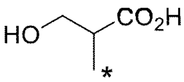 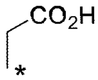 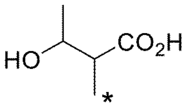 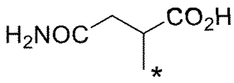 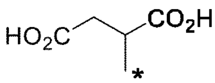

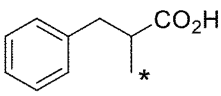 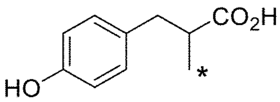 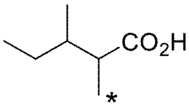 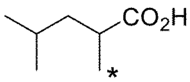 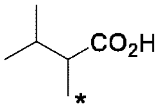

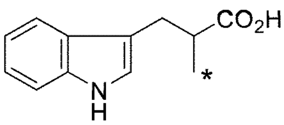 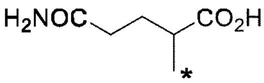 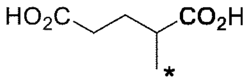

More preferred examples thereof include the following groups.

[Formula 12]

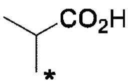 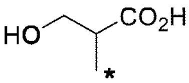 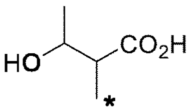 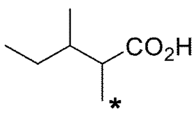 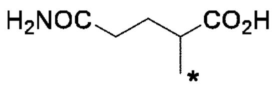

Still more preferred examples thereof include the following groups.

[Formula 13]

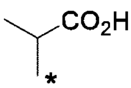 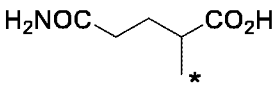

All the groups -$CHR^{aa}$-COOH mentioned above may be converted partially or wholly to a group -$CHR^{aa}$-CONH-$Z^1$-$Z^2$. Examples of the group -$Z^1$-$Z^2$ are as mentioned later.

Another form of the group -$CHR^{aa}$-CO-$X^{1a}$ in the formula (II) is, for example, a group -$CHR^{aa}$-$CONH_2$. Specific examples of the group include the following groups.

[Formula 14]

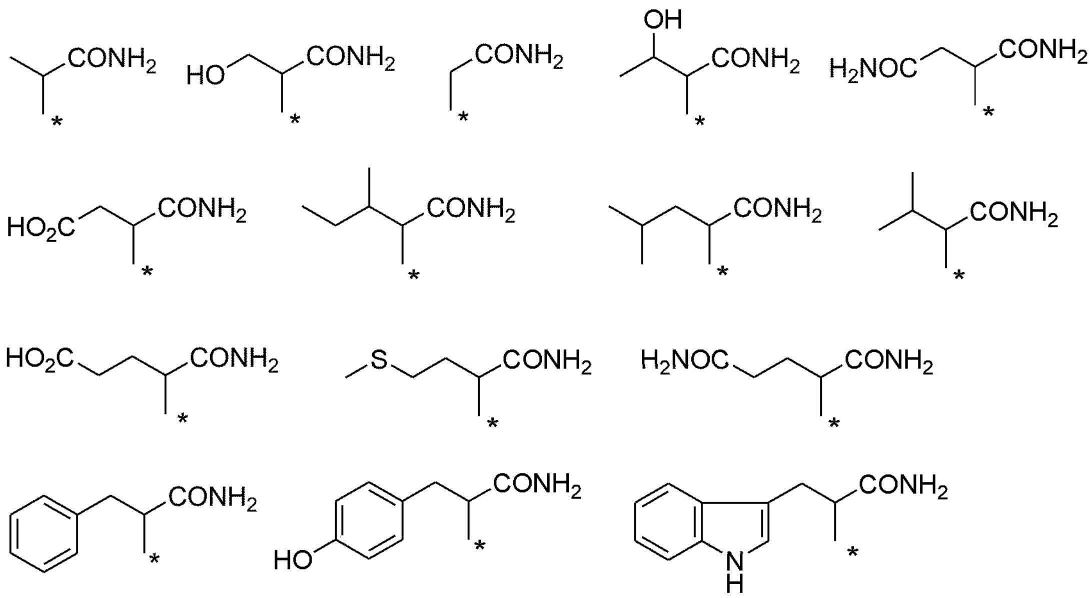

Preferred examples of the group -$CHR^{aa}$-$CONH_2$ include the following groups.

[Formula 15]

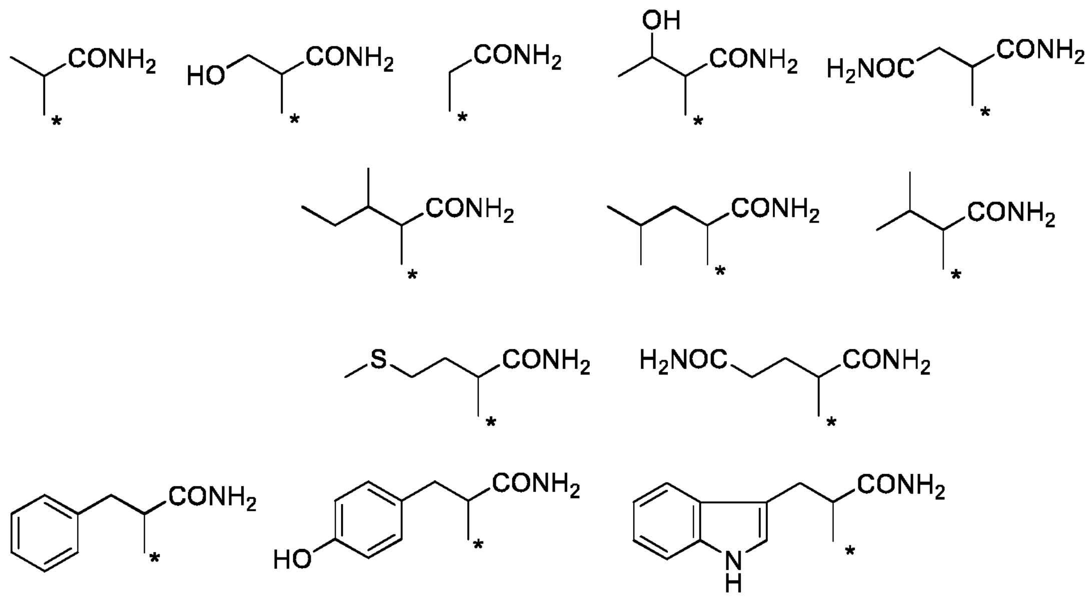

Preferred examples of the group -$CHR^{aa}$-$CONH_2$ include the following groups.

[Formula 16]

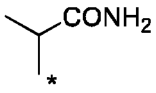 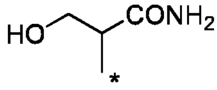 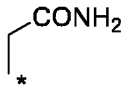 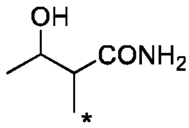 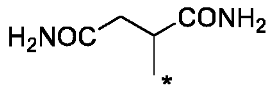

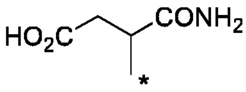 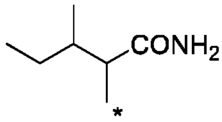 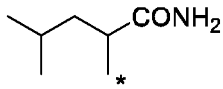 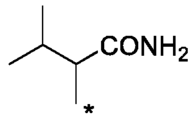

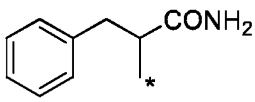 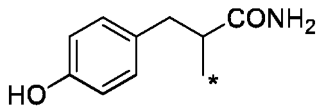

Preferred examples of the group $-CHR^{aa}-CONH_2$ include the following groups.

[Formula 17]

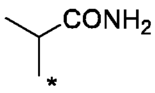 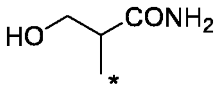 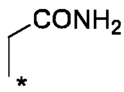 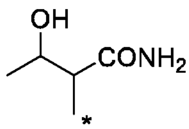 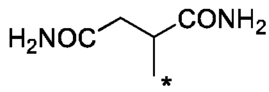

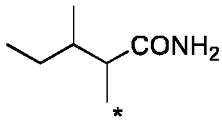 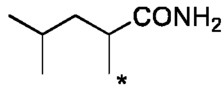 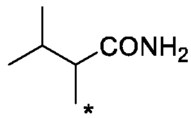

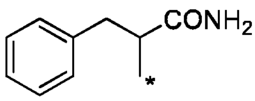 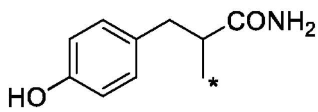

Preferred examples of the group $-CHR^{aa}-CONH_2$ include the following groups.

[Formula 18]

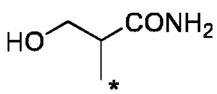 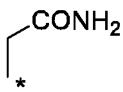 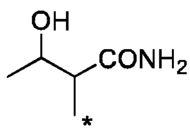 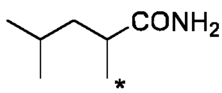 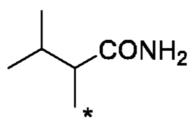

These groups are also preferred groups from the viewpoint of having both characteristics of biodegradability and retention in blood.

Preferred examples of the group $-CHR^{aa}-CONH_2$ also include the following groups from the viewpoint of having both characteristics of biodegradability and retention in blood.

[Formula 19]

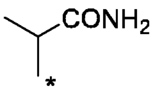 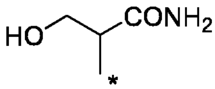 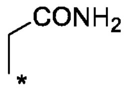 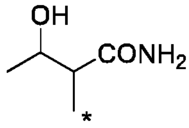 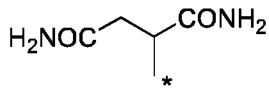

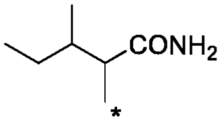 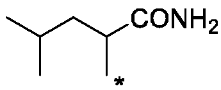 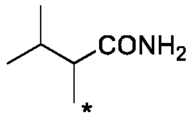

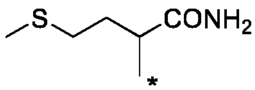 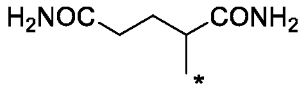

More preferred examples of the group $-CHR^{aa}-CONH_2$ also include the following groups from the viewpoint of having both characteristics of biodegradability and retention in blood.

[Formula 20]

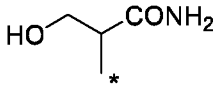 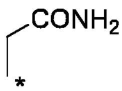 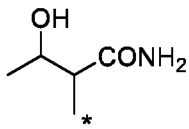 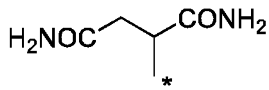

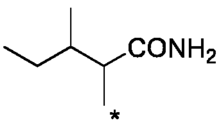 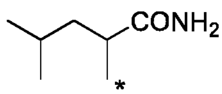 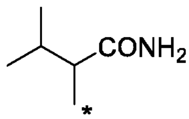

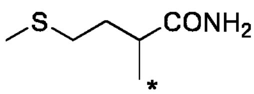

Preferred examples of the group $-CHR^{aa}-CONH_2$ include the following groups from the viewpoint of more suitable dispersibility in pure water.

[Formula 21]

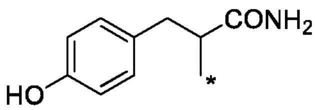 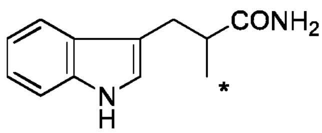

These two groups are also preferred examples from the viewpoint of a base material for an injection formulation for continuous subcutaneous administration.

Preferred examples of the group $-CHR^{aa}-CONH_2$ include the following group from the viewpoint of a base material for an injection formulation for continuous subcutaneous administration.

[Formula 22]

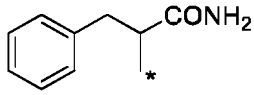

$R^7$ is more preferably a hydrogen atom or methyl, still more preferably a hydrogen atom.

The carboxy defined in the formulas (II), (III) and (IIIc) may form a salt represented by the formula $-COO^-Q^+$. In this context, $Q^+$ is not particularly limited as long as $Q^+$ is a counter cation that forms a salt with carboxy in water. Divalent or higher valent $Q^+$ forms a salt with a plurality of carboxy groups according to the valence. Examples of the

counter cation include: metal ions such as lithium ions, sodium ions, rubidium ions, cesium ions, magnesium ions, and calcium ions; and ammonium ions represented by the formula: $N^+R^jR^kR^1R^m$ (wherein $R^j$, $R^k$, $R^1$ and $R^m$ are each independently selected from a hydrogen atom and $C_{1-6}$ alkyl), and preferably include sodium ions, potassium ions, and tetraalkylammonium ions (e.g., tetra-n-butylammonium ions). $R^j$, $R^k$, $R^1$ and $R^m$ are preferably the same groups selected from $C_{1-6}$ alkyl, and are preferably n-butyl groups.

An alternative form of the group $-CHR^{aa}-CO-X^{1a}$ in the formula (II) is, for example, a group $-CHR^{aa}-CONH-Z^1-Z^2$. Specific examples of the group include the following groups.

[Formula 23]

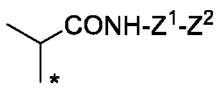
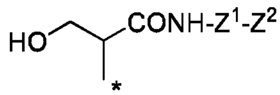
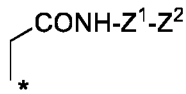
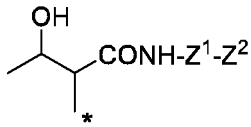
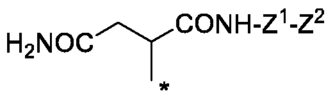
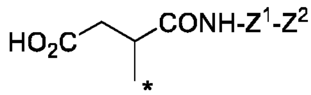
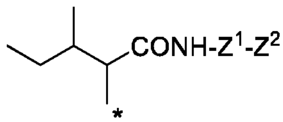
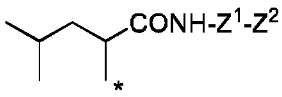
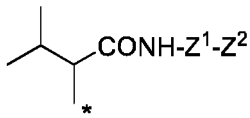
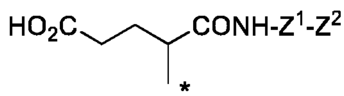
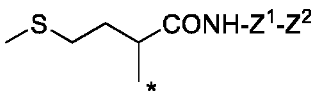
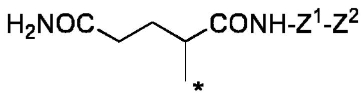
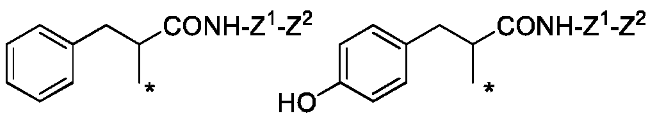
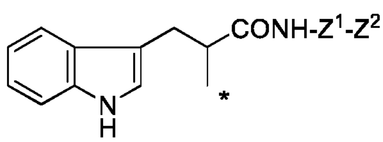

Other specific examples of the group include the following groups.

[Formula 24]

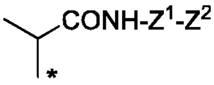 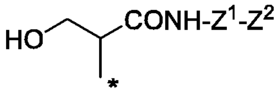 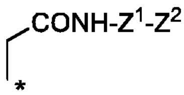 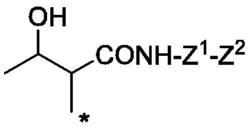

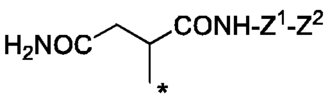 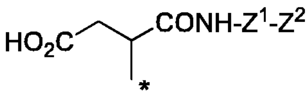 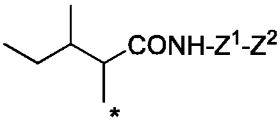

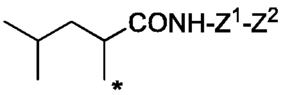 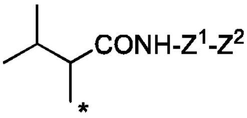 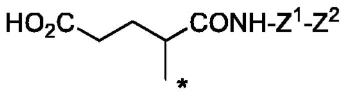

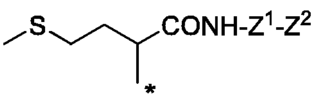 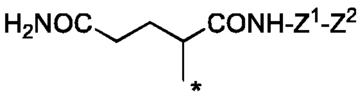

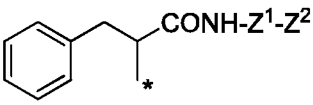 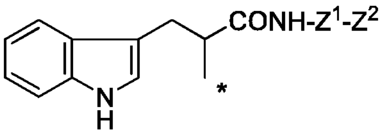

Preferred examples of the group $-CHR^{aa}-CONH-Z^1-Z^2$ include the following groups.

[Formula 25]

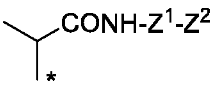 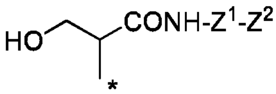 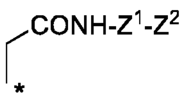 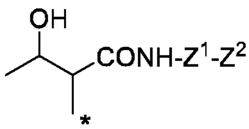

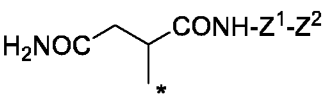 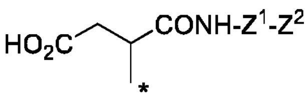 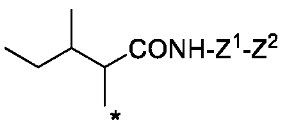

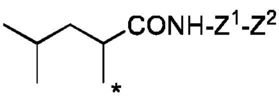 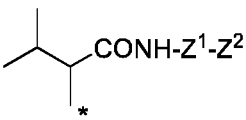

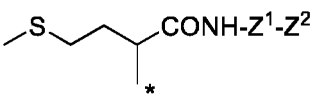 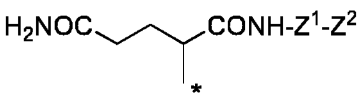

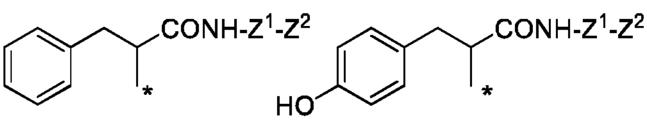 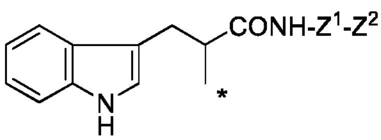

Preferred examples of the group $-CHR^{aa}-CONH-Z^1-Z^2$ include the following groups.

[Formula 26]

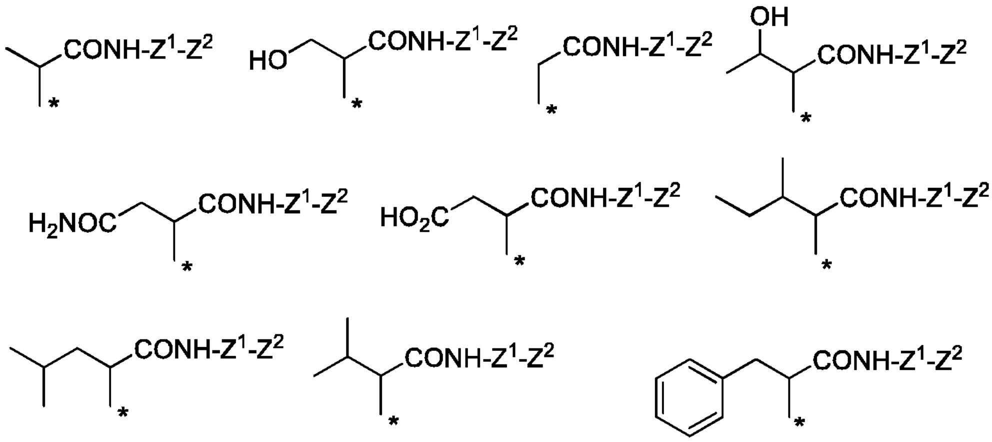

Preferred examples of the group $-CHR^{aa}-CONH-Z^1-Z^2$ include the following groups.

[Formula 27]

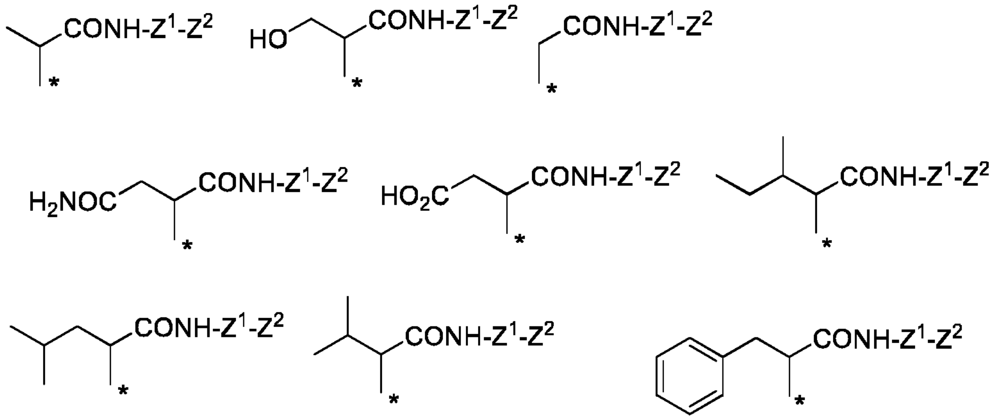

Preferred examples of the group $-CHR^{aa}-CONH-Z^1-Z^2$ include the following groups from the viewpoint of the delivery of the complex to lymph node.

[Formula 28]

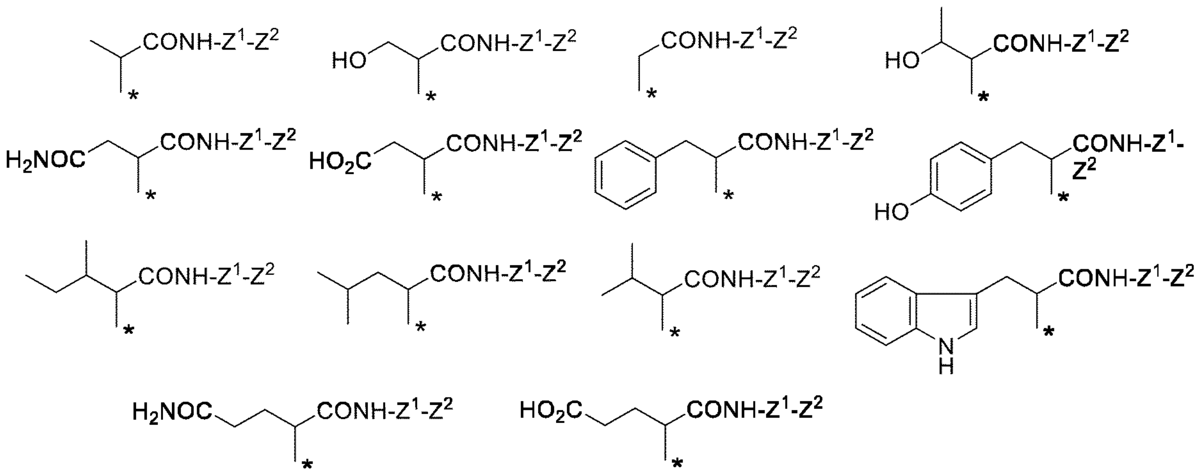

More preferred examples of the group include the following groups.

[Formula 29]

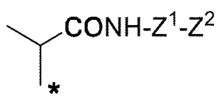 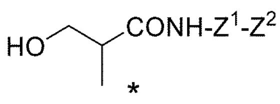 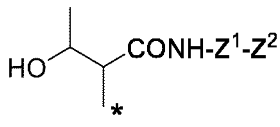

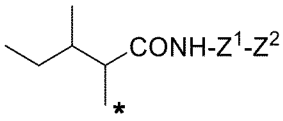 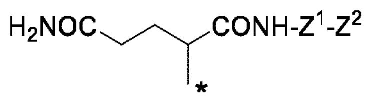

Still more preferred examples thereof include the following groups.

[Formula 30]

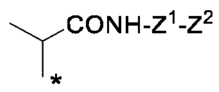 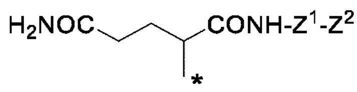

Preferred examples of the group -$CHR^{aa}$-CONH-$Z^1$-$Z^2$ include the following groups from the viewpoint of having both characteristics of biodegradability and retention in blood.

[Formula 31]

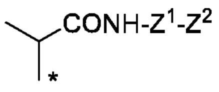 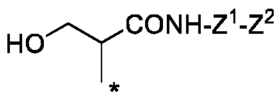 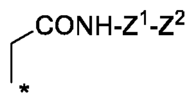 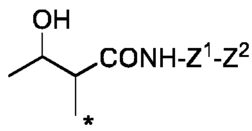

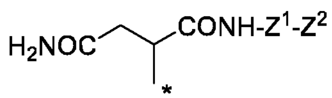 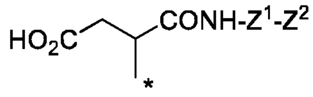 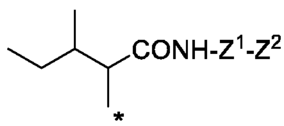

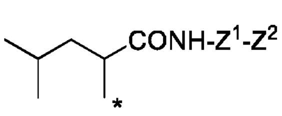 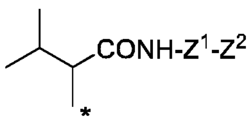

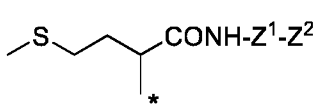 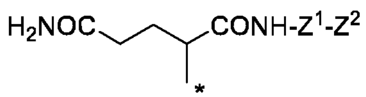

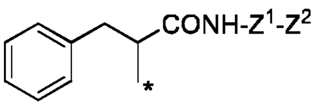

Examples of the group -$Z^1$-$Z^2$ include a group -($C_{2-10}$ alkylene)-NH-COO-$Z^3$. Examples thereof also include a group -($C_{2-12}$ alkylene)-NH-COO-$Z^3$. In this context, examples of the $C_{2-12}$ alkylene preferably include -$(CH_2)_2$-, -$(CH_2)_6$-, -$(CH_2)_8$-, -$(CH_2)_{10}$- and -$(CH_2)_{12}$- and more preferably include -$(CH_2)_2$- and -$(CH_2)_6$-. Examples of the group -$Z^1$-$Z^2$ include a group -$(CH_2CH_2O)_{ma}$-$CH_2CH_2$-NH-$Z^3$. In this context, ma is preferably 1 to 20, more preferably 1 to 10, still more preferably 1 to 3. Specific examples of preferred ma include 2. Examples of the group -$Z^1$-$Z^2$ preferably include a group -(hexane-1,6-diyl)-NH-COO-$Z^3$, a group -(ethane-1,2-diyl)-NH-COO-$Z^3$ and a group -$(CH_2CH_2O)_2$-$CH_2CH_2$-NH-$Z^3$, more preferably include a group -(hexane-1,6-diyl)-NH-COO-cholesteryl, a group -(ethane-1,2-diyl)-NH-COO-cholesteryl and a group-$(CH_2CH_2O)_2$-$CH_2CH_2$-NH-cholanoyl, and still more preferably include a group -(hexane-1,6-diyl)-NH-COO-cholesteryl. Examples of $Z^1$, $Z^2$, and the group -$Z^1$-$Z^2$ also include those corresponding to Y, $X^1$, and the group-Y-$X^1$, respectively, in the hyaluronic acid derivative comprising a repeating unit represented by the formula (I). Examples

of the group $-CO-NR^{ca}-Z^3$ and the group $-O-CO-NR^{ca}-Z^3$ include respective groups wherein $R^{ca}$ is a hydrogen atom.

In one aspect, in the hyaluronic acid derivative comprising a repeating unit represented by the formula (II), the group $X^{1a}$ is $-NR^9-Z^1-Z^2$, $R^9$ is a hydrogen atom, $Z^1$ is, for example, $C_{2-12}$ alkylene, preferably $C_{2-6}$ alkylene, more preferably $C_6$ alkylene, $Z^2$ is $-NR^{ba}-COO-Z^3$, $R^{ba}$ is a hydrogen atom, and $Z^3$ is a steryl group; and the group $R^{aa}$ is a hydrogen atom, or $C_{1-6}$ alkyl, wherein the alkyl is optionally substituted by one or more groups each independently selected from hydroxy, carboxy, carbamoyl, $C_{1-6}$ alkylthio, aryl, and heteroaryl, wherein the aryl is optionally substituted by one or more hydroxy groups.

Specific examples of the group $R^{aa}$ preferably include methyl, hydroxymethyl, a hydrogen atom, 1-hydroxyethyl, carbamoylmethyl, carboxymethyl, benzyl, (4-hydroxyphenyl)methyl, 1-methylpropyl, 2-methylpropyl, isopropyl, indol-3-ylmethyl, 2-carbamoylethyl and 2-carboxyethyl, more preferably include methyl, hydroxymethyl, 1-hydroxyethyl, 1-methylpropyl and 2-carbamoylethyl, still more preferably include methyl and 2-carbamoylethyl, from the viewpoint of the delivery of the complex to lymph node.

In one aspect, the hyaluronic acid derivative comprising a repeating unit represented by the formula (II) preferably also comprises a repeating unit represented by the formula (III). In a more preferred aspect, $X^{1a}$ in the formula (II) and $X^2$ in the formula (III) are the same. In one aspect, the hyaluronic acid derivative comprising a repeating unit represented by the formula (II) may comprise a repeating unit represented by the formula (II), a repeating unit represented by the formula (III) and a repeating unit represented by the formula (IIIc), wherein $X^{1a}$ is $-NR^9-Z^1-Z^2$.

According to a further alternative aspect, in the hyaluronic acid derivative comprising a repeating unit represented by the formula (II), the ratio of the repeating unit of the formula (II) and/or the formula (III) having a group $-NR^9-Z^1-Z^2$ (hereinafter, also referred to as a hydrophobic group) to disaccharide repeating units present (rate of introduction of the hydrophobic group) may be 3 to 50%.

In this context, the rate of introduction of the hydrophobic group is calculated according to the following expression:

[Expression 2]

$$\text{(Rate of introduction of hydrophobic group)} = \frac{\text{(The number of disaccharide repeating units having an introduced hydrophobic group)}}{\text{(The number of disaccharide repeating units present)}} \times 100$$

In this context, the "disaccharide repeating units present in the derivative" include repeating units of the formula (II) and the formula (III), and a repeating unit of the formula (IIIc). The rate of introduction can be controlled by reaction conditions, for example, the ratio of a reagent, and can be determined by, for example, NMR measurement.

The rate of introduction of the hydrophobic group in the hyaluronic acid derivative comprising a repeating unit represented by the formula (II) is, for example, 3 to 50%, preferably 5 to 40%, more preferably 5 to 35%, still more preferably 5 to 25%, even more preferably 5 to 20%, further preferably 5 to 10%. The rate is preferably 3 to 60%, more preferably 7 to 50%, still more preferably 18 to 45%, even more preferably 20 to 35%, from the viewpoint of complex formation with the antigen. The rate is preferably 3 to 60%, more preferably 7 to 50%, still more preferably 18 to 45%, even more preferably 20 to 35%, from the viewpoint of the transfer of the complex to lymph node.

In one aspect, in the hyaluronic acid derivative comprising a repeating unit represented by the formula (II), $X^{1a}$ in the formula (II) is $-NR^9-Z^1-Z^2$ In this case, the ratio of the disaccharide unit of the formula (II) to disaccharide repeating units present in the hyaluronic acid derivative is, for example, 70% or more, preferably 75% or more, more preferably 90% or more, from the viewpoint of having both characteristics of biodegradability and retention in blood. The upper limit can be 100% or less. The range of the ratio is, for example, 70 to 100%, preferably 75 to 100%, more preferably 90 to 100%. The ratio is, for example, 10% or more, preferably 20% or more, more preferably 50% or more, still more preferably 70% or more, even more preferably 90% or more, from the viewpoint of having characteristics of complex formation with the antigen. The upper limit can be 100% or less. The range of the ratio is, for example, 10 to 100%, preferably 20 to 100%, more preferably 50 to 100%, still more preferably 70 to 100%, even more preferably 90 to 100%. The ratio is, for example, 10% or more, preferably 20% or more, more preferably 50% or more, still more preferably 70% or more, even more preferably 90% or more, from the viewpoint of having characteristics of transfer of the complex to lymph node. The upper limit can be 100% or less. The range of the ratio is, for example, 10 to 100%, preferably 20 to 100%, more preferably 50 to 100%, still more preferably 70 to 100%, even more preferably 90 to 100%. The hyaluronic acid derivative comprising a repeating unit represented by the formula (II) may further comprise a repeating unit represented by the formula (III).

In one aspect, the hyaluronic acid derivative comprising a repeating unit represented by the formula (II) does not comprise a repeating unit represented by the formula (II) wherein $X^1$ is $-NR^9-Z^1-Z^2$ In this case, the sum of the ratio of the repeating unit represented by the formula (II), and the ratio of the repeating unit represented by the formula (III) to disaccharide repeating units present is, for example, 70 to 100%, preferably 80 to 100%, more preferably 90 to 100%.

The sum is, for example, 7 to 100%, preferably 20 to 100%, more preferably 30 to 100%, from the viewpoint of complex formation with the antigen. The sum is, for example, 20 to 100%, preferably 30 to 100%, more preferably 70 to 100%, from the viewpoint of the transfer of the complex to lymph node.

In this context, in the hyaluronic acid derivative comprising a repeating unit represented by the formula (II), the ratio of the repeating unit represented by the formula (III) to disaccharide repeating units present is preferably 3 to 50%, more preferably 5 to 40%, still more preferably 5 to 35%, even more preferably 5 to 25%, further preferably 5 to 20%, still further preferably 5 to 10%. The ratio is preferably 3 to 60%, more preferably 7 to 50%, still more preferably 18 to 45%, even more preferably 20 to 35%, from the viewpoint of complex formation with the antigen. The ratio is preferably 3 to 60%, more preferably 7 to 50%, still more preferably 18 to 45%, even more preferably 20 to 35%, from the viewpoint of the transfer of the complex to lymph node.

In the hyaluronic acid derivative comprising a repeating unit represented by the formula (II), the ratio of the repeating unit represented by the formula (II) to disaccharide repeating units present is preferably 20 to 97%, more preferably 30 to 95%, still more preferably 35 to 95%, even more preferably 45 to 95%, further preferably 50 to 95%, still further preferably 60 to 95%. The ratio is, for example, 7 to 100%, preferably 20 to 100%, more preferably 30 to 100%, from the viewpoint of complex formation with the antigen. The ratio is, for example, 20 to 100%, preferably 30 to 100%, more preferably 70 to 100%, from the viewpoint of the transfer of the complex to lymph node.

Examples of the methods for producing the hyaluronic acid derivative comprising a repeating unit represented by the formula (I) and the hyaluronic acid derivative comprising a repeating unit represented by the formula (II) include methods described in International Publication Nos. WO 2010/053140 and WO 2014/038641.

According to one aspect, a feature of the hyaluronic acid derivative comprising a repeating unit represented by the formula (I) or the hyaluronic acid derivative comprising a repeating unit represented by the formula (II) is that the hyaluronic acid derivative forms a fine particle by association in water. It is considered that spontaneous association occurs in water through the hydrophobic interaction of the introduced hydrophobic group so that a fine particle is formed, though the event is not particularly limited thereto. By exploiting such characteristics, the hyaluronic acid derivative can be used as a carrier for a vaccine, a lymph node delivery carrier, a sustained-release carrier in blood, or a targeting carrier. The particle size of the fine particle is not particularly limited and is, for example, 1 μm or smaller, preferably 500 nm or smaller, more preferably 200 nm or smaller, still more preferably 100 nm or smaller, even more preferably 50 nm or smaller. Examples of the methods for forming the fine particles of the hyaluronic acid derivative comprising a repeating unit represented by the formula (I) and the hyaluronic acid derivative comprising a repeating unit represented by the formula (II) include methods described in International Publication Nos. WO 2010/053140 and WO 2014/038641.

Hyaluronic acid or a salt thereof or a derivative thereof can be used as a starting material for producing the hyaluronic acid derivative comprising a repeating unit represented by the formula (I) and the hyaluronic acid derivative comprising a repeating unit represented by the formula (II). Examples of the hyaluronic acid salt can include alkali metal salts such as sodium salts, potassium salts, and lithium salts. The salt is particularly preferably a sodium salt which is frequently used as a medicament. The hyaluronic acid or a pharmaceutically acceptable salt thereof can be produced by use of various methods known in the art, such as a method of obtaining organism-derived extracts from cock's comb, under the swine skin, etc., and a biological fermentation method, or may be obtained by purchasing a commercially available product (e.g., from Denka Co., Ltd., Shiseido Co., Ltd., Seikagaku Corp., and R&D Systems, Inc.).

The antigen in the complex formed with the fine particle of the hyaluronic acid derivative comprising a repeating unit represented by the formula (I) or the hyaluronic acid derivative comprising a repeating unit represented by the formula (II) may be released from the complex by the degradation or disruption of the fine particle of the hyaluronic acid derivative, and the replacement of the antigen with a biocomponent such as albumin, after administration. The velocity of this release may be controlled by the rate of introduction of the hydrophobic group, molecular weight, or rate of amino acid introduction of the hyaluronic acid derivative. The antigen release velocity may be controlled by the chemical cross-linking and gelation of the hyaluronic acid derivative according to a method described in International Publication No. WO 2010/053140. Also, the antigen release velocity may be controlled by conjugating the hyaluronic acid derivative with the antigen according to a method described in International Publication No. WO 2010/053140.

## Antigen

Examples of the antigen that is used for producing a vaccine formulation by forming a complex with the hyaluronic acid derivative comprising a repeating unit represented by the formula (I) or the hyaluronic acid derivative comprising a repeating unit represented by the formula (II) include antigenic peptides and antigenic proteins, and DNA and mRNA encoding any of these antigen sequences. The antigen is preferably an antigenic peptide or an antigenic protein, more preferably an antigenic peptide.

The antigen may be a cancer antigen. The cancer antigen is an antigen often expressed in cancer cells and, in some cases, is expressed only by cancer cells. The cancer antigen may be expressed within cancer cells or on the surface of cancer cells.

The antigenic protein that may be used in the present invention may be selected from, but not limited to, MART-1/Melan-A, gp100, adenosine deaminase binding protein (ADAbp), FAP, cyclophilin b, colorectal associated antigen (CRC)-C017-1A/GA733, carcinoembryonic antigen (CEA), CAP-1, CAP-2, etv6, AML1, prostate-specific antigen (PSA), P SA-1, PSA-2, PSA-3, prostate-specific membrane antigen (PSMA), T cell receptor/CD3-zeta chain, CD20, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8 and GAGE-9, BAGE, RAGE, LAGE-1, NAG, GnT-V, MUM-1, CDK4, tyrosinase, p53, MUC family, HER2/neu, p21ras, RCAS1, $\alpha$-fetoprotein, E-cadherin, $\alpha$-catenin, $\beta$-catenin, $\gamma$-catenin, p120ctn, gp100Pmel117, PRAME, NY-ESO-1, cdc27, adenomatous polyposis coli protein (APC), fodrin, connexin 37, Ig idiotype, p15, gp75, GM2 ganglioside, GD2 ganglioside, human papillomavirus protein, tumor antigen Smad family, lmp-1, P1A, nuclear antigen encoded by EBV (EBNA)-1, brain glycogen phosphorylase, SSX-1, SSX-2(HOM-MEL-40), SSX-1, SSX-4, SSX-5, SCP-1, CT-7, CD20 and c-erbB-2.

The whole sequence of the antigenic protein may be used, or a sequence thereof having a partial deletion may be used.

The antigenic peptide is an antigenic peptide comprising one or more CD8-positive cytotoxic T cell recognition epitopes and/or CD4-positive helper T cell recognition epitopes in the sequences of antigenic proteins. In one aspect, the antigenic peptide is an antigenic peptide preferably comprising two or more epitopes from the viewpoint of being loaded to a MHC class I molecule or a MHC class II molecule through degradation in antigen-presenting cells. Specifically, examples of the antigenic peptide include antigenic peptides comprising antigenic protein epitopes of tumor cells.

In one aspect, the antigenic peptide has, for example, 8 to 120 amino acids, preferably 8 to 80 amino acids, more preferably 15 to 80 amino acids, still more preferably 16 to 80 amino acids, even more preferably 23 to 80 amino acids, further preferably 23 to 60 amino acids, still further preferably 23 to 50 amino acids.

In one aspect, the antigenic peptide is an antigenic peptide comprising one or more each of CD8-positive cytotoxic T cell recognition epitopes and CD4-positive helper T cell recognition epitopes from the viewpoint of inducing the activation of cytotoxic T cells (CTL) by helper T cells.

In one aspect, when two or more epitopes are contained, an amino acid linker may be disposed between the epitopes. The linker has, for example, 2 to 10 amino acids, preferably 4 to 10 amino acids, more preferably 4 to 8 amino acids. Examples of the amino acids for use in the linker include glycine (G), tyrosine (Y), leucine (L), and tryptophan (W). The amino acids are preferably tyrosine (Y), leucine (L), or tryptophan (W). Specific examples of the amino acid linker include -YYYY- (4Y), -LLLL- (4L), -WWWW-(4W), -GGGGGG- (6G), -YYYYYY- (6Y), -LLLLLL- (6L), -WWWWWW- (6W), - YYYYYYYY- (8Y), -LLLLLLLL- (8L) and -WWWWWWWW- (8W). 6Y, 6L and 6W are preferred.

Complex

The complex of a hyaluronic acid derivative having an introduced hydrophobic group, and an antigen according to the present invention can be produced by mixing a hyaluronic acid derivative comprising a repeating unit represented by the formula (I) or a hyaluronic acid derivative comprising a repeating unit represented by the formula (II) with an antigen in an appropriate solution. Examples of the solution used include water, saline, various buffer solutions, sugar solutions, DMSO, ethanol, DMF, and combinations thereof. Solvent or buffer solution replacement may be performed by a method such as dialysis.

The complex of the present invention can be produced, for example, by mixing a fine particle of a hyaluronic acid derivative comprising a repeating unit represented by the formula (I) or a hyaluronic acid derivative comprising a repeating unit represented by the formula (II) with an antigen. The fine particle of the hyaluronic acid derivative and the antigen form a complex, which can be used as the complex of the hyaluronic acid derivative and the antigen, though the event is not particularly limited thereto. Alternatively, the complex of the hyaluronic acid derivative and the antigen can be formed by enclosing the antigen in the fine particle of the hyaluronic acid derivative. The complex includes the antigen enclosed in the fine particle of the hyaluronic acid derivative. In the antibody enclosed therein, the antigen is coated with the hyaluronic acid derivative.

Examples of the complex formation method include a method of adding an antigen solution to a fine particle formed in advance, of a hyaluronic acid derivative comprising a repeating unit represented by the formula (I) or a hyaluronic acid derivative comprising a repeating unit represented by the formula (II). In this method, the formed fine particle of the hyaluronic acid derivative and the antigen form a complex through interaction such as hydrophobic interaction, electrostatic interaction, or a hydrogen bond. The interaction occurs on the surface of the fine particle or in the inside of the fine particle. Conditions such as a solvent, a salt concentration, pH, a temperature, a time, and the addition of a denaturant are not particularly limited and can be appropriately selected such that the antigen forms a complex stably at high yields. Depending on, for example, a salt concentration or pH at the time of complex formation, the degree of swelling or density of the fine particle of the hyaluronic acid derivative varies, and the ionization state of the antigen also varies. Therefore, suitable conditions can be appropriately used according to a combination thereof. When the

complex is formed under a low salt concentration, a fine particle density can be decreased and the amount of the complex formed can be increased through the use of electrostatic repulsion between carboxyl groups of hyaluronic acid derivatives, or a complex with a higher-molecular-weight antigen can be formed. After complex formation, the electrostatic repulsion is weakened by the elevation of the salt concentration. The fine particle density is thereby elevated so that gel networks are smaller than the antigen size. As a result, the antigen is firmly retained, and its release can be delayed. In this respect, the salt concentration may be set to a physiological salt concentration. Ultrasonic irradiation may be performed using an ultrasonic homogenizer or a single-point focused ultrasonic irradiation apparatus for miniaturization or size uniformization. The ultrasonic irradiation may be performed after mixing of the hyaluronic acid derivative with the antigen or may be performed only on the hyaluronic acid derivative. Free uncomplexed antigens can be separated and removed by a dialysis method or size exclusion chromatography (SEC).

In another complex formation method, the hyaluronic acid derivative comprising a repeating unit represented by the formula (I) or the hyaluronic acid derivative comprising a repeating unit represented by the formula (II) can be dissolved in an aprotic polar organic solvent such as DMSO or DMF and mixed with the antigen, followed by replacement with water, an aqueous salt solution, or any of various aqueous solutions of buffer solutions to perform fine particle formation and complex formation at the same time. The replacement can be performed by, for example, dialysis. Ultrasonic irradiation may be performed using an ultrasonic homogenizer or a single-point focused ultrasonic irradiation apparatus for miniaturization or size uniformization. The ultrasonic irradiation may be performed after mixing of the hyaluronic acid derivative with the antigen or may be performed only on the hyaluronic acid derivative. Also, the ultrasonic irradiation may be performed before or after dialysis. The antigen complexed with the hyaluronic acid derivative by this method is less likely to be released from complex. Conditions for mixing the hyaluronic acid derivative with the antigen, such as a solvent species, a salt concentration, pH, a temperature, a time, the addition of a denaturant, a hyaluronic acid derivative concentration, an antigen concentration, and the ratio between the hyaluronic acid derivative and the antigen are not particularly limited and can be appropriately selected such that the antigen forms a complex stably at high yields. Conditions for replacement with water, an aqueous salt solution, or any of various aqueous solutions of buffer solutions, such as a solvent species, a salt concentration, pH, a temperature, a time, the number of runs, the presence or absence of addition of a denaturant, a hyaluronic acid derivative concentration, an antigen concentration, and the ratio between the hyaluronic acid derivative and the antigen are not particularly limited and can be appropriately selected such that the antigen forms a complex stably at high yields. The size of the fine particles is also obtained as intended by appropriately selecting these conditions. Free uncomplexed antigens can be separated and removed by a dialysis method or size exclusion chromatography (SEC).

When the complex is a particle, its particle size is not particularly limited and is, for example, 20 to 200 nm, preferably 20 to 100 nm, more preferably 20 to 50 nm, from the viewpoint of transfer to lymph node.

Vaccine formulation

The complex can be used to produce the vaccine formulation of the present invention, specifically, the vaccine formulation for use in the prevention and/or treatment of a cancer.

The vaccine formulation of the present invention may be administered through an oral, extraintestinal, intranasal, intravaginal, intraocular, subcutaneous, intravenous, intramuscular, intracutaneous, intraperitoneal, joint cavity, intracerebral or intraoral route. Preferably, the vaccine formulation may be administered subcutaneously, intramuscularly, intravenously, or intracutaneously.

The vaccine formulation of the present invention can be administered as a pharmaceutical composition comprising one or more pharmaceutically acceptable additives such as diluent, a wetting agent, an emulsifier, a dispersant, an aid, an antiseptic, a buffer, a binder, and/or a stabilizer, in any appropriate form according to an intended administration route. The administration route may be a parenteral route or may be an oral route.

The vaccine formulation of the present invention may be administered in combination with at least one type of adjuvant. The adjuvant used can be a substance having an effect of potentiating the activity of antigen-presenting cells and is more specifically selected from a substance activating an innate immune receptor (pattern recognition receptor) and other antigen-presenting cell-stimulating substances, and a substance having an effect of inhibiting the acquirement of immunosuppressive activity by antigen-presenting cells. The innate immune receptor is classified into Toll-like receptor (TLR), C-type lectin receptor (CLR), NOD-like receptor (NLR), RIG-I-like receptor (RLR) and cytoplasmic DNA sensor. The adjuvant which is an innate immune receptor agonist can be selected from inactivated microbial cells, a microbial cell extract, a nucleic acid, a lipopolysaccharide, a lipopeptide, a synthetic low-molecular compound, and the like. Preferably, CpG oligo DNA, polyIC RNA, imidazoquinoline (e.g., R848 and imiquimod), saponin (e.g., QuilA and QS21), a STING agonist (e.g., cyclic di-GMP), monophosphoryl lipid, a lipopeptide, or the like is used. A taxane drug or an anthracycline drug can be used as the adjuvant which is an antigen-presenting cell-stimulating agent. The adjuvant which is an agent having an effect of inhibiting the acquirement of immunosuppressive activity by antigen-presenting cells is selected from a substance inhibiting JAK/STAT, a substance inhibiting indole dioxygenase (IDO), a substance

inhibiting tryptophan dioxygenase (TDO), and the like. These inhibiting substances include compounds having an antagonistic effect on the factors as well as neutralizing antibodies, small interfering RNA (siRNA), and antisense DNA against the factors.

The adjuvant that is used for the vaccine formulation of the present invention is preferably an adjuvant which is an innate immune receptor agonist, more preferably an agonist of Toll-like receptor (TLR) or cytoplasmic DNA sensor. The TLR agonist is preferably CpG oligo DNA, polyIC RNA, imidazoquinoline such as R848 or imiquimod, saponin such as QuilA or QS21, a STING agonist, or monophosphoryl lipid.

The adjuvant and the vaccine formulation may be administered as separate formulations or may be administered as one formulation. The adjuvant may be conjugated, for use, with the hyaluronic acid derivative comprising a repeating unit represented by the formula (I) by a method described in International Publication No. WO 2010/053140. For administration in combination, the amount of the vaccine formulation of the present invention administered is, for example, 0.01 to 100 mg/dose, preferably 0.1 to 50 mg/dose, more preferably, for example, 0.1 to 20 mg/dose, and the amount of the adjuvant administered is, for example, 0.01 to 100 mg/kg body weight, preferably 0.1 to 50 mg/kg body weight, more preferably 0.1 to 10 mg/kg body weight. The adjuvant may be administered at the same timing as in the vaccine formulation of the present invention (including the case where the adjuvant is contained in the vaccine formulation), or the adjuvant and the vaccine formulation may be administered at different timings. For administration at different timings, preferably, one of the adjuvant and the vaccine formulation is administered, and the other is administered, for example, within 1 minute to 5 hours, preferably within 1 minute to 1 hour, thereafter.

The vaccine formulation of the present invention may be administered in combination with at least one type of antibody for use in cancer treatment. The antibody is, for example, an antibody inhibiting an immunosuppressive signal from tumor, or at least one type of antibody activating a costimulatory signal of immunocytes, preferably an antibody inhibiting an immunosuppressive signal from tumor or an antibody activating a costimulatory signal of immunocytes. Specifically, the antibody is at least one type of antibody selected from an anti-CTLA4 antibody, an anti-PD1 antibody, an anti-PDL1 antibody, an anti-OX40 antibody, and an anti-4-1BB antibody. For administration in combination, the amount of the vaccine formulation of the present invention administered is, for example, 0.01 to 100 mg/dose, preferably 0.1 to 50 mg/dose, more preferably 0.1 to 20 mg/dose, and the amount of the antibody administered is, for example, 0.01 to 200 mg/kg body weight, preferably 0.1 to 100 mg/kg body weight, more preferably 1 to 40 mg/kg body weight. The antibody may be administered at the same timing as in the vaccine formulation of the present invention (including the case where the antibody is contained in the vaccine formulation), or the antibody and the vaccine formulation may be administered at different timings. For administration at different timings, preferably, one of the antibody and the vaccine formulation is administered, and the other is administered, for example, within 1 minute to 24 hours, preferably within 1 minute to 5 hours, thereafter.

The vaccine formulation of the present invention may be administered in combination with both the adjuvant and the antibody. In this case, the amount of the vaccine formulation of the present invention administered is, for example, 0.01 to 100 mg/dose, preferably 0.1 to 50 mg/dose, more preferably, for example, 0.1 to 20 mg/dose, the amount of the adjuvant administered is, for example, 0.01 to 100 mg/kg body weight, preferably 0.1 to 50 mg/kg body weight, more preferably 0.1 to 10 mg/kg body weight, and the amount of the antibody administered is, for example, 0.01 to 200 mg/kg body weight, preferably 0.1 to 100 mg/kg body weight, more preferably 1 to 40 mg/kg body weight. The adjuvant and the antibody may be administered at the same timing as in the vaccine formulation of the present invention (including the case where the adjuvant and the antibody are contained in the vaccine formulation), or the adjuvant and the antibody and the vaccine formulation may be administered at different timings. For administration at different timings, preferably, all the vaccine formulation, the adjuvant, and the antibody are administered, for example, within 1 minute to 24 hours, preferably within 1 minute to 5 hours.

## EXAMPLES

Hereinafter, preferred specific aspects of the present invention will be described as Examples.

### [Example 1] Synthesis of hyaluronic acid (HA) derivative

HA derivatives were synthesized by the method described in International Publication No. WO 2010/053140 or WO 2014/038641.

### [Example 2] Obtainment of antigen

Antigenic peptides were purchased from Sigma-Genosys LP.

mERK2 p121 (amino acid sequence: NDHIAYFLYQILRGLQYIHSANVLHRDLKPSNLLLNT (SEQ ID NO: 1))

The sequence contained a 9-amino acid sequence (SEQ ID NO: 2: QYIHSANVL) from Q at position 16 to L at

position 24 as a CD8-positive cytotoxic T cell recognition epitope sequence, and a 17-amino acid sequence (SEQ ID NO: 3: RGLQYIHSANVLHRDLK) from R at position 13 to K at position 29 as a CD4-positive helper T cell recognition epitope sequence.

MAGE-A4 p264 (amino acid sequence: GSNPARYEFLWGPRALAETSYVKVLEHVVRVNARVRIAYP (SEQ ID NO: 4))

The sequence contained a 9-amino acid sequence (SEQ ID NO: 5: SNPARYEFL) from S at position 2 to L at position 10 as a CD8-positive cytotoxic T cell recognition epitope sequence and a 16-amino acid sequence (SEQ ID NO: 6: VKVLEHVVRVNARVRI) from V at position 22 to I at position 37 as a CD4-positive helper T cell recognition epitope sequence.

MAGE-A4 p265 (amino acid sequence: SNPARYEFL (SEQ ID NO: 7)).

MAGE-A4 p285 (amino acid sequence: VKVLEHVVRVNARVRIAYP (SEQ ID NO: 8)).

TRP2TRP1gp100-6G (amino acid sequence :SVYDFFVWLGGGGGGTWHRYHLLGGGGGGEGSRNQDWL (SEQ ID NO: 9)).

The sequence contained TRP2 (amino acid sequence: SVYDFFVWL (SEQ ID NO: 13)), TRP1 (amino acid sequence: TWHRYHLL (SEQ ID NO: 14)), and gp100 (amino acid sequence: EGSRNQDWL (SEQ ID NO: 15)) given below as CD8-positive cytotoxic T cell recognition epitope sequences, and the epitopes were connected via 6 G.

TRP2TRP1gp100-6Y (amino acid sequence :SVYDFFVWLYYYYYYTWHRYHLLYYYYYYEGSRNQDWL (SEQ ID NO: 10)).

The sequence contained TRP2 (amino acid sequence: SVYDFFVWL (SEQ ID NO: 13)), TRP1 (amino acid sequence: TWHRYHLL (SEQ ID NO: 14)), and gp100 (amino acid sequence: EGSRNQDWL (SEQ ID NO: 15)) given below as CD8-positive cytotoxic T cell recognition epitope sequences, and the epitopes were connected via 6 Y.

TRP2TRP1gp100-4L (amino acid sequence :SVYDFFVWLLLLLTWHRYHLLLLLLLEGSRNQDWL (SEQ ID NO: 11)).

The sequence contained TRP2 (amino acid sequence: SVYDFFVWL (SEQ ID NO: 13)), TRP1 (amino acid sequence: TWHRYHLL (SEQ ID NO: 14)), and gp100 (amino acid sequence: EGSRNQDWL (SEQ ID NO: 15)) given below as CD8-positive cytotoxic T cell recognition epitope sequences, and the epitopes were connected via 4 L.

TRP2TRP1gp100-6L (amino acid sequence :SVYDFFVWLLLLLLLTWHRYHLLLLLLLLEGSRNQDWL (SEQ ID NO: 12)).

The sequence contained TRP2 (amino acid sequence: SVYDFFVWL (SEQ ID NO: 13)), TRP1 (amino acid sequence: TWHRYHLL (SEQ ID NO: 14)), and gp100 (amino acid sequence: EGSRNQDWL (SEQ ID NO: 15)) given below as CD8-positive cytotoxic T cell recognition epitope sequences, and the epitopes were connected via 6 L.

TRP2 (amino acid sequence: SVYDFFVWL (SEQ ID NO: 13)).

TRP1 (amino acid sequence: TWHRYHLL (SEQ ID NO: 14)).

gp100 (amino acid sequence: EGSRNQDWL (SEQ ID NO: 15)).

AH1gp70-6G (amino acid sequence: LVQFIKDRISVVQAGGGGGGSPSYVYHQF (SEQ ID NO: 16)).

The sequence contained gp70 (amino acid sequence: LVQFIKDRISVVQA (SEQ ID NO: 21)) given below as a CD4-positive helper T cell recognition epitope and AH1 (amino acid sequence: SPSYVYHQF (SEQ ID NO: 20)) given below as a CD8-positive cytotoxic T cell recognition epitope sequence, and the epitopes were connected via 6 G.

AH1gp70-6Y (amino acid sequence: LVQFIKDRISVVQAYYYYYYSPSYVYHQF (SEQ ID NO: 17)).

The sequence contained gp70 (amino acid sequence: LVQFIKDRISVVQA (SEQ ID NO: 21)) given below as a CD4-positive helper T cell recognition epitope and AH1 (amino acid sequence: SPSYVYHQF (SEQ ID NO: 20)) given below as a CD8-positive cytotoxic T cell recognition epitope sequence, and the epitopes were connected via 6 Y.

AH1gp70-4L (amino acid sequence: LVQFIKDRISVVQALLLLSPSYVYHQF (SEQ ID NO: 18)).

The sequence contained gp70 (amino acid sequence: LVQFIKDRISVVQA (SEQ ID NO: 21)) given below as a CD4-positive helper T cell recognition epitope and AH1 (amino acid sequence: SPSYVYHQF (SEQ ID NO: 20)) given below as a CD8-positive cytotoxic T cell recognition epitope sequence, and the epitopes were connected via 4 L.

AH1gp70-6L (amino acid sequence: LVQFIKDRISVVQALLLLLLSPSYVYHQF (SEQ ID NO: 19)).

The sequence contained gp70 (amino acid sequence: LVQFIKDRISVVQA (SEQ ID NO: 21)) given below as a CD4-positive helper T cell recognition epitope and AH1 (amino acid sequence: SPSYVYHQF (SEQ ID NO: 20)) given below as a CD8-positive cytotoxic T cell recognition epitope sequence, and the epitopes were connected via 6 L.

AH1 (amino acid sequence: SPSYVYHQF (SEQ ID NO: 20)).

gp70 (amino acid sequence: LVQFIKDRISVVQA (SEQ ID NO: 21)).

MAGE-A4 p264-4L (amino acid sequence: GSNPARYEFLWGPRALLLLLYVKVLEHVVRVNARVRIAYP (SEQ ID NO: 22)).

The sequence contained a 9-amino acid sequence (SEQ ID NO: 5: SNPARYEFL) from S at position 2 to L at position 10 as a CD8-positive cytotoxic T cell recognition epitope sequence and a 16-amino acid sequence (SEQ ID NO: 6: VKVLEHVVRVNARVRI) from V at position 22 to I at position 37 as a CD4-positive helper T cell recognition epitope sequence, and the epitopes were connected via 4 L.

MAGE-A4 p264-4W (amino acid sequence: GSNPARYEFLWGPRALWWWWYVKVLEHVVRVNARVRIAYP (SEQ ID NO: 23)).

The sequence contained a 9-amino acid sequence (SEQ ID NO: 5: SNPARYEFL) from S at position 2 to L at position 10 as a CD8-positive cytotoxic T cell recognition epitope sequence and a 16-amino acid sequence (SEQ ID NO: 6: VKVLEHVVRVNARVRI) from V at position 22 to I at position 37 as a CD4-positive helper T cell recognition epitope sequence, and the epitopes were connected via 4 W.

[Example 3] Production of complex of HA derivative and antigen

(Example 3-1) Preparation of complex of antigen and HA derivative

Each HA derivative (99k HA-$C_6$-Chol-42%, etc.) was dissolved at 12 mg/mL in ultrapure water, and ultrasonication (manufactured by Covaris Inc., E220X) was carried out. Each antigenic peptide was dissolved at 50 mg/mL in DMSO. The solution of the antigenic peptide in DMSO was added to the aqueous HA derivative solution, and the mixture was ultrasonicated for 30 minutes in a bath-type sonicator. The resultant was transferred to a dialysis kit (Slide-A-Lyzer, molecular weight cutoff: 3.5 K) and dialyzed against a 5 mM carbonate buffer solution (pH 9), a 10 mM phosphate buffer solution (pH 7), and a 10 mM phosphate buffer solution (pH 7) containing 10% sucrose in this order. The antigenic peptide concentration was quantified by reverse-phase chromatography analysis under conditions given below. The concentration was adjusted to 250 to 500 μg/mL to prepare an administration solution. If the concentration did not reach the intended one, the solution was concentrated with an ultrafiltration concentrator (Vivaspin 6, 5000 MwCO, Sartorius AG). The HA derivative was synthesized as described in Examples 1 and 2 of International Publication No. WO 2010/053140 A1. For example, 99k HA-$C_6$-Chol-42% shown in Table 1 of Example 4-1 denotes a HA derivative of the formula (I) wherein Z is a direct bond, $R^a$ is a hydrogen atom, Y is $C_6$ alkylene, $X^1$ is -$NR^b$-COO-R, $R^b$ is a hydrogen atom, and R is a cholesteryl group (i.e., cholesteryl 6-aminohexyl carbamate was introduced); the weight-average molecular weight is 99 kDa; and the rate of introduction of the cholesteryl group is 42%. Likewise, a HA derivative wherein the rate of introduction of the cholesteryl group is 41% is designated as 99k HA-$C_6$-Chol-41%, and a HA derivative wherein the rate of introduction of the cholesteryl group is 43% is designated as 99k HA-$C_6$-Chol-43%.

Reverse-phase chromatography analysis conditions

Analytical column: PLRP-S 1000 angstroms (Agilent Technologies, Inc.)
Column temperature: 40°C
Mobile phase A: 0.1% aqueous TFA solution, mobile phase B: 0.1% TFA solution in acetonitrile
Flow rate: 2 mL/min
Detection: UV 254 nm
Injection volume: 50 **μL**
[Comparative Example 1] Production of complex of CHP and antigen
A complex was prepared in accordance with International Publication

No. WO 2015/050158. Cholesterol-modified pullulan (abbreviation: CHP) (CHP-80T; 1 to 2 cholesterol molecules were introduced per 100 monosaccharides) was obtained from NOF Corp. CHP was dissolved at a concentration of 10 mg/mL in phosphate-buffered saline (PBS) containing 6 M urea. A solution of an antigenic peptide in DMSO was added to the CHP solution, and the mixture was left overnight at room temperature in the dark. The mixed solution was transferred to a dialysis membrane (molecular weight cutoff: 3,500, Thermo Fisher Scientific Inc.) and dialyzed at 4°C for 2 hours to overnight against PBS containing 0.6 M urea at a volume ratio of 100 times or more as an outer solution of the dialysis membrane. Subsequently, the resultant was dialyzed at 4°C for 2 hours to overnight against PBS containing 0.06 M urea at a volume ratio of 100 times or more as an outer solution of the dialysis membrane. Again, the resultant was dialyzed at 4°C for 2 hours to overnight against PBS at a volume ratio of 100 times or more as an outer solution of the dialysis membrane. After recovery of an inner solution of the dialysis membrane, the antigen concentration was quantified by the method described in Example 3-1. The concentration was adjusted to prepare an administration solution. If the concentration did not reach the intended one, the solution was concentrated with an ultrafiltration concentrator (Vivaspin 6, 5000 MwCO, Sartorius AG).

[Example 4] Antigen-specific T cell induction test

Experimental method

The complex of the antigenic peptide and the HA derivative prepared in Example 3-1 or the complex of CHP and the antigenic peptide prepared in Comparative Example 1 was subcutaneously administered in an amount corresponding to 0.05 to 0.1 mg of the antigen to the right back of each BALB/c mouse (female, Japan SLC, Inc.; body weight: 15 to 25 g) or C57BL/6 mouse (female, Japan SLC, Inc.; body weight: 15 to 25 g). In the case of administering an adjuvant (CpG oligo DNA (ODN1668, manufactured by InvivoGen), 0.05 mg), the adjuvant was subsequently administered subcutaneously near the administration site. The number of doses is 2, and the interval between the doses was 1 week. 1 week after final administration, spleen cells were separated from the recipient mouse by the following procedures: the spleen was isolated from the mouse and washed with RPMI1640 medium containing 10% fetal bovine serum (FBS), and blood was removed. The spleen was ground, and liberated cells were then recovered into RPMI1640 medium containing FBS. After centrifugation (300 $\times$ g, 5 min, 4°C), the supernatant was removed, and 1 to 2 mL of an ACK solution (manufactured by Sigma-Aldrich Co. LLC) was added to the cells. 1 minute later, 30 mL of RPMI1640 medium containing FBS was added thereto, and the mixture was centrifuged (300 $\times$ g, 5 min, 4°C). The supernatant was removed, and 10 mL of RPMI1640 medium containing FBS was added to the cells, followed by filter treatment using a 40 $\mu$m cell strainer and centrifugation (300 $\times$ g, 5 min, 4°C). The supernatant was removed, and 20 mL of RPMI1640 medium containing FBS was added to the cells, followed by centrifugation (300 $\times$ g, 5 min, 4°C). The supernatant was removed, and the cells were suspended in an appropriate amount of RPMI1640 medium containing FBS. The number of cells was counted, and the cells were then suspended at a cell density of 2 $\times$ $10^7$ cells/mL in RPMI1640 medium containing FBS.

The mouse spleen cells were added at 5 $\times$ $10^6$ cells/0.4 mL per well to a 24-well culture plate (Nunc). 50 $\mu$L of 100 $\mu$g/mL peptide for T cell stimulation was added to each well, and the cells were cultured at 37°C for 30 minutes under 5% $CO_2$. Then, GolgiPlug (BD Biosciences) diluted 100-fold with RPMI1640 medium containing FBS was added at 50 $\mu$L per well, and the cells were cultured at 37°C for 5 hours under 5% $CO_2$. The cells were recovered and transferred to a 96-well V-bottom microplate (Nunc). A supernatant was removed by centrifugation (2000 rpm, 2 min, 4°C; the same holds true for the conditions below). Then, the cells were suspended in 200 $\mu$L of a buffer for staining (PBS containing 0.5% bovine serum albumin (BSA)) per well. A supernatant was removed by centrifugation. Then, 50 $\mu$L of a solution containing a fluorescently labeled anti-CD8 antibody (manufactured by Invitrogen Corp.) and a fluorescently labeled anti-CD4 antibody (BD Biosciences) was added to each well and mixed, and the plate was then left standing at 4°C for 15 minutes in the dark. 150 $\mu$L of a buffer for staining was added to each well. A supernatant was removed by centrifugation, and the cells were then further washed with 200 $\mu$L of a buffer for staining. 100 $\mu$L of Cytofix/Cytoperm buffer (BD Biosciences) was added to each well and gently mixed. The plate was left standing at room temperature for 20 minutes in the dark, and the cells were then further washed twice with a buffer for staining. 200 $\mu$L of a buffer for staining was added to each well and refrigerated overnight with light shielded. A supernatant was removed by centrifugation. Then, 200 $\mu$L of Perm/Wash buffer (BD Biosciences) was added to each well, and the plate was left standing at room temperature for 3 minutes with light shielded. A supernatant was removed by centrifugation. Then, 50 $\mu$L of Perm/Wash buffer supplemented with each anti-cytokine antibody was added to the cells for gentle suspension, and the plate was then left standing at room temperature for 15 minutes in the dark. The cells were washed with 150 $\mu$L of Perm/Wash buffer, then resuspended in 200 $\mu$L of a buffer for staining, and transferred to a round-bottom polystyrene tube (BD Biosciences). The cells were analyzed in a flow cytometer (FACS Canto II, BD Biosciences) using attached analytical software (FACSDiva).

(Example 4-1) T cell induction test using mERK2 peptide

The test was conducted according to the method described in Example 4 by using BALB/c mice and administering each sample of Table 1 twice. The adjuvant used was CpG oligo DNA (ODN1668, manufactured by InvivoGen). Figure 1 shows the ratio of the number of interferon gamma-producing $CD8^+$ cells to the number of all $CD8^+$ cells when mERK2 p121 was used as a peptide for $CD8^+$ T cell stimulation.

[Table 1]

Table 1 Administration sample

| Abbreviation | Carrier | Peptide species, Dose | Adjuvant species, Dose |
|---|---|---|---|
| CpG | None | mERK2 p121<br>0.1 mg | CpG<br>0.05 mg |
| CHP+CpG | CHP-80T;80k | mERK2 p121<br>0.1 mg | CpG<br>0.05 mg |

(continued)

| Abbreviation | Carrier | Peptide species, Dose | Adjuvant species, Dose |
|---|---|---|---|
| 99k41+CpG | 99k HA-$C_6$-Chol-41% | mERK2 p121 0.1 mg | CpG 0.05 mg |

Use of the HA derivative as a carrier was confirmed to be able to induce T cells at a high level.

(Example 4-2) T cell induction test using mERK2 peptide (adjuvant effect - 1)

The test was conducted according to the method described in Example 4 by using BALB/c mice and administering each sample of Table 2 twice. The adjuvant used was CpG oligo DNA (ODN1668, manufactured by InvivoGen), polyIC (HMW VacciGrade, manufactured by InvivoGen), QuilA (manufactured by InvivoGen), or a Sting agonist (2'3'-cGAM(PS)2(Rp/Sp), manufactured by InvivoGen). Figure 2-1 shows the ratio of the number of interferon gamma-producing $CD8^+$ cells to the number of all $CD8^+$ cells when mERK2 p121 was used as a peptide for $CD8^+$ T cell stimulation. Figure 2-2 shows the ratio of the number of interferon gamma-producing $CD4^+$ cells to the number of all $CD4^+$ cells when mERK2 p121 was used as a peptide for $CD4^+$ T cell stimulation.

[Table 2]

Table 2 Administration sample

| Abbreviation | Carrier | Peptide species, Dose | Adjuvant species, Dose |
|---|---|---|---|
| 99k41 | 99k HA-$C_6$-Chol-41% | mERK2 p121 0.1 mg | None |
| 99k41+CpG | 99k HA-$C_6$-Chol-41% | mERK2 p121 0.1 mg | CpG 0.05 mg |
| 99k41+PolyIC | 99k HA-$C_6$-Chol-41% | mERK2 p121 0.1 mg | PolyIC 0.05 mg |
| 99k41+QuilA | 99k HA-$C_6$-Chol-41% | mERK2 p121 0.1 mg | QuilA 0.05 mg |
| 99k41+Sting | 99k HA-$C_6$-Chol-41% | mERK2 p121 0.1 mg | Sting 0.05 mg |

Use of any of the adjuvants was confirmed to be able to induce T cells.

(Example 4-3) T cell induction test using mERK2 peptide (adjuvant effect - 2)

The test was conducted according to the method described in Example 4 by using BALB/c mice and administering each sample of Table 3 twice. The adjuvant used was CpG oligo DNA (ODN1668, manufactured by InvivoGen), R848 (VacciGrade, VacciGrade, manufactured by InvivoGen), or MPL (MPLAs VacciGrade, manufactured by InvivoGen). Figure 3-1 shows the ratio of the number of interferon gamma-producing $CD8^+$ cells to the number of all $CD8^+$ cells when mERK2 p121 was used as a peptide for $CD8^+$ T cell stimulation. Figure 3-2 shows the ratio of the number of interferon gamma-producing $CD4^+$ cells to the number of all $CD4^+$ cells when mERK2 p121 was used as a peptide for $CD4^+$ T cell stimulation.

[Table 3]

Table 3 Administration sample

| Abbreviation | Carrier | Peptide species, Dose | Adjuvant species, Dose |
|---|---|---|---|
| 99k41+CpG | 99k HA-$C_6$-Chol-41% | mERK2 p121 0.1 mg | CpG 0.05 mg |
| 99k41+R848 | 99k HA-$C_6$-Chol-41% | mERK2 p121 0.1 mg | R848 0.05 mg |
| 99k41+MPL | 99k HA-$C_6$-Chol-41% | mERK2 p121 0.1 mg | MPL 0.05 mg |

Use of any of the adjuvants was confirmed to be able to induce T cells.

(Example 4-4) T cell induction test using MAGE-A4 p264 peptide

The test was conducted according to the method described in Example 4 by using BALB/c mice and administering each sample of Table 4 twice. Figure 4 shows the ratio of the number of interferon gamma-producing $CD8^+$ cells to the number of all $CD8^+$ cells when MAGE-A4 p265 was used as a peptide for $CD8^+$ T cell stimulation.

[Table 4]

Table 4 Administration sample

| Abbreviation | Carrier | Peptide species, Dose | Adjuvant species, Dose |
|---|---|---|---|
| peptide+CpG | None | MAGE-A4 p264 0.075 mg | CpG 0.05 mg |
| CHP/Wt+CpG | CHP-80T;80k | MAGE-A4 p264 0.075 mg | CpG 0.05 mg |
| 99k41/Wt+CpG | 99k HA-$C_6$-Chol-41% | MAGE-A4 p264 0.075 mg | CpG 0.05 mg |

Use of the HA derivative was confirmed to be able to induce T cells at a high level.

(Example 4-5) T cell induction test using MAGE-A4 p264 peptide (without adjuvant)

The test was conducted according to the method described in Example 4 by using BALB/c mice and administering each sample of Table 5 twice. Figure 5 shows the ratio of the number of interferon gamma-producing $CD8^+$ cells to the number of all $CD8^+$ cells when MAGE-A4 p265 was used as a peptide for $CD8^+$ T cell stimulation.

[Table 5]

Table 5 Administration sample

| Abbreviation | Carrier | Peptide species, Dose | Adjuvant species, Dose |
|---|---|---|---|
| CHP/Wt | CHP-80T;80k | MAGE-A4 p264 0.075 mg | None |
| 99k41/Wt | 99k HA-$C_6$-Chol-41% | MAGE-A4 p264 0.075 mg | None |

Use of the HA derivative as a carrier was confirmed to be able to induce T cells at a high level even when no adjuvant was used.

(Example 4-6) T cell induction test using MAGE-A4 p264 peptide

The test was conducted according to the method described in Example 4 by using BALB/c mice and administering each sample of Table 6 twice. Figure 6 shows the ratio of the number of interferon gamma-producing $CD8^+$ cells to the number of all $CD8^+$ cells when MAGE-A4 p265 was used as a peptide for $CD8^+$ T cell stimulation.

[Table 6]

Table 6 Administration sample

| Abbreviation | Carrier | Peptide species, Dose | Adjuvant species, Dose |
|---|---|---|---|
| 99k41/Wt+CpG | 99k HA-$C_6$-Chol-41% | MAGE-A4 p264<br>0.075 mg | CpG<br>0.05 mg |
| 99k412/LLLL+CpG | 99k HA-$C_6$-Chol-41% | MAGE-A4 p264-4L<br>0.075 mg | CpG<br>0.05 mg |
| 99k41/WWWW+CpG | 99k HA-$C_6$-Chol-41% | MAGE-A4 p264-4W<br>0.075 mg | CpG<br>0.05 mg |

Use of the peptide containing any of the amino acid linkers was found to be able to induce T cells at a high level.

(Example 4-7) T cell induction test using TRP2TRP1gp100 peptide

The test was conducted according to the method described in Example 4 by using C57BL/6 mice and administering each sample of Table 7 twice. Figures 7-1, 7-2, and 7-3 show the ratio of the number of interferon gamma-producing $CD8^+$ cells to the number of all $CD8^+$ cells when TRP1, TRP2, and gp100, respectively, were used as peptides for $CD8^+$ T cell stimulation.

[Table 7]

Table 7 Administration sample

| Abbreviation | Carrier | Peptide species, Dose | Adjuvant species, Dose |
|---|---|---|---|
| CHP/6Y+CpG | CHP-80T;80k | TRP2TRP1gp100-6Y<br>0.1 mg | CpG<br>0.05 mg |
| 99k43/6Y+CpG | 99k HA-$C_6$-Chol-43% | TRP2TRP1gp100-6Y<br>0.1 mg | CpG<br>0.05 mg |

Use of the HA derivative as a carrier was confirmed to be able to induce T cells at a high level for each of TRP1, TRP2, and gp100.

(Example 4-8) T cell induction test using TRP2TRP1gp100 peptide

The test was conducted according to the method described in Example 4 by using C57BL/6 mice and administering each sample of Table 8 twice. Figures 8-1, 8-2, and 8-3 show the ratio of the number of interferon gamma-producing $CD8^+$ cells to the number of all $CD8^+$ cells when TRP1, TRP2, and gp100, respectively, were used as peptides for $CD8^+$ T cell stimulation.

[Table 8]

Table 8 Administration sample

| Abbreviation | Carrier | Peptide species, Dose | Adjuvant species, Dose |
|---|---|---|---|
| 99k43/6G+CpG | 99k HA-$C_6$-Chol-43% | TRP2TRP1gp100-6G 0.1 mg | CpG 0.05 mg |
| 99k43/6Y+CpG | 99k HA-$C_6$-Chol-43% | TRP2TRP1gp100-6Y 0.1 mg | CpG 0.05 mg |
| 99k43/4L+CpG | 99k HA-$C_6$-Chol-43% | TRP2TRP1gp100-4L 0.1 mg | CpG 0.05 mg |
| 99k43/6L+CpG | 99k HA-$C_6$-Chol-43% | TRP2TRP1gp100-6L 0.1 mg | CpG 0.05 mg |

Use of any peptide linker was found to be able to induce T cells at a high level.

(Example 4-9) T cell induction test using AH1gp70 peptide

The test was conducted according to the method described in Example 4 by using BALB/c mice and administering each sample of Table 9 twice. Figure 9-1 shows the ratio of the number of interferon gamma-producing $CD8^+$ cells to the number of all $CD8^+$ cells when AH1 was used as a peptide for $CD8^+$ T cell stimulation. Figure 9-2 shows the ratio of the number of interferon gamma-producing $CD4^+$ cells to the number of all $CD4^+$ cells when gp70 was used as a peptide for $CD4^+$ T cell stimulation.

[Table 9]

Table 9 Administration sample

| Abbreviation | Carrier | Peptide species, Dose | Adjuvant species, Dose |
|---|---|---|---|
| CHP/6Y+CpG | CHP-80T;80k | AH1gp70-6Y 0.1 mg | CpG 0.05 mg |
| 99k43/6Y+CpG | 99k HA-$C_6$-Chol-43% | AH1gp70-6Y 0.1 mg | CpG 0.05 mg |

Use of the HA derivative was confirmed to be able to induce T cells at a high level for each of AH1 and gp70.

(Example 4-10) T cell induction test using AH1gp70 peptide

The test was conducted according to the method described in Example 4 by using BALB/c mice and administering each sample of Table 10 twice. Figure 10-1 shows the ratio of the number of interferon gamma-producing $CD8^+$ cells to the number of all $CD8^+$ cells when AH1 was used as a peptide for $CD8^+$ T cell stimulation. Figure 10-2 shows the ratio of the number of interferon gamma-producing $CD4^+$ cells to the number of all $CD4^+$ cells when gp70 was used as a peptide for $CD4^+$ T cell stimulation.

[Table 10]

Table 10 Administration sample

| Abbreviation | Carrier | Peptide species, Dose | Adjuvant species, Dose |
|---|---|---|---|
| CHP/6L+CpG | CHP-80T;80k | AH1gp70-6L 0.1 mg | CpG 0.05 mg |

(continued)

| Abbreviation | Carrier | Peptide species, Dose | Adjuvant species, Dose |
|---|---|---|---|
| 99k43/6L+CpG | 99k HA-$C_6$-Chol-43% | AH1gp70-6L 0.1 mg | CpG 0.05 mg |

Use of the HA derivative was confirmed to be able to induce T cells at a high level for each of AH1 and gp70.

(Example 4-11) T cell induction test using AH1gp70 peptide

The test was conducted according to the method described in Example 4 by using BALB/c mice and administering each sample of Table 11 twice. Figure 11-1 shows the ratio of the number of interferon gamma-producing $CD8^+$ cells to the number of all $CD8^+$ cells when AH1 was used as a peptide for $CD8^+$ T cell stimulation. Figure 11-2 shows the ratio of the number of interferon gamma-producing $CD4^+$ cells to the number of all $CD4^+$ cells when gp70 was used as a peptide for $CD4^+$ T cell stimulation.

[Table 11]

Table 11 Administration sample

| Abbreviation | Carrier | Peptide species, Dose | Adjuvant species, Dose |
|---|---|---|---|
| 99k43/6G+CpG | 99k HA-$C_6$-Chol-43% | AH1gp70-6G 0.1 mg | CpG 0.05 mg |
| 99k43/6Y+CpG | 99k HA-$C_6$-Chol-43% | AH1gp70-6Y 0.1 mg | CpG 0.05 mg |
| 99k43/4L+CpG | 99k HA-$C_6$-Chol-43% | AH1gp70-4L 0.1 mg | CpG 0.05 mg |
| 99k43/6L+CpG | 99k HA-$C_6$-Chol-43% | AH1gp70-6L 0.1 mg | CpG 0.05 mg |

Use of the peptide species containing any of the amino acid linkers was found to be able to induce T cells at a high level.

[Example 5] Mouse tumor growth test

Experimental method

A mouse fibrosarcoma CMS5a cell line expressing mERK2, a mouse colorectal cancer cell CT26 cell line expressing AH1 and gp70, or a mouse melanoma B16F10 cell line expressing TRP2, TRP1, and gp100 was subcutaneously transplanted. The CMS5a cell line and the CT26 cell line were each subcutaneously transplanted in an amount of $1 \times 10^6$ cultured cells/100 μL/individual to each BALB/c mouse (female; body weight: 15 to 25 g). The B16F10 cell line was subcutaneously transplanted in an amount of $2 \times 10^5$ cultured cells/100 μL/individual to each C57BL/6 mouse (female; body weight: 15 to 25 g). Then, tumor volumes were measured over time.

(Comparative Example 5-1) Preparation of emulsion formulation

An emulsion was produced from an antigenic peptide, 0.5 mL of a Freund's incomplete adjuvant (manufactured by InvivoGen), and 0.5 mL of saline using two hard glass syringes with lock fittings connected through a syringe connector. The antigenic peptide concentration was set to 0.5 mg/mL.

(Example 5-1) Mouse fibrosarcoma CMS5a

Each sample of Table 12 was subcutaneously administered with the frequency described in Table 12 to the mouse fibrosarcoma CMS5a-bearing mice prepared by the method described in Example 5 to conduct a mouse tumor growth test. Figure 12-1 shows changes in average tumor volume, and Figure 12-2 shows changes in each individual.

[Table 12]

Table 12 Administration sample

| Abbreviation | Carrier | Peptide species, Dose | Adjuvant species, Dose | Administration day (after transplantation) |
|---|---|---|---|---|
| Peptide | None | mERK2 p121 0.05 mg | CpG 0.05 mg | Day 4, day 11 |
| Emulsion | Emulsion | mERK2 p121 0.05 mg | CpG 0.05 mg | Day 4, day 11 |
| CHP | CHP-80T; 80k | mERK2 p121 0.05 mg | CpG 0.05 mg | Day 4, day 11 |
| 99k41 | 99k HA-$C_6$-Chol-41% | mERK2 p121 0.05 mg | CpG 0.05 mg | Day 4, day 11 |

The HA derivative (99k41) group was shown to have a high tumor growth suppressive effect as compared with the peptide group, the emulsion group, and the CHP group.

(Example 5-2) Mouse fibrosarcoma CMS5a

Each sample of Table 13 was subcutaneously administered with the frequency described in Table 13 to the mouse fibrosarcoma CMS5a-bearing mice prepared by the method described in Example 5 to conduct a mouse tumor growth test. Figure 13-1 shows changes in average tumor volume, and Figure 13-2 shows changes in each individual.

[Table 13]

Table 13 Administration sample

| Abbreviation | Carrier | Peptide species, Dose | Adjuvant species, Dose | Administration day (after transplantation) |
|---|---|---|---|---|
| CpG | None | None | CpG 0.05 mg | Day 4, day 11 |
| CHP | CHP-80T; 80k | mERK2 p121 0.05 mg | CpG 0.05 mg | Day 4, day 11 |
| 99k41 | 99k HA-$C_6$-Chol-41% | mERK2 p121 0.05 mg | CpG 0.05 mg | Day 4, day 11 |

The HA derivative (99k41) group was shown to have a high tumor growth suppressive effect as compared with the CpG-alone group and the CHP group.

(Example 5-3) Mouse fibrosarcoma CMS5a

Each sample of Table 14 was subcutaneously administered with the frequency described in Table 14 to the mouse fibrosarcoma CMS5a-bearing mice prepared by the method described in Example 5 to conduct a mouse tumor growth test. The antibodies were intraperitoneally administered. Figure 14-1 shows changes in average tumor volume, and Figure 14-2 shows changes in each individual. The anti-CTLA4 antibody, the anti-PDI antibody, and the anti-PDLI antibody were each obtained from Bio X Cell.

[Table 14]

Table 14 Administration sample

| Abbreviation | Carrier | Peptide species, Dose | Adjuvant species, Dose | Antibody species Dose | Administration day (after transplantation) |
|---|---|---|---|---|---|
| aCTLA4/aPD1/ aPDL1 | None | None | None | Anti-CTLA4 antibody (0.2mg)+Anti-PD1 antibody (0.2 mg)+Anti-PDL1 antibody (0.2 mg) | Day 7, day 11, day 14, day 18 |
| 99k41 | 99k HA-$C_6$-Chol-41% | mERK2 p121 0.05 mg | CpG 0.05 mg | None | Day 7, day 13 |

The HA derivative (99k41) group exhibited a high tumor growth suppressive effect even by administration from day 7, and the effect was shown to be higher than that of the antibodies.

(Example 5-4) Mouse fibrosarcoma CMS5a

Each sample of Table 15 was subcutaneously administered with the frequency described in Table 15 to the mouse fibrosarcoma CMS5a-bearing mice prepared by the method described in Example 5 to conduct a mouse tumor growth test. The antibodies were intraperitoneally administered. Figure 15-1 shows changes in average tumor volume, and

Figure 15-2 shows changes in each individual. The anti-CTLA4 antibody, the anti-PDLI antibody, the anti-OX40 antibody, and the anti-4-1BB antibody were each obtained from Bio X Cell.

[Table 15]

Table 15 Administration sample

| Abbreviation | Carrier | Peptide species, Dose | Adjuvant species, Dose | Antibody species Dose | Administration day (after transplantation) |
|---|---|---|---|---|---|
| aCTLA4/aPDL1/ aOX40/a4-1BB | None | None | None | Anti-CTLA4 antibody (0.2 mg)+Anti-PDL1 antibody (0.2 mg)+Anti-OX40 antibody (0.2 mg)+Anti-4-1BB antibody (0.2 mg) | Day 7, day 11, day 14, day 18 |
| 99k41 | 99k HA-$C_6$-Chol-41% | mERK2 p121 0.05 mg | CpG 0.05 mg | None | Day 7, day 13 |
| 99k41+aCTLA4/ aPDL1/aOX40/a4 -1BB | 99k HA-$C_6$-Chol-41% | mERK2 p121 0.05 mg | CpG 0.05 mg | Anti-CTLA4 antibody (0.2 mg)+Anti-PDL1 antibody (0.2 mg)+Anti-OX40 antibody (0.2 mg)+Anti-4-1BB antibody (0.2 mg) | 99k42: day 7, day 13, antibody: day 7, day 11, day 14, day 18 |

The HA derivative (99k41) was shown to have a higher tumor growth suppressive effect based on the mERK2 peptide by combination with the antibodies (aCTLA4/aPDL1/aOX40/a4-1BB).

(Example 5-5) Mouse colorectal cancer cell CT26

Each sample of Table 16 was subcutaneously administered with the frequency described in Table 16 to the mouse colorectal cancer cell CT26-bearing mice prepared by the method described in Example 5 to conduct a mouse tumor growth test. Figure 16-1 shows changes in average tumor volume, and Figure 16-2 shows changes in each individual.

[Table 16]

Table 16 Administration sample

| Abbreviation | Carrier | Peptide species, Dose | Adjuvant species, Dose | Administration day (after transplantation) |
|---|---|---|---|---|
| Emulsion | Emulsion | AH1gp70-6L 0.05 mg | CpG 0.05 mg | Day 9, day 16, day 23 |
| CHP | CHP-80T;80k | AH1gp70-6L 0.05 mg | CpG 0.05 mg | Day 9, day 16, day 23 |
| 99k43 | 99k HA-$C_6$-Chol-43% | AH1gp70-6L 0.05 mg | CpG 0.05 mg | Day 9, day 16, day 23 |

The HA derivative (99k43) group was shown to have a high tumor growth suppressive effect as compared with the emulsion group and the CHP group.

(Example 5-6) Mouse melanoma B16F10

Each sample of Table 17 was subcutaneously administered with the frequency described in Table 17 to the mouse melanoma B16F10-bearing mice prepared by the method described in Example 5 to conduct a mouse tumor growth test. Figure 17-1 shows changes in average tumor volume, and Figure 17-2 shows changes in each individual.

[Table 17]

Table 17 Administration sample

| Abbreviation | Carrier | Peptide species, Dose | Adjuvant species, Dose | Administration day (after transplantation) |
|---|---|---|---|---|
| CHP | CHP-80T; 80k | TRP2TRP1gp100-6Y 0.05 mg | CpG 0.05 mg | Day 10, day 13, day 17 |
| 99k43 | 99k HA-$C_6$-Chol-43% | TRP2TRP1gp100-6Y 0.05 mg | CpG 0.05 mg | Day 10, day 13, day 17 |

The HA derivative (99k43) group was shown to have a high tumor growth suppressive effect as compared with the CHP group.

[Example 6] Lymph node delivery

Experimental method

Each sample prepared by the method described in Example 3 was subcutaneously administered to the right back of each BALB/c mouse (body weight: 15 to 25 g) using fluorescently labeled mERK2 p121 (fluorescein-conjugated). 24 hours later and 72 hours later, the right inguinal lymph node was harvested, stained with an anti-CD11b antibody and an anti-F4/80 antibody, and then analyzed for the uptake of the fluorescently labeled peptide into F4/80$^+$CD11b$^+$

cells by flow cytometry. The cells were analyzed in a flow cytometer (FACS Canto II, BD Biosciences) using attached analytical software (FACSDiva).

(Example 6-1) Lymph node delivery of various HA derivatives

Each sample described in Table 18 was administered and analyzed by the method described in Example 6 to conduct a lymph node migration test. Figure 18-1 shows fluorescence intensity per cell 24 hours later, and Figure 18-2 shows fluorescence intensity per cell 72 hours later.

[Table 18]

Table 18 Administration sample

| Abbreviation | Carrier | Peptide species, Dose | Adjuvant |
|---|---|---|---|
| Peptide | None | Fluorescently labeled mERK2 p121 0.05 mg | None |
| CHP | CHP-80T;80k | Fluorescently labeled mERK2 p121 0.05 mg | None |
| 10k17 | 10k HA-$C_6$-Chol-17% | Fluorescently labeled mERK2 p121 0.05 mg | None |
| 10k25 | 10k HA-$C_6$-Chol-25% | Fluorescently labeled mERK2 p121 0.05 mg | None |
| 10k42 | 10k HA-$C_6$-Chol-42% | Fluorescently labeled mERK2 p121 0.05 mg | None |
| 50k23 | 50k HA-$C_6$-Chol-23% | Fluorescently labeled mERK2 p121 0.05 mg | None |
| 50k43 | 50k HA-$C_6$-Chol-43% | Fluorescently labeled mERK2 p121 0.05 mg | None |
| 99k24 | 99k HA-$C_6$-Chol-24% | Fluorescently labeled mERK2 p121 0.05 mg | None |
| 99k43 | 99k HA-$C_6$-Chol-43% | Fluorescently labeled mERK2 p121 0.05 mg | None |

The HA derivative group was shown to transfer more mERK2 to lymph node as compared with the peptide group and the CHP group.

(Example 6-2) Lymph node delivery of various amino acid-modified HA derivatives

Each sample described in Table 19 was administered and analyzed by the method described in Example 6 to conduct a lymph node migration test. Figure 19 shows fluorescence intensity per cell 24 hours later. The amino acid-modified HA derivatives were synthesized as described in Example 1 of International Publication No. WO 2014/038641. For example, 10kHA-Ala-$C_6$-Chol-30% of Table 19 below denotes a HA derivative of the formula (II) wherein $R^{aa}$ is methyl ($C_1$ alkyl), $R^{6a}$ is a hydrogen atom, $X^{1a}$ is $-NR^9-Z^1-Z^2$, $R^9$ is a hydrogen atom, $Z^1$ is $C_6$ alkylene, $Z^2$ is $-NR^{ba}-COO-Z^3$, $R^{ba}$ is a hydrogen atom, and $Z^3$ is a cholesteryl group (i.e., alanine and cholesteryl 6-aminohexyl carbamate were introduced); the weight-average molecular weight is 10 kDa; and the rate of introduction of the cholesteryl group is 30%. The synthesized amino acid-modified HA derivatives are described in Example 3-8 of International Publication No. WO 2014/038641.

[Table 19]

Table 19 Administration sample

| Abbreviation | Carrier | Peptide species, Dose | Adjuvant |
|---|---|---|---|
| Peptide | None | Fluorescently labeled mERK2 p121 0.05 mg | None |
| 99k41 | 99k HA-$C_6$-Chol-41% | Fluorescently labeled mERK2 p121 0.05 mg | None |
| 10kHA-Ala-Chol30 | 10kHA-Ala-$C_6$-Chol-30% | Fluorescently labeled mERK2 p121 0.05 mg | None |
| 10kHA-Ser-Chol28 | 10kHA-Ser-$C_6$-Chol-28% | Fluorescently labeled mERK2 p121 0.05 mg | None |

(continued)

| Abbreviation | Carrier | Peptide species, Dose | Adjuvant |
|---|---|---|---|
| 10kHA-Gly-Chol32 | 10kHA-Gly-$C_6$-Chol-32% | Fluorescently labeled mERK2 p121 0.05 mg | None |
| 10kHA-Thr-Chol32 | 10kHA-Thr-$C_6$-Chol-32% | Fluorescently labeled mERK2 p121 0.05 mg | None |
| 10kHA-Asn-Chol20 | 10kHA-Asn-$C_6$-Chol-20% | Fluorescently labeled mERK2 p121 0.05 mg | None |
| 10kHA-Asp-Chol32 | 10kHA-Asp-$C_6$-Chol-32% | Fluorescently labeled mERK2 p121 0.05 mg | None |
| 10kHA-Phe-Chol31 | 10kHA-Phe-$C_6$-Chol-31% | Fluorescently labeled mERK2 p121 0.05 mg | None |
| 10kHA-Tyr-Chol32 | 10kHA-Tyr-$C_6$-Chol-32% | Fluorescently labeled mERK2 p121 0.05 mg | None |
| 10kHA-Ile-Chol28 | 10kHA-Ile-$C_6$-Chol-28% | Fluorescently labeled mERK2 p121 0.05 mg | None |
| 10kHA-Leu-Chol31 | 10kHA-Leu-$C_6$-Chol-31% | Fluorescently labeled mERK2 p121 0.05 mg | None |
| 10kHA-Val-Chol32 | 10kHA-Val-$C_6$-Chol-32% | Fluorescently labeled mERK2 p121 0.05 mg | None |
| 10kHA-Trp-Chol24 | 10kHA-Trp-$C_6$-Chol-24% | Fluorescently labeled mERK2 p121 0.05 mg | None |
| 10kHA-Gln-Chol32 | 10kHA-Gln-$C_6$-Chol-32% | Fluorescently labeled mERK2 p121 0.05 mg | None |
| 10kHA-Glu-Chol32 | 10kHA-Glu-$C_6$-Chol-32% | Fluorescently labeled mERK2 p121 0.05 mg | None |

The amino acid-modified HA derivative group was shown to transfer mERK2 to lymph node.

[Example 7] Mouse tumor growth test

(Example 7-1) Mouse melanoma B16F10

Each sample of Table 20 was subcutaneously administered with the frequency described in Table 20 to the mouse melanoma B16F10-bearing mice prepared by the method described in Example 5 to conduct a mouse tumor growth test. Figure 20-1 shows changes in average tumor volume, and Figure 20-2 shows changes in each individual.

[Table 20]

Table 20 Administration sample

| Abbreviation | Carrier | Peptide species, Dose | Adjuvant species, Dose | Administration day (after transplantation) |
|---|---|---|---|---|
| 99k42 | 99k HA-$C_6$-Chol-42% | TRP2TRP1gp100-6Y 0.05 mg | CpG 0.05 mg | Day 10, day 14, day 17, day 21 |
| 50k42 | 50k HA-$C_6$-Chol-42% | TRP2TRP1gp100-6Y 0.05 mg | CpG 0.05 mg | Day 10, day 14, day 17, day 21 |
| 10k43 | 10k HA-$C_6$-Chol-43% | TRP2TRP1gp100-6Y 0.05 mg | CpG 0.05 mg | Day 10, day 14, day 17, day 21 |

The HA derivative (50k42) group and the HA derivative (10k43) group, which differed in the molecular weight of HA from the HA derivative (99k42) group, were also shown to have a high tumor growth suppressive effect, as in the HA derivative (99k42) group.

(Example 7-2) Mouse melanoma B16F10

Each sample of Table 21 was subcutaneously administered with the frequency described in Table 21 to the

mouse melanoma B16F10-bearing mice prepared by the method described in Example 5 to conduct a mouse tumor growth test. Figure 21-1 shows changes in average tumor volume, and Figure 21-2 shows changes in each individual.

[Table 21]

Table 21 Administration sample

| Abbreviation | Carrier | Peptide species, Dose | Adjuvant species, Dose | Administration day (after transplantation) |
|---|---|---|---|---|
| 10kHA-Ala-Chol30 | 10kHA-Ala-$C_6$-Chol-30% | TRP2TRP1gp100-6Y 0.05 mg | CpG 0.05 mg | Day 10, day 14, day 17, day 20 |
| 10kHA-Gln-Chol32 | 10kHA-Gln-$C_6$-Chol-32% | TRP2TRP1gp100-6Y 0.05 mg | CpG 0.05 mg | Day 10, day 14, day 17, day 20 |

The amino acid-modified HA derivative groups (10kHA-Ala-Chol30 and 10kHA-Gln-Chol32) were also shown to have a high tumor growth suppressive effect.

(Example 7-3) Mouse melanoma B16F10

Each sample of Table 22 was subcutaneously administered with the frequency described in Table 22 to the mouse melanoma B16F10-bearing mice prepared by the method described in Example 5 to conduct a mouse tumor growth test. Figure 22-1 shows changes in average tumor volume, and Figure 22-2 shows changes in each individual.

[Table 22]

Table 22 Administration sample

| Abbreviation | Carrier | Peptide species, Dose | Adjuvant species, Dose | Administration day (after transplantation) |
|---|---|---|---|---|
| 99k42+PolyIC | 99k HA-$C_6$-Chol-42% | TRP2TRP1gp100-6Y 0.05 mg | PolyIC 0.05 mg | Day 10, day 14, day 17, day 21 |

Use of polyIC as an adjuvant was also shown to produce a high tumor growth suppressive effect.

(Example 7-4) Mouse melanoma B16F10

Each sample of Table 23 was subcutaneously administered with the frequency described in Table 23 to the mouse melanoma B16F10-bearing mice prepared by the method described in Example 5 to conduct a mouse tumor growth test. Figure 23-1 shows changes in average tumor volume, and Figure 23-2 shows changes in each individual.

[Table 23]

Table 23 Administration sample

| Abbreviation | Carrier | Peptide species, Dose | Adjuvant species, Dose | Administration day (after transplantation) |
|---|---|---|---|---|
| 99k42+Sting | 99k HA-$C_6$-Chol-42% | TRP2TRP1gp100-6Y 0.05 mg | Sting 0.015 mg | Day 10, day 14, day 17, day 20 |
| 99k42+R848 | 99k HA-$C_6$-Chol-42% | TRP2TRP1gp100-6Y 0.05 mg | R848 0.05 mg | Day 10, day 14, day 17, day 20 |

Use of Sting or R848 as an adjuvant was also shown to produce a high tumor growth suppressive effect.

[Example 8] Tumor and lymph node analysis

Each sample of Table 24 was subcutaneously administered with the frequency described in Table 24 to the mouse melanoma B16F10-bearing mice prepared by the method described in Example 5. On days 12, 14, and 18 after transplantation, tumor and lymph node (from a total of 4 locations of the right and left infra-axillary portions and the right and left inguinal portions) were harvested to conduct tetramer assay (mentioned later). The harvested tumor and lymph node from 2 animals were pooled, and two samples (from 4 animals) per group were analyzed. Figure 24-1 shows the ratio of the number of TRP-2 antigen-specific $CD8^+$ cells to the number of $CD8^+$ cells, and Figure 24-2 shows the ratio of the number of gp100 antigen-specific $CD8^+$ cells to the number of $CD8^+$ cells.

[Table 24]

Table 24 Administration sample

| Abbreviation | Carrier | Peptide species, Dose | Adjuvant species, Dose | Administration day (after transplantation) |
|---|---|---|---|---|
| 99k41 | 99k HA-$C_6$-Chol-41% | TRP2TRP1gp100-6Y 0.05 mg | CpG 0.05 mg | Day 10, day 13, day 17 |

It was shown that the TRP-2 antigen-specific $CD8^+$ cells and the gp100 antigen-specific $CD8^+$ cells invaded tumor and lymph node. This indicates that the antitumor effect brought about by the HA derivative is caused by antigen-specific CD8 cells.

Tetramer assay

Each harvested tumor tissue sample was placed in a 6-well plate containing 1 mL/well of RPMI-1640, chopped into 2 mm square or smaller with scissors, and then recovered into gentleMACS C-tube (Miltenyi Biotech). After addition of Enzyme Mix (Miltenyi Biotech), the plate was loaded in gentleMACS Dissociator (Miltenyi Biotech), and the sample was disrupted. Then, the plate was incubated at 37°C for 40 minutes and loaded again in gentleMACS Dissociator, and the sample was disrupted. The obtained cell suspension was passed through a strainer and then centrifuged (300 $\times$ g, 5 min, 4°C), and the pellets were suspended in MACS Buffer (Miltenyi Biotech) to prepare a cell suspension.

The harvested lymph node was mashed using the tail of the inner cylinder of a syringe to prepare a cell suspension.

5 $\times$ $10^7$ cells/mL of the tumor cell suspension or the lymph node cell suspension was added at 20 $\mu$L/well to a 96-well V-bottom microplate. A mouse FcR blocking reagent diluted 50-fold was added at 10 $\mu$L/well and reacted at 4°C for 5 minutes. H-$2K^b$ TRP-2 Tetramer-SVYDFFVWL-APC (Medical & Biological Laboratories Co., Ltd. (MBL)) or H-$2D^b$ gp100 Tetramer-EGSRNQDWL-PE (MBL) was added at 10 $\mu$L/well and reacted at 4°C for 30 minutes with light shielded. MACS Buffer was added at 200 $\mu$L/well, and a supernatant was removed by centrifugation (310 to 400 $\times$ g, 5 min, 4°C). This operation was repeated twice. Next, an antibody solution containing a fluorescently labeled anti-CD8 antibody (MBL) was added at 20 $\mu$L/well and reacted at 4°C for 30 minutes with light shielded. MACS Buffer was added

at 200 μL/well, and a supernatant was removed by centrifugation (310 to 400 × g, 5 min, 4°C). This operation was repeated twice. The cells were suspended by the addition of MACS Buffer at 200 μL/well, and analyzed in a flow cytometer (BD LSRFortessa X-20, BD Biosciences) using attached analytical software (FACSDiva).

## Claims

1. A vaccine formulation for use in the prevention or treatment of a cancer, comprising a hyaluronic acid derivative having an introduced hydrophobic group, and an antigen, wherein

   the hyaluronic acid derivative having an introduced hydrophobic group is the following:
   a hyaluronic acid derivative comprising at least one repeating unit represented by the formula (I):

[Formula 1]

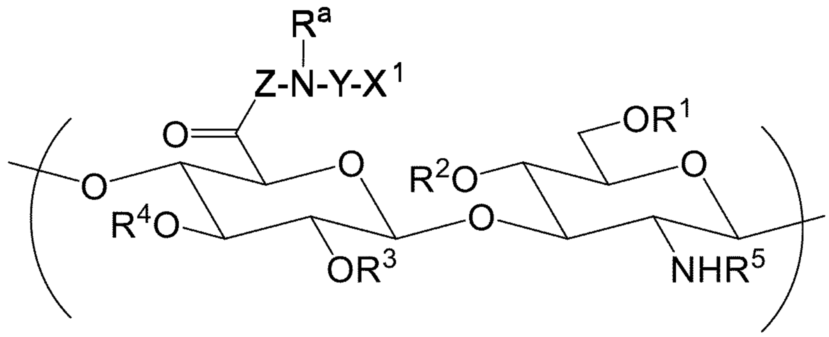

(I)

wherein $R^1$, $R^2$, $R^3$, and $R^4$ are each independently selected from a hydrogen atom, $C_{1-6}$ alkyl, formyl and $C_{1-6}$ alkylcarbonyl;

$R^5$ is a hydrogen atom, formyl, or $C_{1-6}$ alkylcarbonyl;
Z represents a direct bond, or a peptide linker having 2 to 30 arbitrary amino acid residues;
$X^1$ is a hydrophobic group selected from groups represented by the following formulas:

$-NR^b-R$,

$-NR^b-COO-R$,

$-NR^b-CO-R$,

$-NR^b-CO-NR^c-R$,

-COO-R,

-O-COO-R,

-S-R,

$-CO-Y^a-S-R$,

$-O-CO-Y^b-S-R$,

$-NR^b-CO-Y^b-S-R$,

and

-S-S-R;

$R^a$, $R^b$ and $R^c$ are each independently selected from a hydrogen atom, $C_{1-20}$ alkyl, amino-$C_{2-20}$ alkyl and hydroxy-$C_{2-20}$ alkyl, wherein in the alkyl moiety of each of the groups, 1 to 3 groups each independently selected from -O- and $-NR^f-$ are optionally inserted;
$R^f$ is selected from a hydrogen atom, $C_{1-12}$ alkyl, amino-$C_{2-12}$ alkyl and hydroxy-$C_{2-12}$ alkyl, wherein in the alkyl moiety of each of the groups, 1 or 2 groups each independently selected from -O- and -NH- are optionally inserted;
R is a steryl group;
Y is $C_{2-30}$ alkylene, or $-(CH_2CH_2O)_m-CH_2CH_2-$, wherein in the alkylene, 1 to 5 groups each independently selected from -O-, $-NR^g-$ and -S-S- are optionally inserted;
$R^g$ is selected from a hydrogen atom, $C_{1-20}$ alkyl, amino-$C_{2-20}$ alkyl or hydroxy-$C_{2-20}$ alkyl, wherein in the alkyl moiety of each of the groups, 1 to 3 groups each independently selected from -O- and -NH- are optionally inserted;
$Y^a$ is $C_{1-5}$ alkylene;

$Y^b$ is $C_{2-8}$ alkylene or $C_{2-8}$ alkenylene; and
m is an integer selected from 1 to 100; or

a hyaluronic acid derivative comprising a repeating unit represented by the formula (II):

[Formula 2]

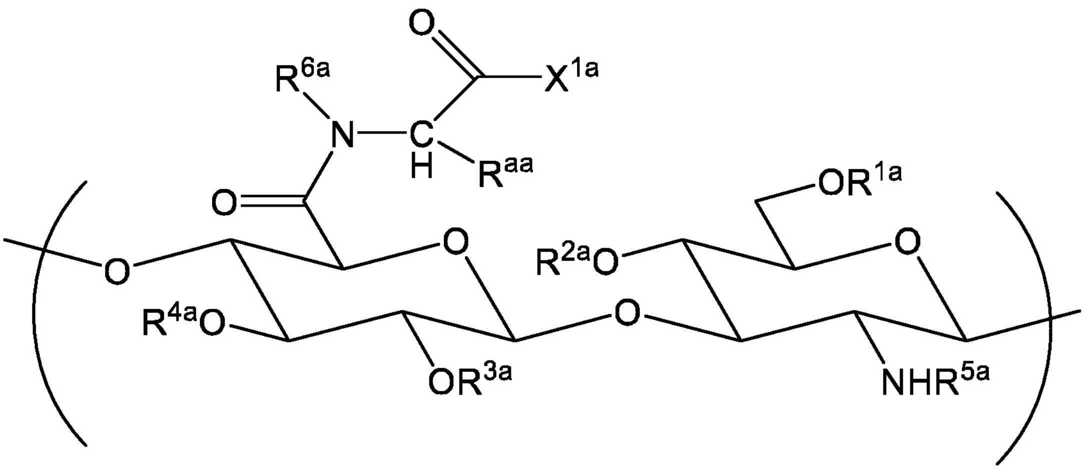

(II)

wherein $R^{1a}$, $R^{2a}$, $R^{3a}$, and $R^{4a}$ are each independently selected from a hydrogen atom, $C_{1-6}$ alkyl, formyl, and $C_{1-6}$ alkylcarbonyl;

$R^{5a}$ is a hydrogen atom, formyl, or $C_{1-6}$ alkylcarbonyl;
$X^{1a}$ is hydroxy, $-O^-Q^+$, $C_{1-6}$ alkoxy, $-NR^7R^8$, or $-NR^9-Z^1-Z^2$;
$Q^+$ represents a counter cation;
$R^{6a}$, $R^7$, $R^8$, and $R^9$ are each independently selected from a hydrogen atom, and $C_{1-6}$ alkyl;
$R^{aa}$ is a hydrogen atom, or $C_{1-6}$ alkyl, wherein the alkyl is optionally substituted by one or more groups each independently selected from hydroxy, carboxy, carbamoyl, $C_{1-6}$ alkylthio, aryl, and heteroaryl, wherein the aryl is optionally substituted by one or more hydroxy groups;
$Z^1$ is $C_{2-30}$ alkylene, or $-(CH_2CH_2O)_{ma}-CH_2CH_2-$, wherein in the alkylene, 1 to 5 groups each independently selected from-O-, $-NR^{ga}-$ and -S-S- are optionally inserted, and ma is an integer selected from 1 to 100;
$Z^2$ is selected from groups represented by the following formulas:

$-NR^{ba}-Z^3$,

$-NR^{ba}-COO-Z^3$,

$-NR^{ba}-CO-Z^3$,

$-NR^{ba}-CO-NR^{ca}-Z^3$,

$-COO-Z^3$,

$-CO-NR^{ca}-Z^3$,

$-O-CO-NR^{ca}-Z^3$,

$-O-COO-Z^3$,

$-S-Z^3$,

$-CO-Z^a-S-Z^3$,

$-O-CO-Z^b-S-Z^3$,

$-NR^{ba}-CO-Z^b-S-Z^3$,

and

$-S-S-Z^3$;

$R^{ba}$ and $R^{ca}$ are each independently selected from a hydrogen atom, $C_{1-20}$ alkyl, amino-$C_{2-20}$ alkyl and hydroxy-$C_{2-20}$ alkyl, wherein in the alkyl moiety of each of the groups, 1 to 3 groups each independently selected from -O- and $-NR^{fa}-$ are optionally inserted;
$R^{fa}$ is independently selected from a hydrogen atom, $C_{1-12}$ alkyl, amino-$C_{2-12}$ alkyl and hydroxy-$C_{2-12}$ alkyl, wherein in the alkyl moiety of each of the groups, 1 or 2 groups each independently selected from -O- and -NH- are optionally inserted;
$R^{ga}$ is independently selected from a hydrogen atom, $C_{1-20}$ alkyl, amino-$C_{2-20}$ alkyl and hydroxy-$C_{2-20}$ alkyl, wherein in the alkyl moiety of each of the groups, 1 to 3 groups each independently selected from -O- and -NH- are optionally inserted;
$Z^3$ is a steryl group;
$Z^a$ is $C_{1-5}$ alkylene; and
$Z^b$ is $C_{2-8}$ alkylene or $C_{2-8}$ alkenylene,

the hyaluronic acid derivative, when not comprising a repeating unit represented by the formula (II) wherein $X^{1a}$ is $-NR^9-Z^1-Z^2$, further comprising a repeating unit represented by the formula (III):

[Formula 3]

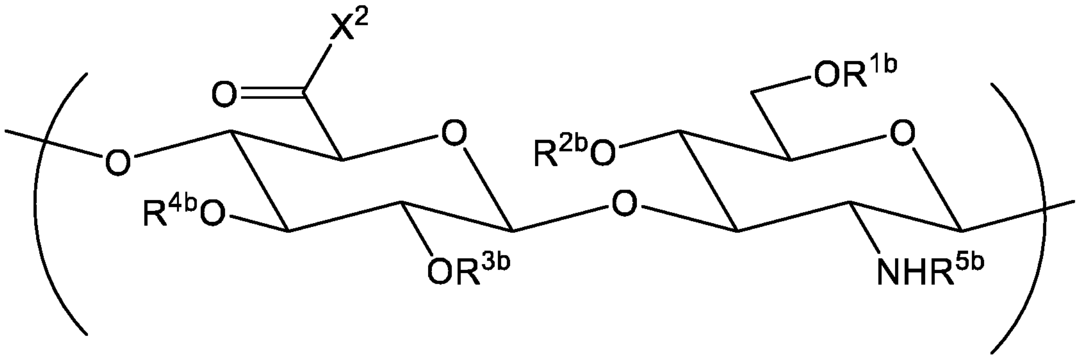

(III)

wherein $R^{1b}$, $R^{2b}$, $R^{3b}$ and $R^{4b}$ are each independently selected from a hydrogen atom, $C_{1-6}$ alkyl, formyl, and $C_{1-6}$ alkylcarbonyl;

$R^{5b}$ is a hydrogen atom, formyl, or $C_{1-6}$ alkylcarbonyl; and
$X^2$ is $-NR^9-Z^1-Z^2$, wherein $R^9$, $Z^1$, and $Z^2$ are as already defined.

2. The vaccine formulation according to claim 1, wherein the hyaluronic acid derivative having an introduced hydrophobic group further comprises a repeating unit represented by the formula (IIIc):

[Formula 4]

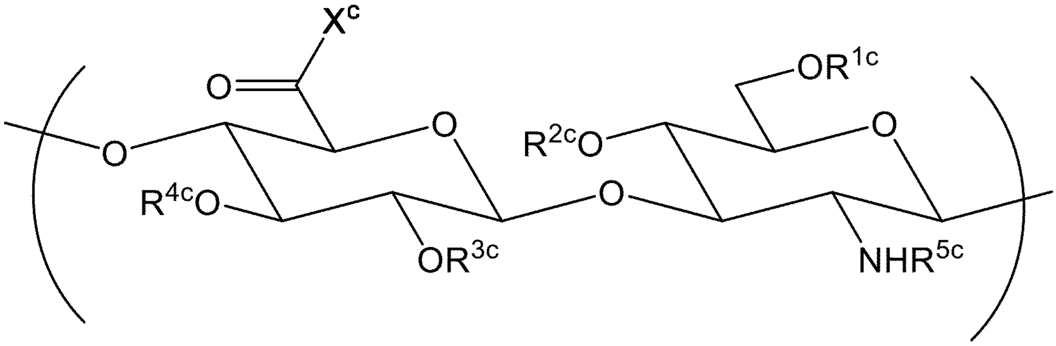

(IIIc)

wherein $R^{1c}$, $R^{2c}$, $R^{3c}$ and $R^{4c}$ are each independently selected from a hydrogen atom, $C_{1-6}$ alkyl, formyl and $C_{1-6}$ alkylcarbonyl;

$R^{5c}$ is selected from a hydrogen atom, formyl and $C_{1-6}$ alkylcarbonyl; and
$X^c$ is selected from hydroxy and $-O^-Q^+$, wherein $Q^+$ represents a counter cation.

**3.** The vaccine formulation according to claim 1 or 2, wherein the vaccine formulation comprises a hyaluronic acid derivative comprising a repeating unit represented by the formula (I), wherein a ratio of the repeating unit represented by the formula (I) to disaccharide repeating units present is 5 to 50%.

**4.** The vaccine formulation according to claim 1 or 2, wherein the vaccine formulation comprises a hyaluronic acid derivative comprising a repeating unit represented by the formula (II), wherein a ratio of a disaccharide unit comprising the group $-NR^9-Z^1-Z^2$ to disaccharide repeating units present is 5 to 50%.

**5.** The vaccine formulation according to any one of claims 1 to 3, wherein the vaccine formulation comprises a hyaluronic acid derivative comprising a repeating unit represented by the formula (I), wherein Z is a direct bond, Y is $C_{2-10}$ alkylene, $X^1$ is -NH-COO-R, and R is a cholesteryl group.

**6.** The vaccine formulation according to any one of claims 1, 2 and 4, wherein the vaccine formulation comprises a hyaluronic acid derivative comprising a repeating unit represented by the formula (II), wherein $Z^1$ is $C_{2-10}$ alkylene, $Z^2$ is $-NH-COO-Z^3$, and $Z^3$ is a cholesteryl group.

**7.** The vaccine formulation according to any one of claims 1 to 6, wherein the hyaluronic acid derivative is produced using hyaluronic acid composed only of a disaccharide unit represented by the formula (IIIc) defined in claim 2, wherein when all of $R^{1c}$, $R^{2c}$, $R^{3c}$, and $R^{4c}$ are hydrogen atoms, $R^{5c}$ is acetyl, and $X^c$ is $-O^-Na^+$, a weight-average molecular weight is 5 kilodaltons to 200 kilodaltons.

**8.** The vaccine formulation according to any one of claims 1 to 7, wherein the hyaluronic acid derivative and the antigen form a complex.

**9.** The vaccine formulation according to any one of claims 1 to 8 for administration in combination with at least one type of adjuvant.

**10.** The vaccine formulation according to any one of claims 1 to 9, wherein the antigen is an antigenic peptide or an antigenic protein.

**11.** The vaccine formulation according to claim 10, wherein the antigenic peptide comprises two or more CD8-positive cytotoxic T cell recognition epitopes or CD4-positive helper T cell recognition epitopes.

**12.** The vaccine formulation according to claim 11, wherein the antigenic peptide has an amino acid linker between the epitopes.

**13.** The vaccine formulation according to any one of claims 1 to 12 for administration in combination with at least one

type of antibody for use in cancer treatment.

**14.** A complex formed from a hyaluronic acid derivative comprising a repeating unit represented by the formula (I) or the formula (II) according to any one of claims 1 to 7, and an antigen that is used in a vaccine for use in the prevention or treatment of a cancer.

# Figure 1

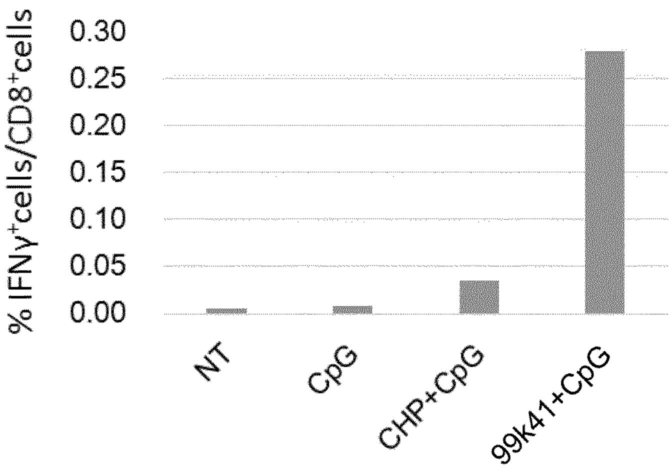


# Figure 2-1

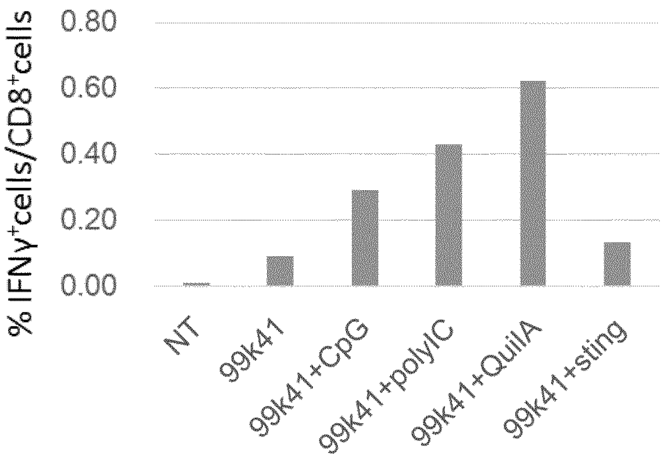

Figure 2-2

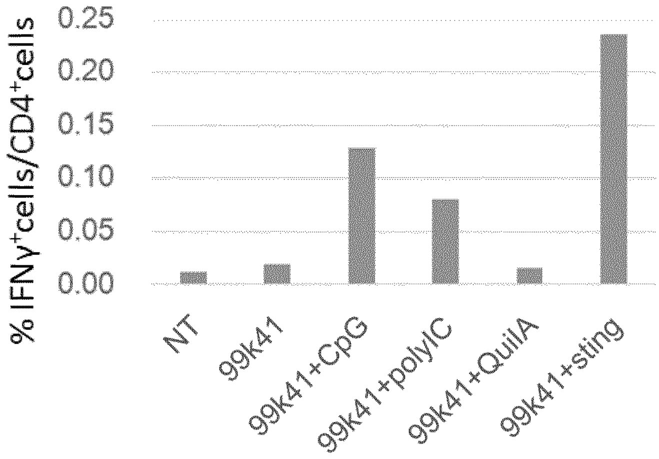


Figure 3-1

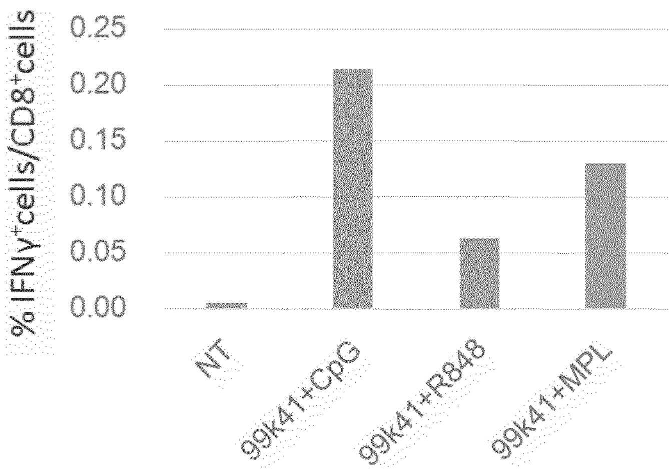

# Figure 3-2

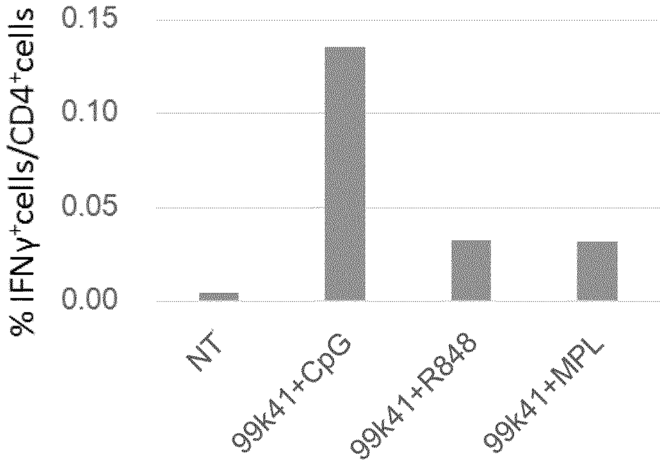


# Figure 4

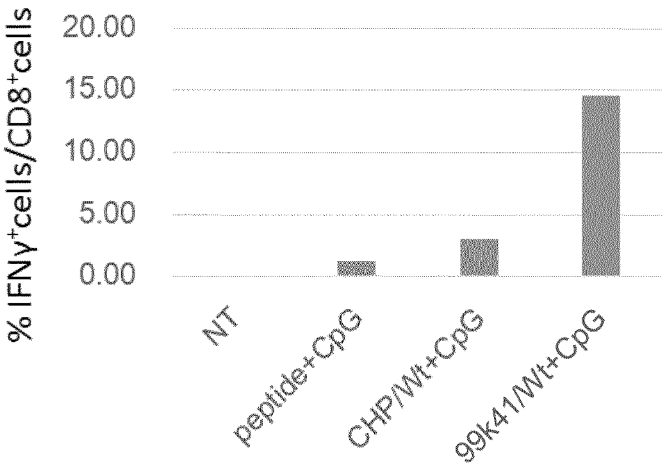

# Figure 5

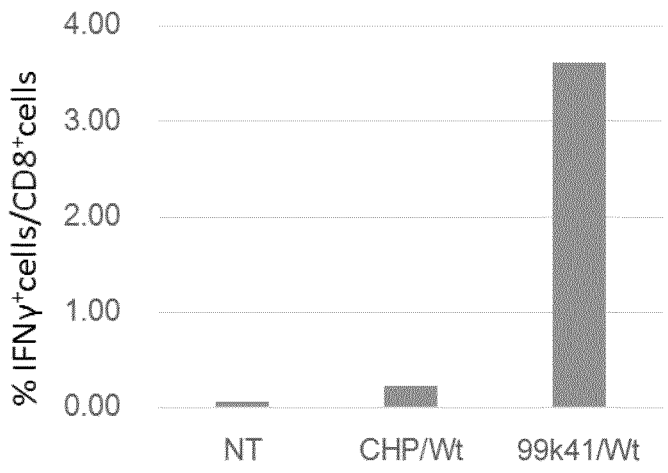


# Figure 6

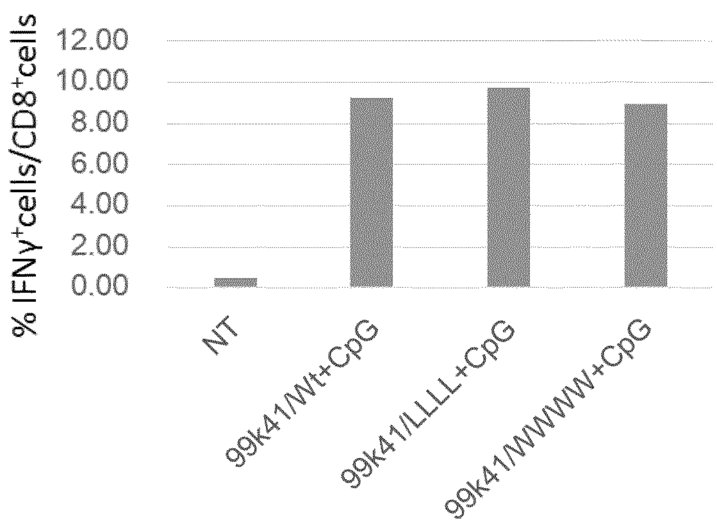

Figure 7-1

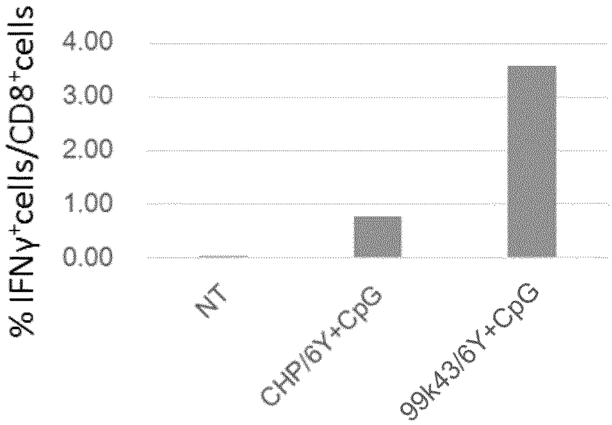


Figure 7-2

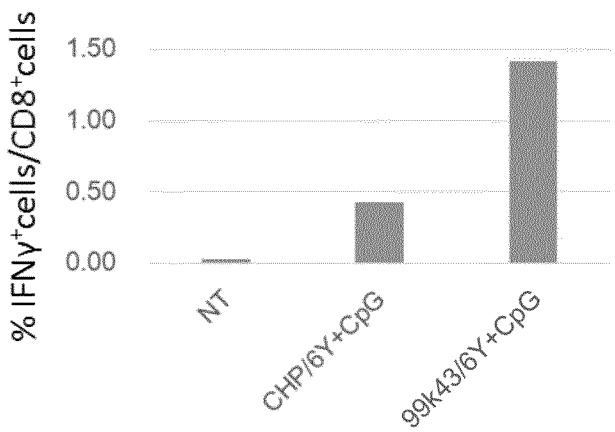

# Figure 7-3

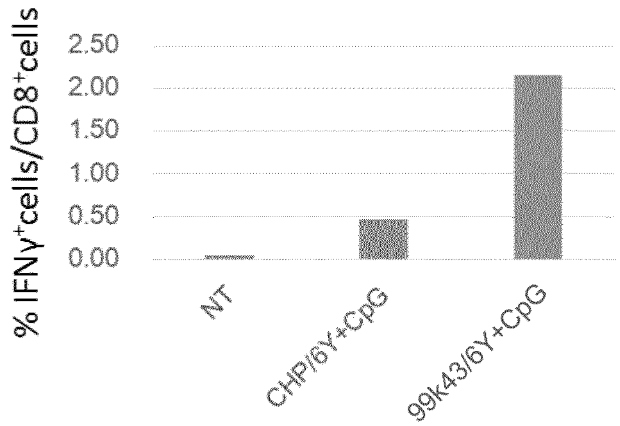


# Figure 8-1

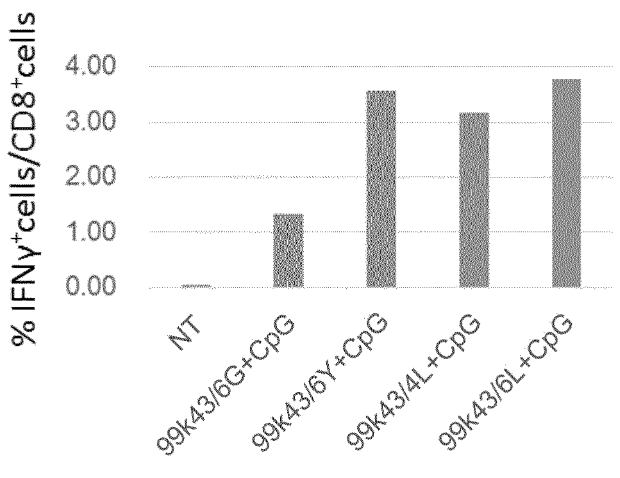

# Figure 8-2

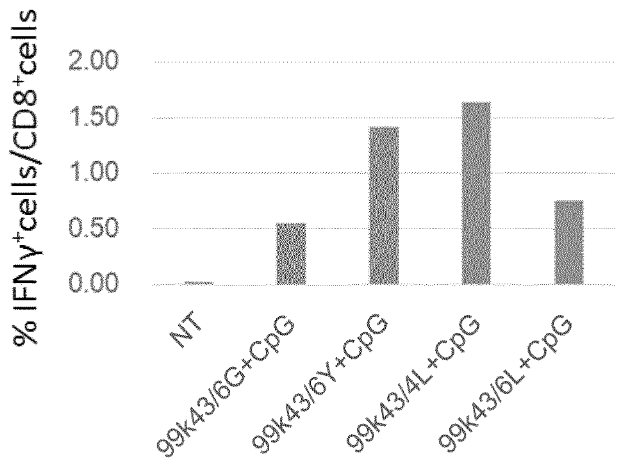


# Figure 8-3

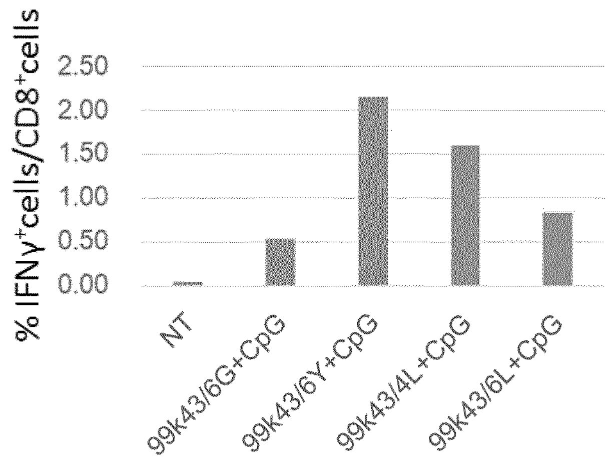

# Figure 9-1

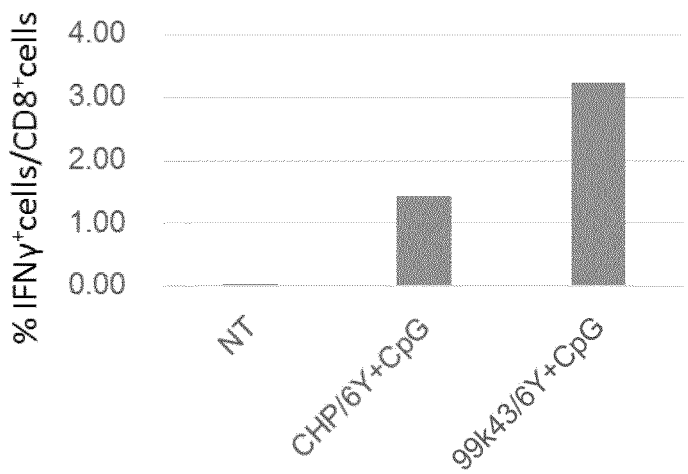


# Figure 9-2

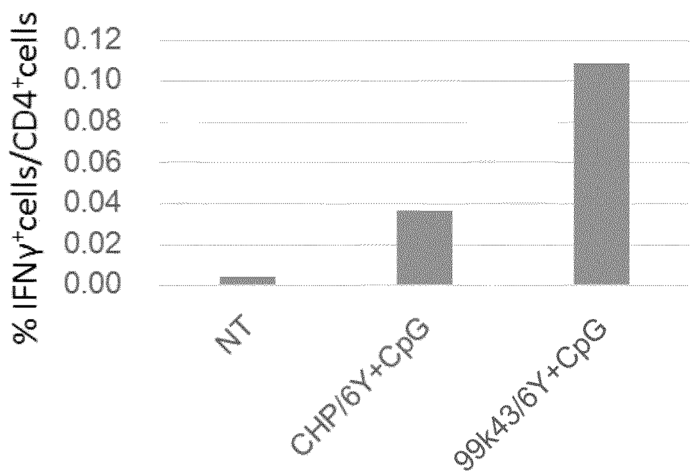

# Figure 10-1

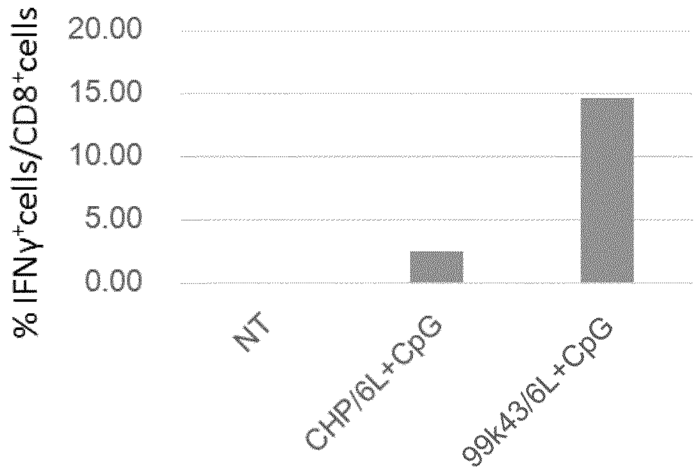


# Figure 10-2

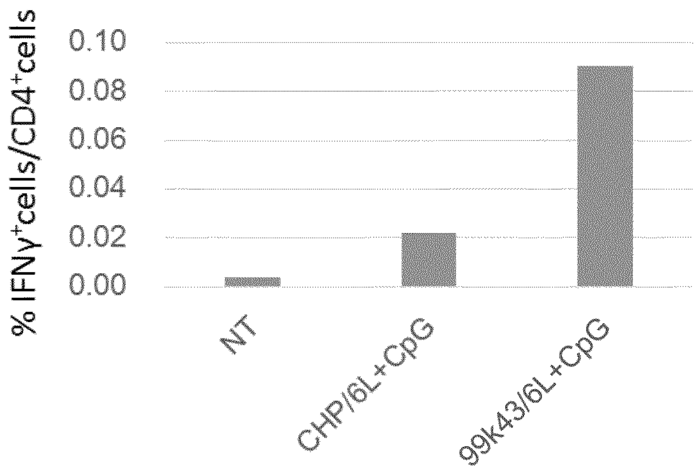

# Figure 11-1

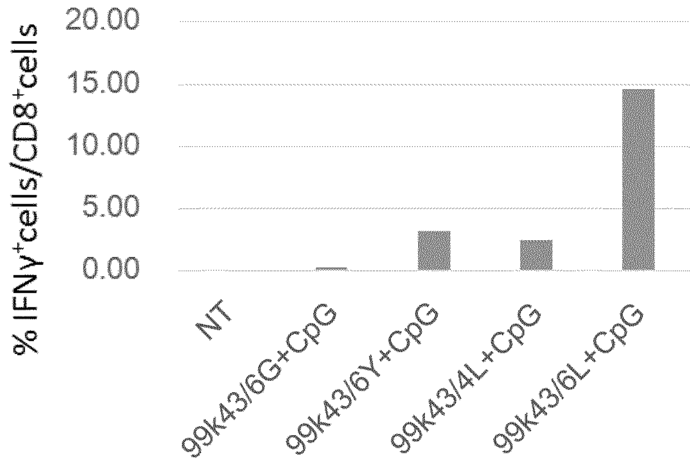


# Figure 11-2

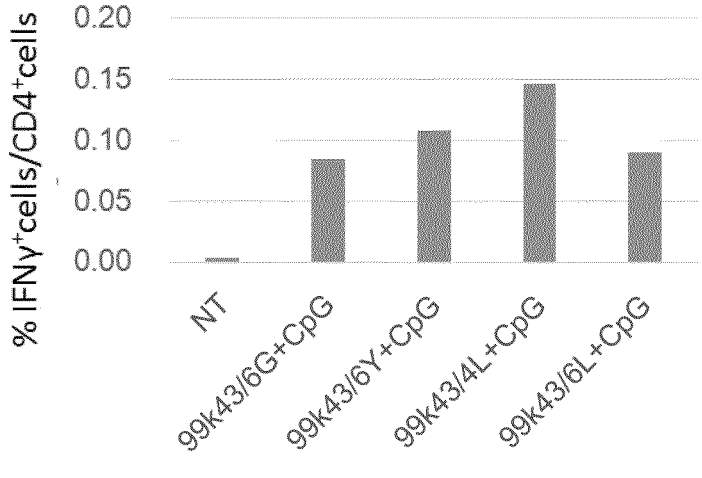

# Figure 12-1

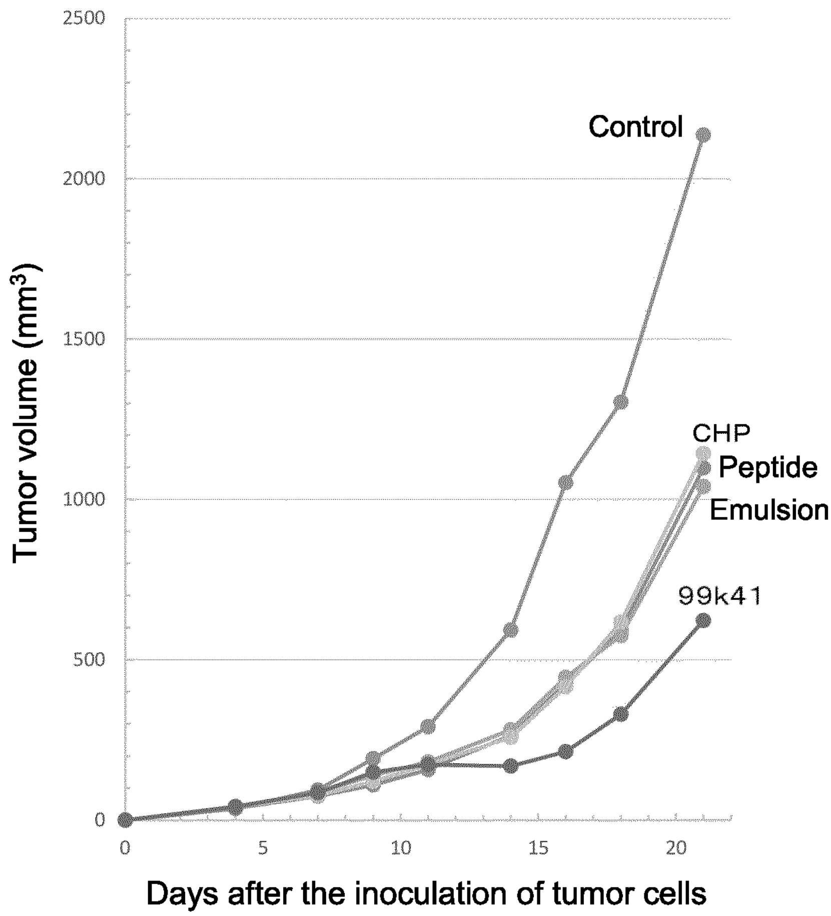
2500
2000
1500
1000
500
0
Tumor volume (mm3)
Control
CHP
Peptide
Emulsion
99k41
0
5
10
15
20
Days after the inoculation of tumor cells

# Figure 12-2

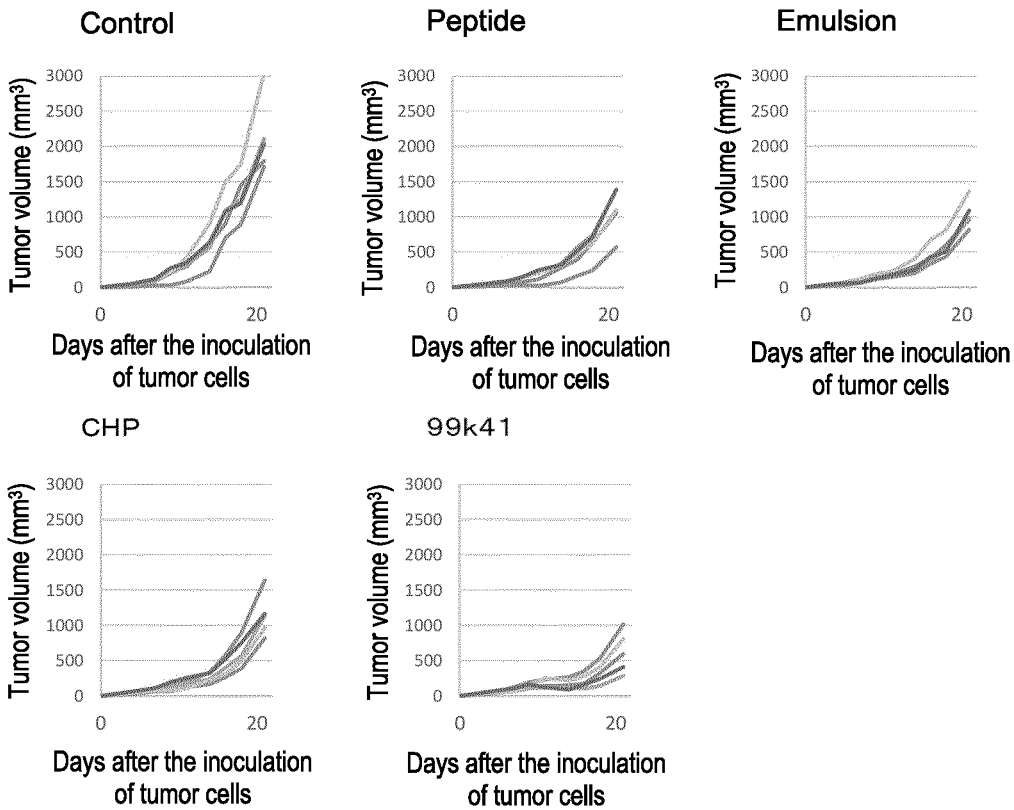
Control
Peptide
Emulsion
CHP
99k41
Tumor volume (mm3)
3000
2500
2000
1500
1000
500
0
0
20
Days after the inoculation of tumor cells

# Figure 13-1

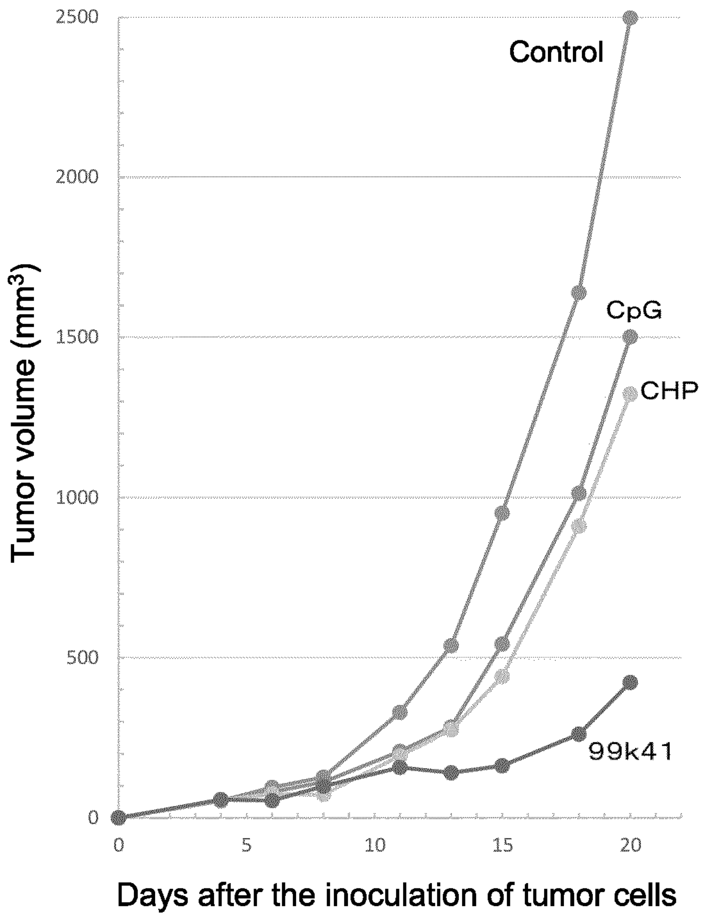
2500
2000
1500
1000
500
0
0
5
10
15
20
Tumor volume (mm3)
Days after the inoculation of tumor cells
Control
CpG
CHP
99k41

# Figure 13-2

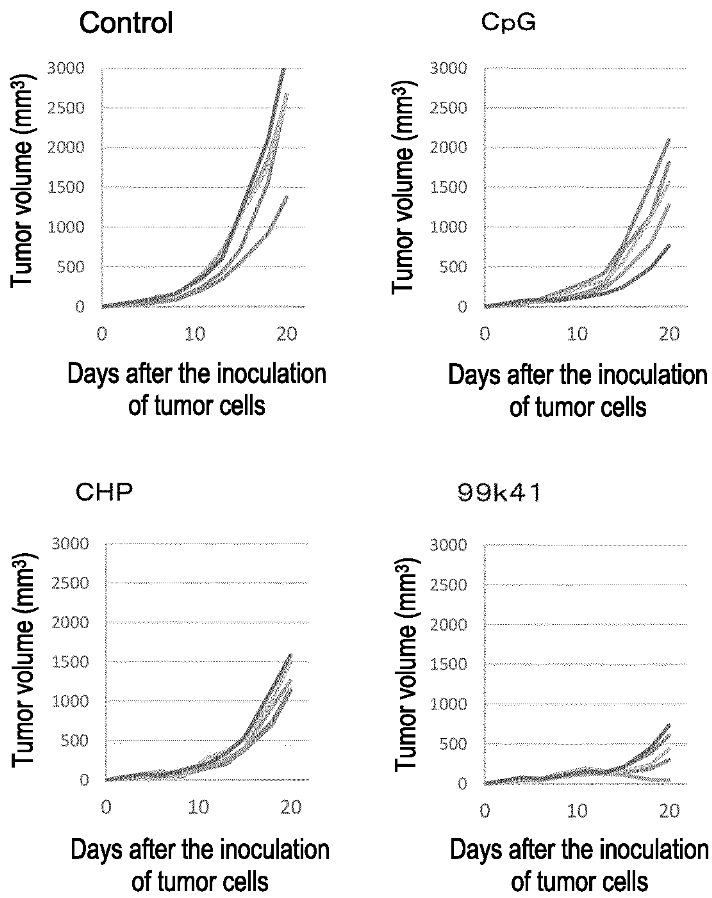
Control
CpG
CHP
99k41
Tumor volume (mm3)
3000
2500
2000
1500
1000
500
0
0
10
20
Days after the inoculation of tumor cells

# Figure 14-1

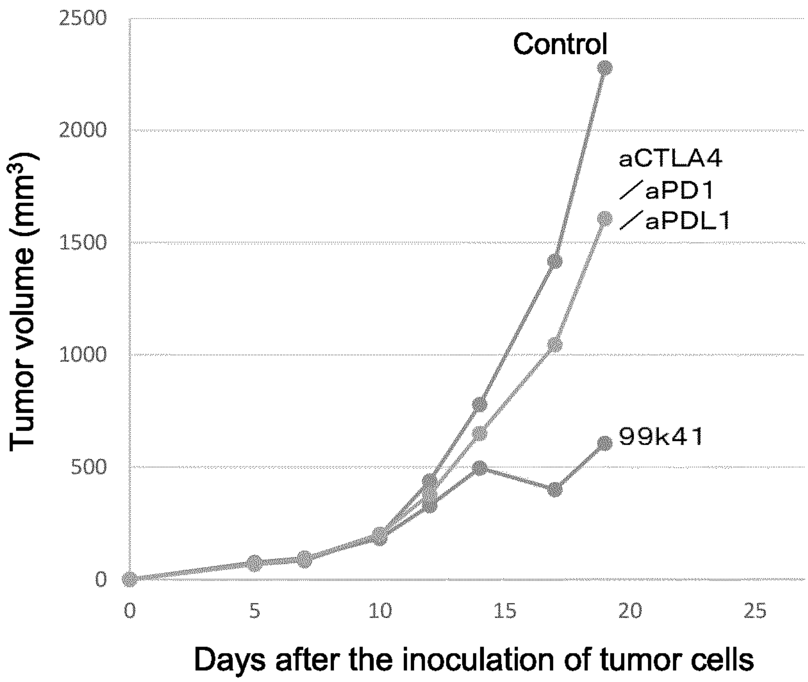
2500
2000
1500
1000
500
0
Tumor volume (mm3)
Control
aCTLA4
／aPD1
／aPDL1
99k41
0
5
10
15
20
25
Days after the inoculation of tumor cells

# Figure 14-2

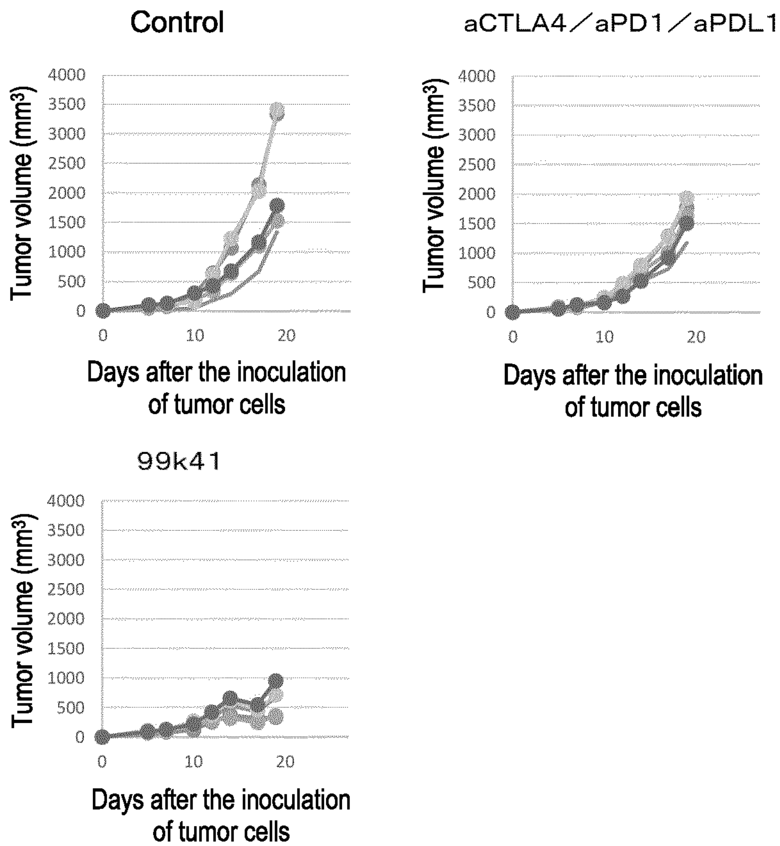

Control
aCTLA4／aPD1／aPDL1
99k41
Tumor volume (mm³)
4000
3500
3000
2500
2000
1500
1000
500
0
0
10
20
Days after the inoculation of tumor cells

# Figure 15-1

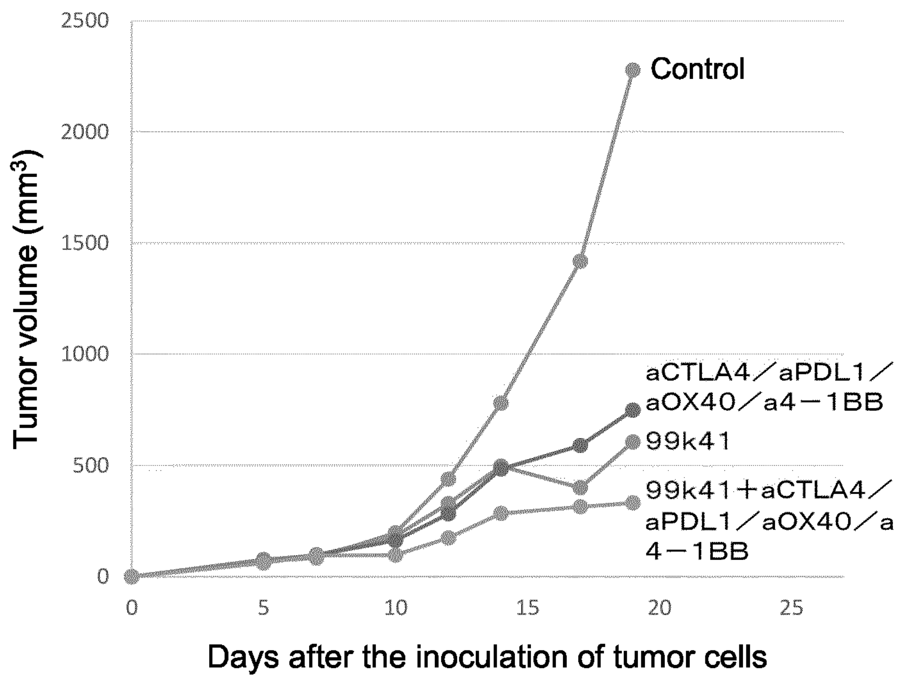
2500
2000
1500
1000
500
0
Tumor volume (mm3)
Control
aCTLA4／aPDL1／
aOX40／a4－1BB
99k41
99k41＋aCTLA4／
aPDL1／aOX40／a
4－1BB
0
5
10
15
20
25
Days after the inoculation of tumor cells

# Figure 15-2

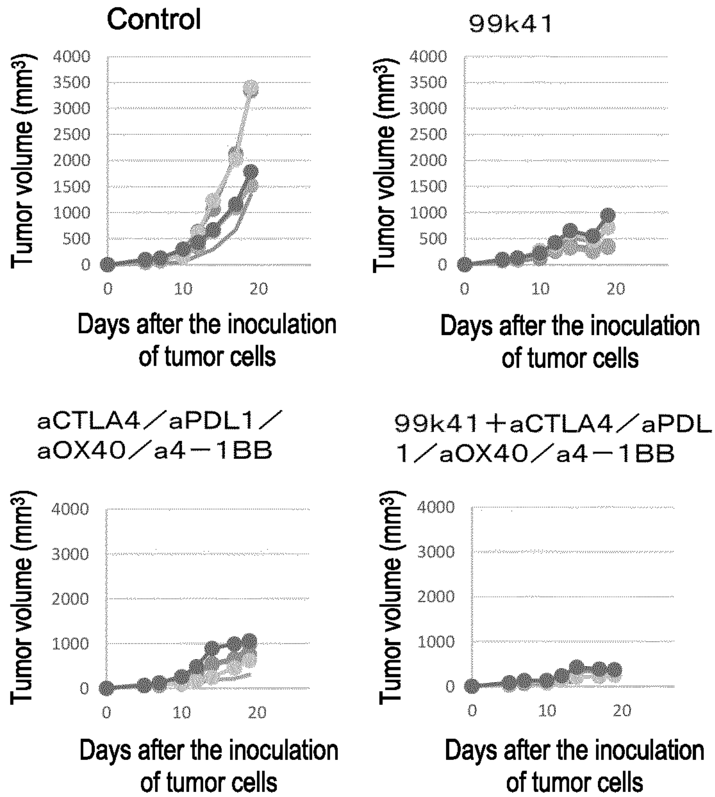
Control
Tumor volume (mm³)
4000
3500
3000
2500
2000
1500
1000
500
0
0
10
20
Days after the inoculation of tumor cells
99k41
Tumor volume (mm³)
Days after the inoculation of tumor cells
aCTLA4／aPDL1／aOX40／a4－1BB
Tumor volume (mm³)
Days after the inoculation of tumor cells
99k41＋aCTLA4／aPDL1／aOX40／a4－1BB
Tumor volume (mm³)
Days after the inoculation of tumor cells

# Figure 16-1

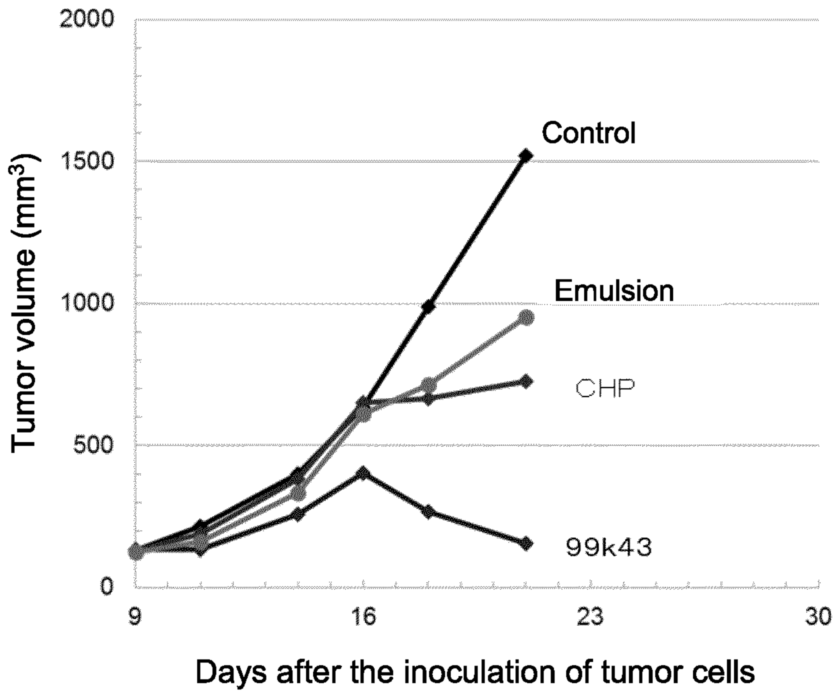
2000
1500
1000
500
0
Tumor volume (mm3)
Control
Emulsion
CHP
99k43
9
16
23
30
Days after the inoculation of tumor cells

# Figure 16-2

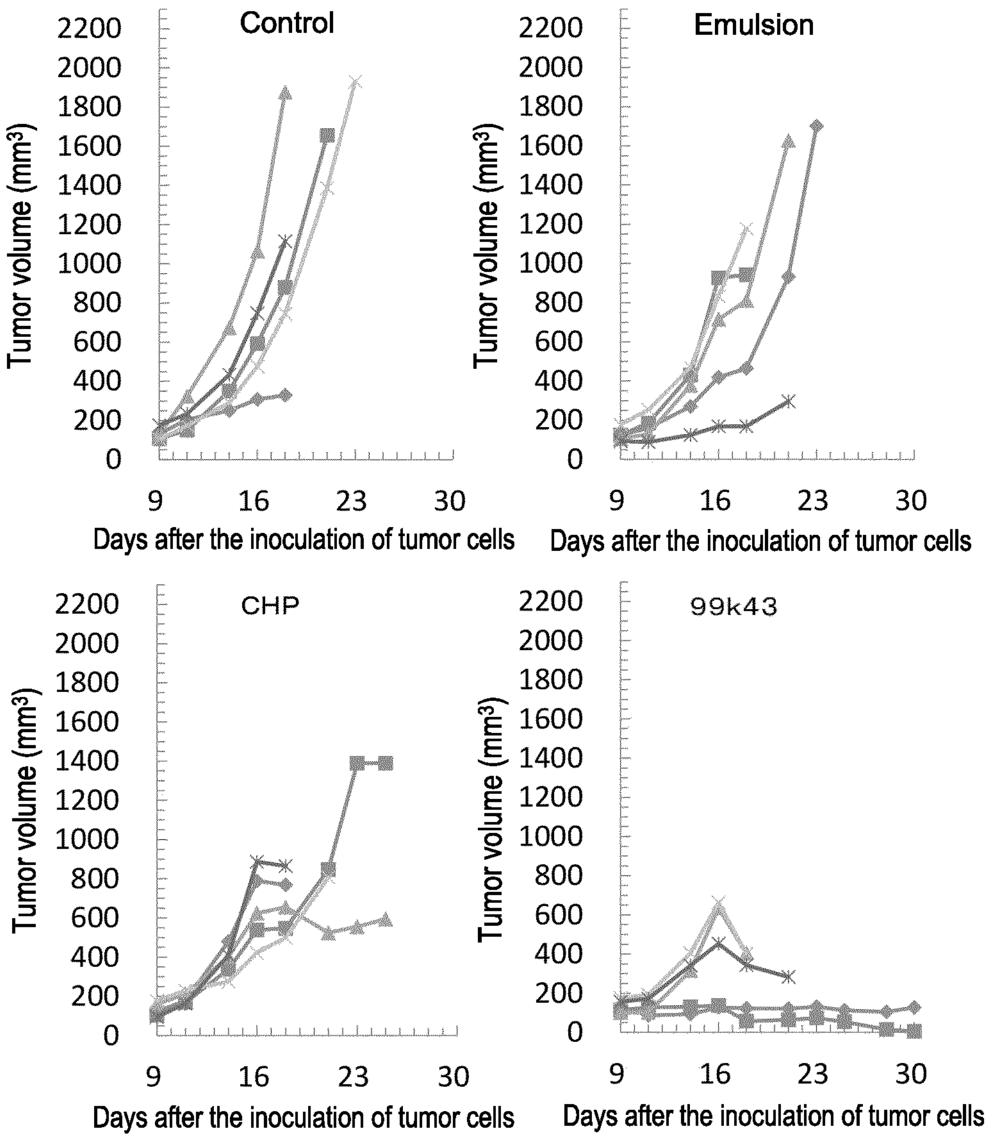
Control
Emulsion
CHP
99k43
Tumor volume (mm3)
2200
2000
1800
1600
1400
1200
1000
800
600
400
200
0
9
16
23
30
Days after the inoculation of tumor cells

# Figure 17-1

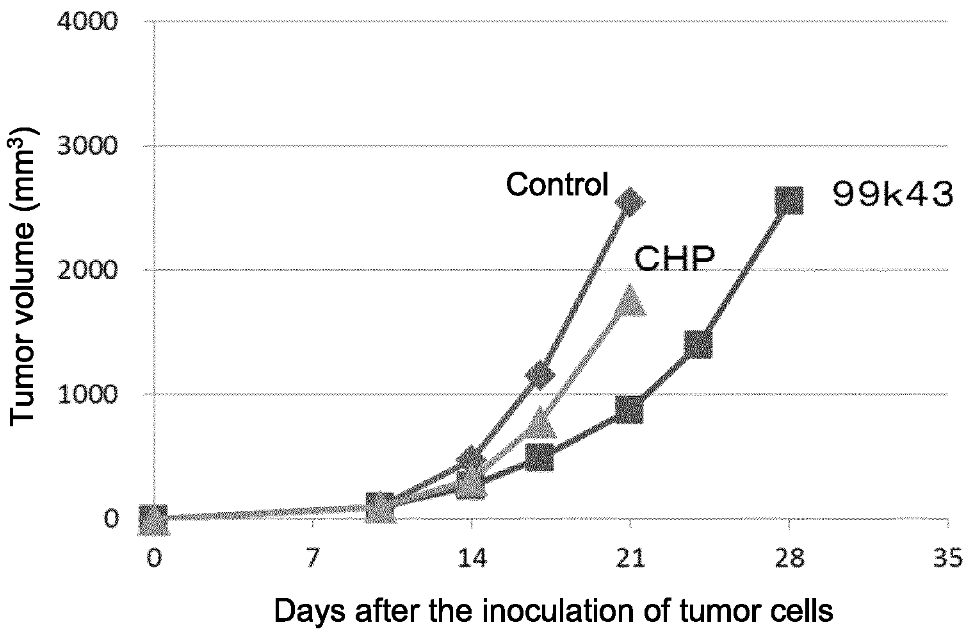
4000
3000
2000
1000
0
Tumor volume (mm3)
Control
CHP
99k43
0
7
14
21
28
35
Days after the inoculation of tumor cells

# Figure 17-2

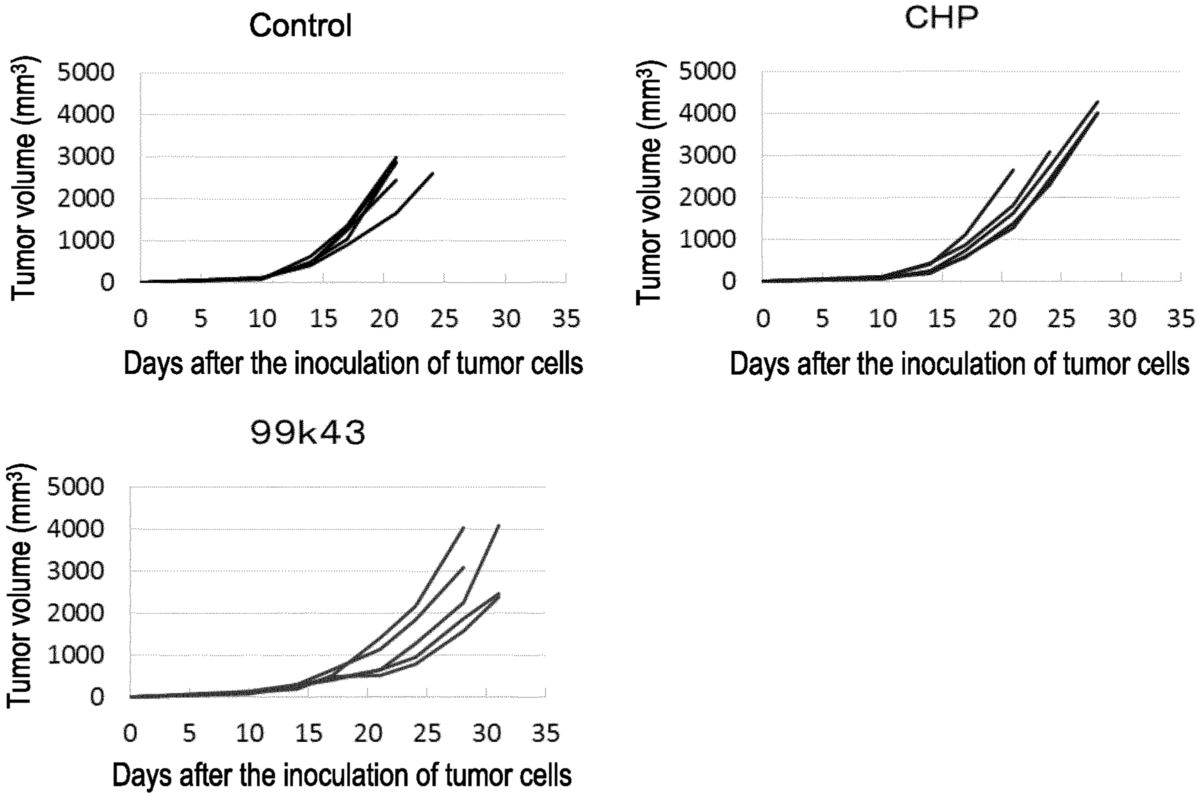
Control
Tumor volume (mm3)
5000
4000
3000
2000
1000
0
0 5 10 15 20 25 30 35
Days after the inoculation of tumor cells
CHP
Tumor volume (mm3)
5000
4000
3000
2000
1000
0
0 5 10 15 20 25 30 35
Days after the inoculation of tumor cells
99k43
Tumor volume (mm3)
5000
4000
3000
2000
1000
0
0 5 10 15 20 25 30 35
Days after the inoculation of tumor cells

# Figure 18-1

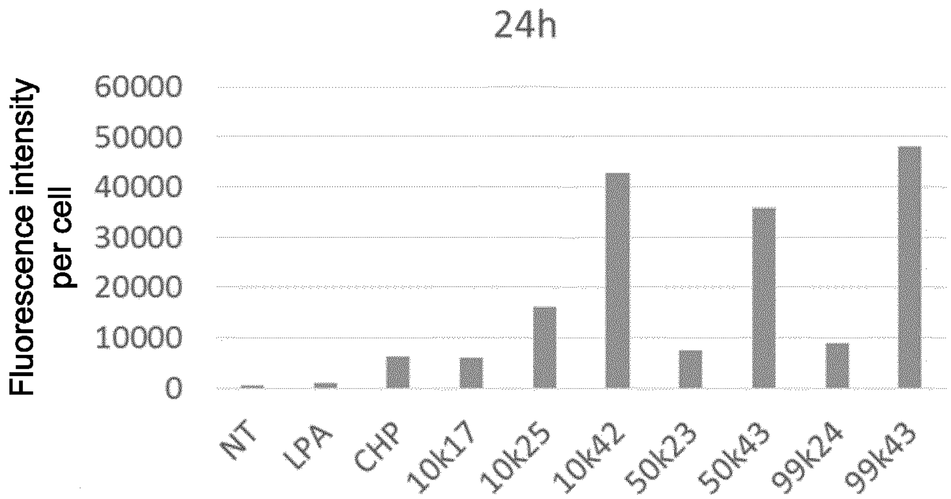


# Figure 18-2

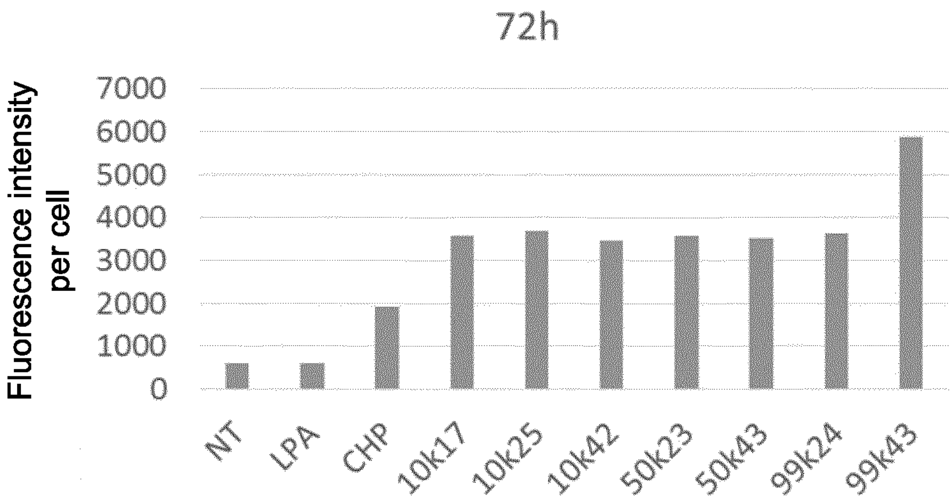

# Figure 19

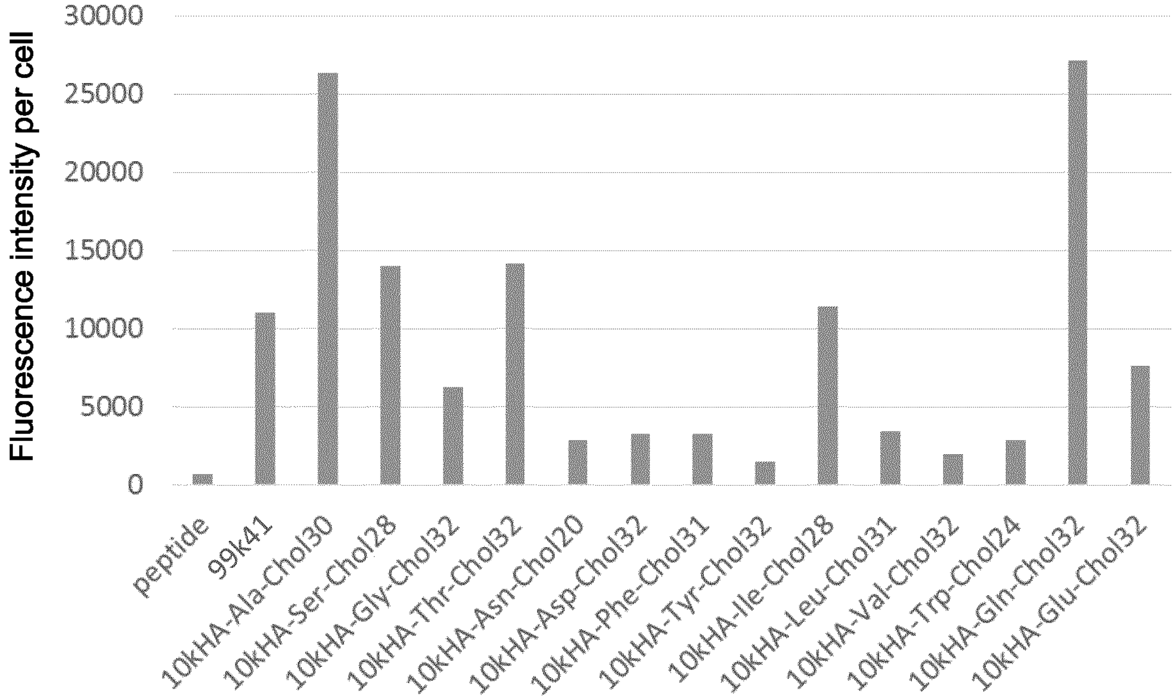
Fluorescence intensity per cell
30000
25000
20000
15000
10000
5000
0
peptide
99k41
10kHA-Ala-Chol30
10kHA-Ser-Chol28
10kHA-Gly-Chol32
10kHA-Thr-Chol32
10kHA-Asn-Chol20
10kHA-Asp-Chol32
10kHA-Phe-Chol31
10kHA-Tyr-Chol32
10kHA-Ile-Chol28
10kHA-Leu-Chol31
10kHA-Val-Chol32
10kHA-Trp-Chol24
10kHA-Gln-Chol32
10kHA-Glu-Chol32

# Figure 20-1

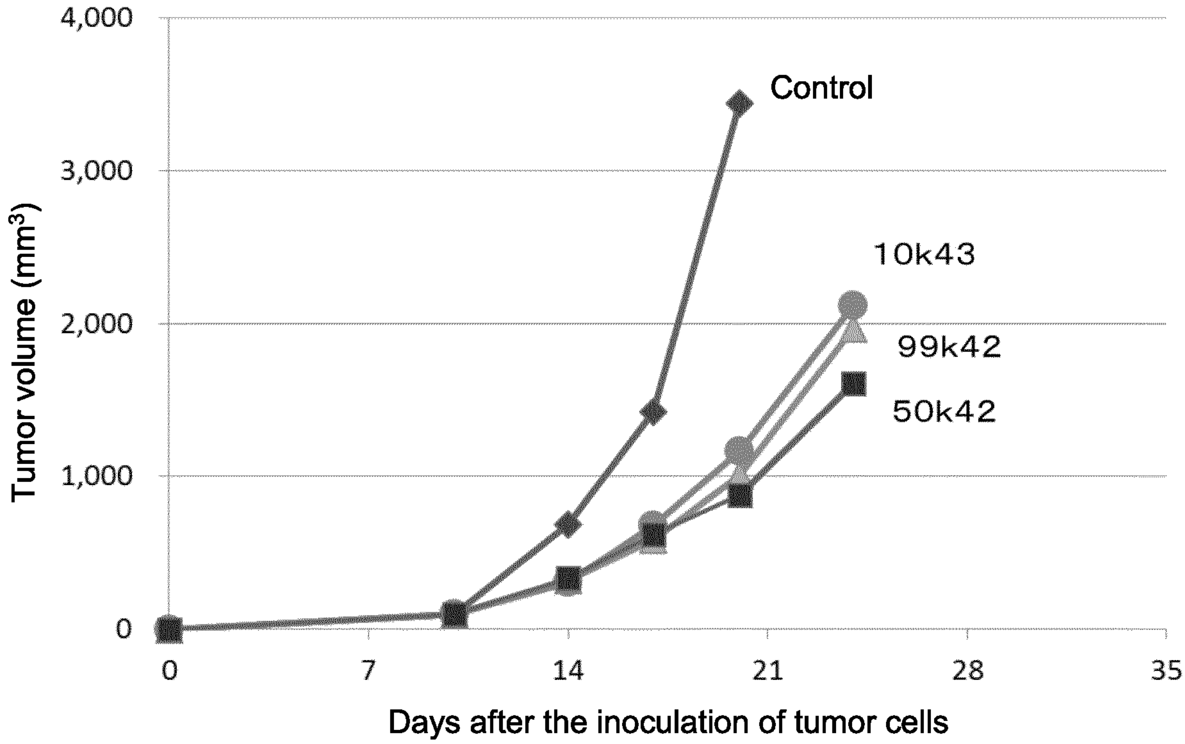
4,000
3,000
2,000
1,000
0
Tumor volume (mm3)
Control
10k43
99k42
50k42
0
7
14
21
28
35
Days after the inoculation of tumor cells

# Figure 20-2

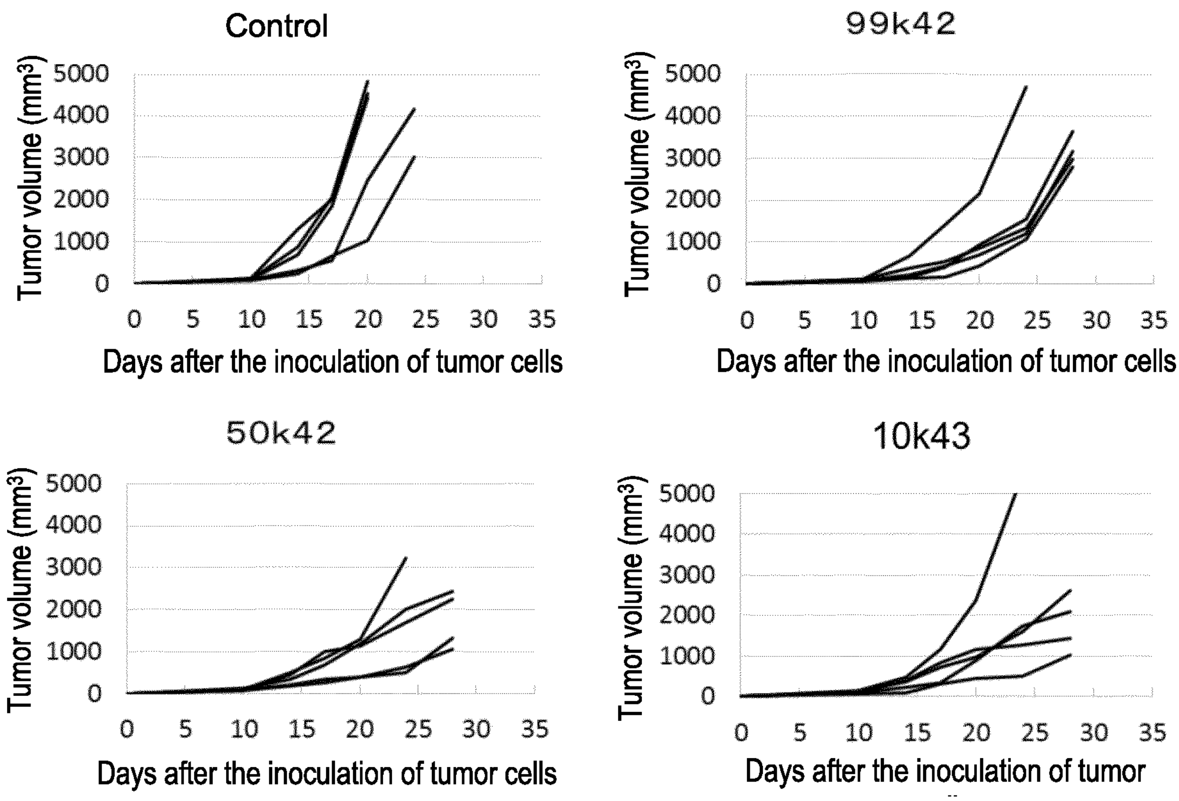
Control
Tumor volume (mm3)
5000
4000
3000
2000
1000
0
0 5 10 15 20 25 30 35
Days after the inoculation of tumor cells
99k42
Tumor volume (mm3)
5000
4000
3000
2000
1000
0
0 5 10 15 20 25 30 35
Days after the inoculation of tumor cells
50k42
Tumor volume (mm3)
5000
4000
3000
2000
1000
0
0 5 10 15 20 25 30 35
Days after the inoculation of tumor cells
10k43
Tumor volume (mm3)
5000
4000
3000
2000
1000
0
0 5 10 15 20 25 30 35
Days after the inoculation of tumor

# Figure 21-1

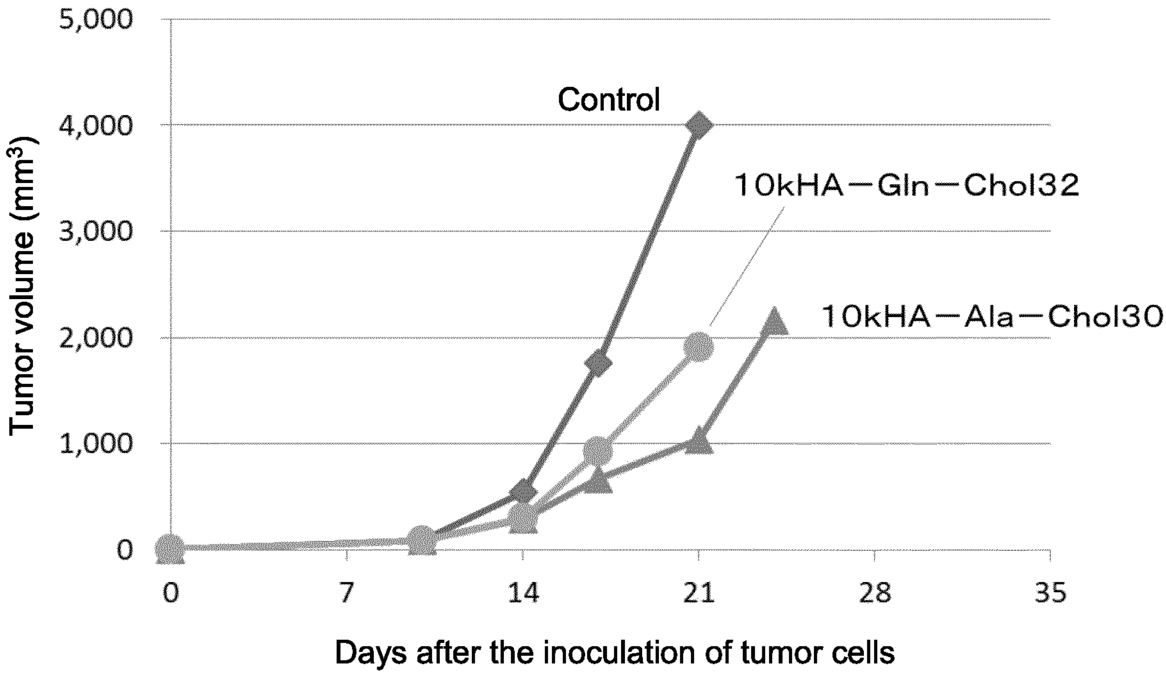
5,000
4,000
3,000
2,000
1,000
0
Tumor volume (mm3)
Control
10kHA−Gln−Chol32
10kHA−Ala−Chol30
0
7
14
21
28
35
Days after the inoculation of tumor cells

# Figure 21-2

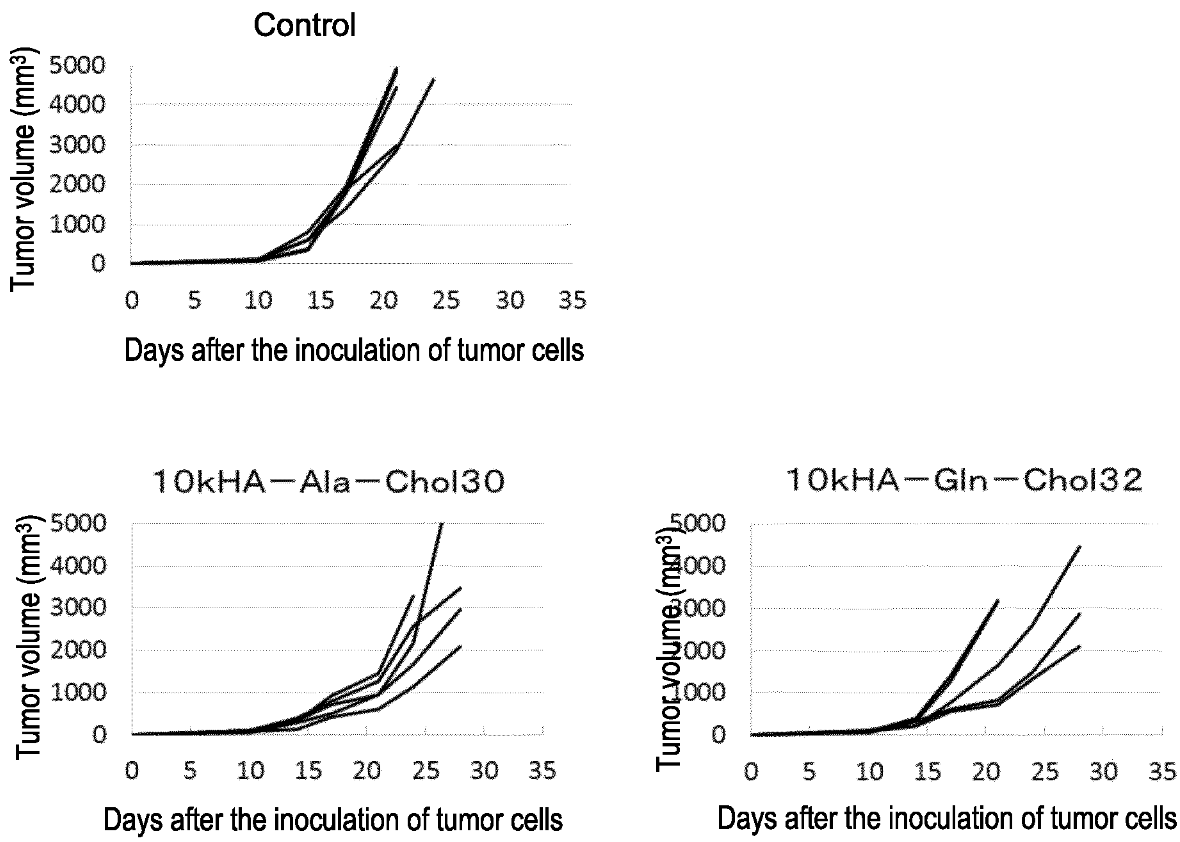
Control
Tumor volume (mm³)
5000
4000
3000
2000
1000
0
0 5 10 15 20 25 30 35
Days after the inoculation of tumor cells
10kHA−Ala−Chol30
Tumor volume (mm³)
5000
4000
3000
2000
1000
0
0 5 10 15 20 25 30 35
Days after the inoculation of tumor cells
10kHA−Gln−Chol32
Tumor volume (mm³)
5000
4000
3000
2000
1000
0
0 5 10 15 20 25 30 35
Days after the inoculation of tumor cells

# Figure 22-1

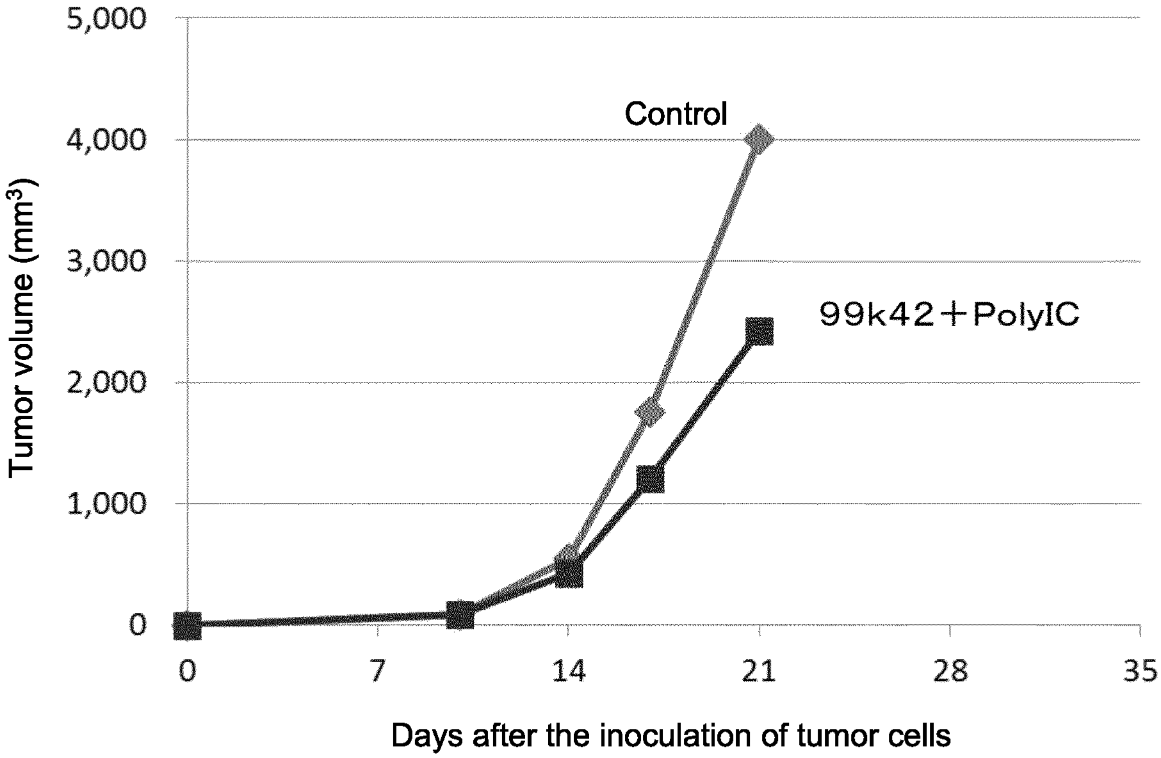


# Figure 22-2

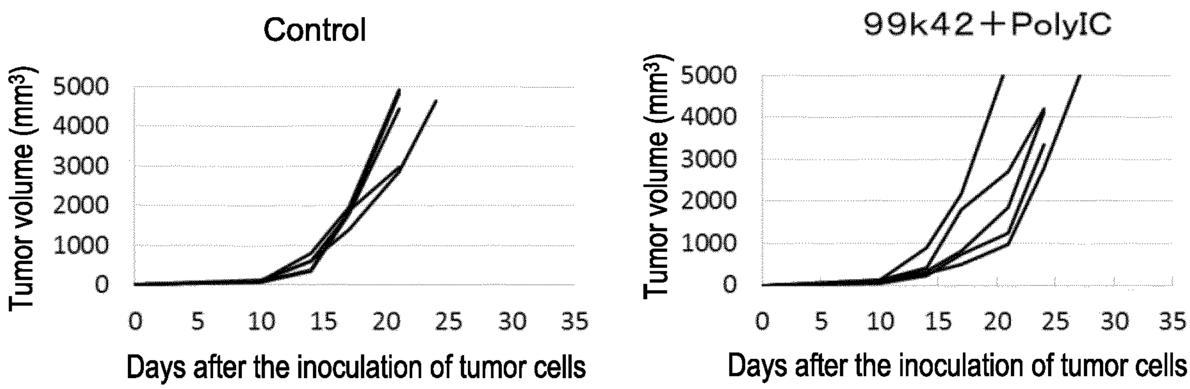

# Figure 23-1

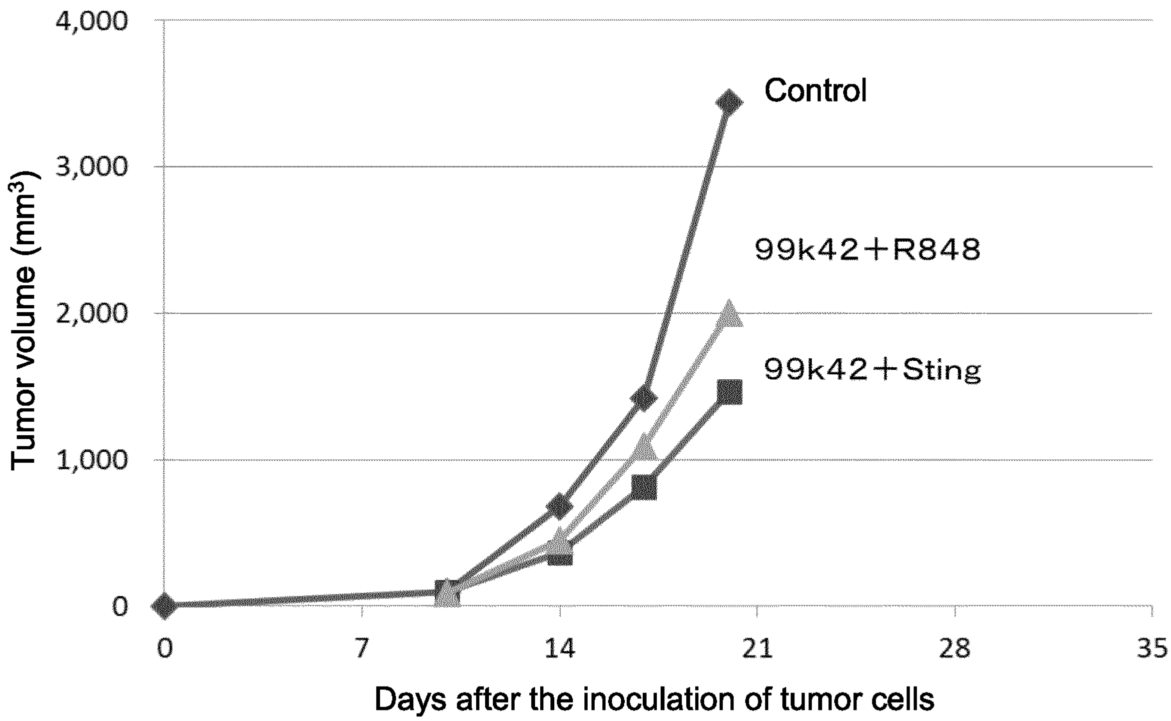
4,000
3,000
2,000
1,000
0
Tumor volume (mm3)
0
7
14
21
28
35
Days after the inoculation of tumor cells
Control
99k42+R848
99k42+Sting

# Figure 23-2

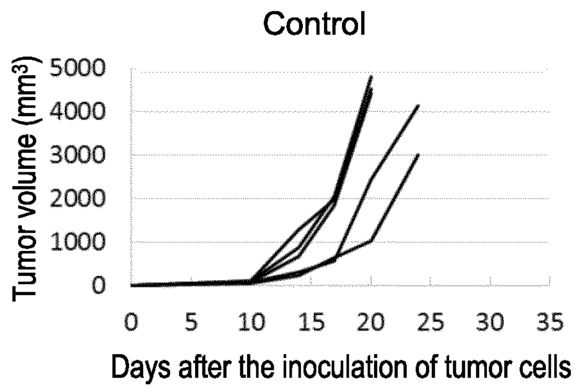


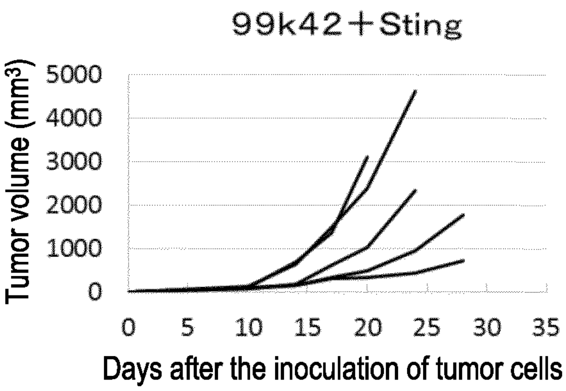


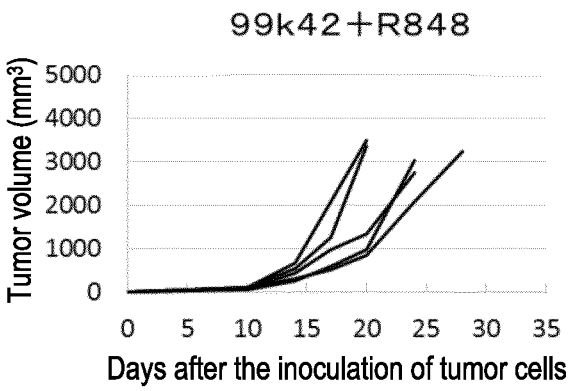


# Figure 24-1

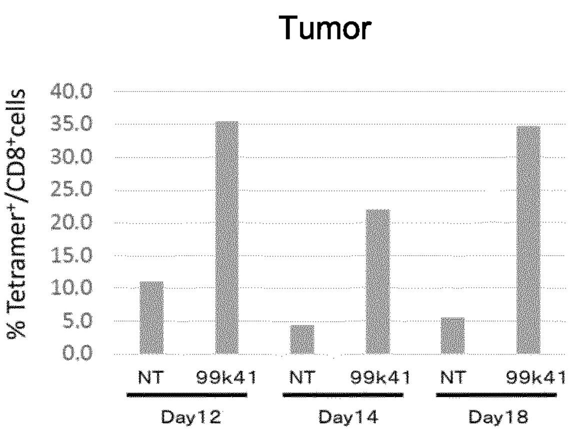


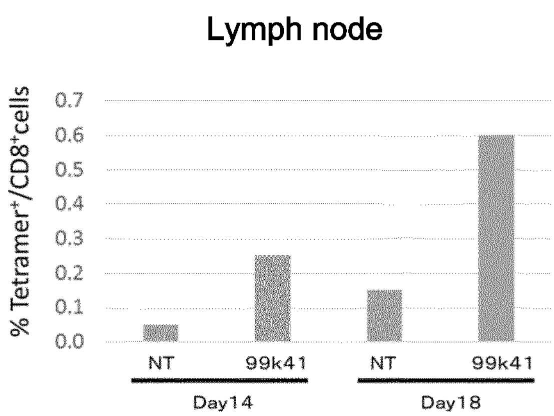

# Figure 24-2

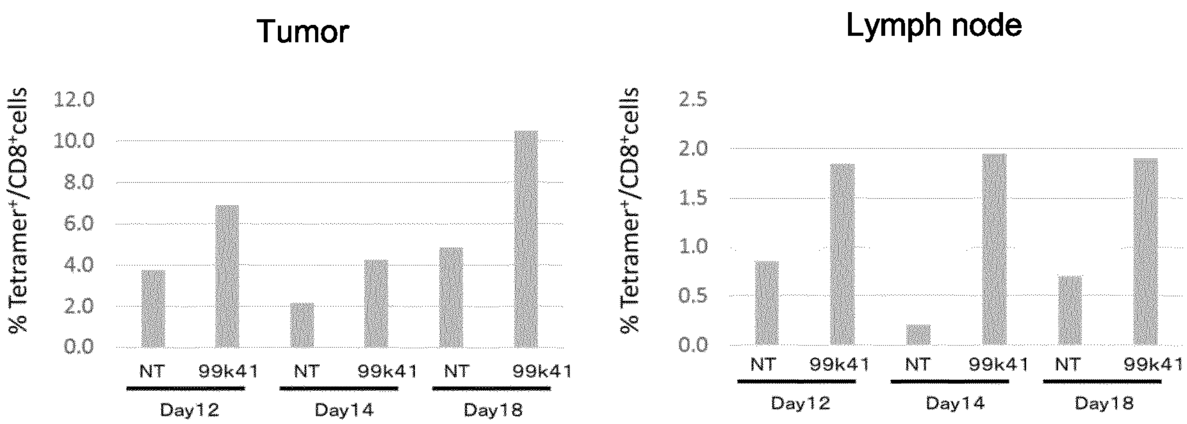
Tumor
% Tetramer+/CD8+cells
12.0
10.0
8.0
6.0
4.0
2.0
0.0
NT 99k41 NT 99k41 NT 99k41
Day12 Day14 Day18
Lymph node
% Tetramer+/CD8+cells
2.5
2.0
1.5
1.0
0.5
0.0
NT 99k41 NT 99k41 NT 99k41
Day12 Day14 Day18

## INTERNATIONAL SEARCH REPORT

International application No. PCT/JP2020/003076

A. CLASSIFICATION OF SUBJECT MATTER
A61K 39/00(2006.01)i; A61K 39/39(2006.01)i; A61P 35/00(2006.01)i; A61P 37/04(2006.01)i; A61K 47/69(2017.01)i
FI: A61K39/00 G; A61K39/39; A61P35/00; A61P37/04; A61K47/69
According to International Patent Classification (IPC) or to both national classification and IPC

B. FIELDS SEARCHED

Minimum documentation searched (classification system followed by classification symbols)
A61K39/00; A61K39/39; A61P35/00; A61P37/04; A61K47/69

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2013/031882 A1 (MIE UNIVERSITY) 07.03.2013 (2013-03-07) claims 1, 2, 12, 13, 8-10 | 1-14 |
| Y | JP 55-120520 A (PHARMACIA AB) 17.09.1980 (1980-09-17) claim 1 | 1-14 |
| Y | WO 2010/053140 A1 (TOKYO MEDICAL AND DENTAL UNIVERSITY) 14.05.2010 (2010-05-14) claims 1, 16, 18, paragraphs [0058], [0130], [0131] | 1-14 |
| Y | WO 2014/038641 A1 (CHUGAI PHARMACEUTICAL CO., LTD.) 13.03.2014 (2014-03-13) claim 1, paragraphs [0187], [0200], [0201] | 1-14 |
| Y | JP 2016-516083 A (IMMUNOVACCINE TECHNOLOGIES INC.) 02.06.2016 (2016-06-02) claims | 1-14 |

☐ Further documents are listed in the continuation of Box C.    ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April 2020 (01.04.2020) | 14 April 2020 (14.04.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/003076

| Patent Documents referred in the Report | Publication Date | Patent Family | Publication Date |
|---|---|---|---|
| WO 2013/031882 A1 | 07 Mar. 2013 | US 2014/0322344 A1 claims 1, 2, 12, 13, 8-10<br>EP 2752198 A1<br>CN 103957930 A | |
| JP 55-120520 A | 17 Sep. 1980 | EP 14995 A2 claim 1 | |
| WO 2010/053140 A1 | 14 May 2010 | US 2011/0212901 A1 claims 1, 16, 18, paragraphs [0171], [0353], [0354]<br>EP 2360188 A1 | |
| WO 2014/038641 A1 | 13 Mar. 2014 | US 2015/0231268 A1 claim 1, paragraphs [0436], [0449], [0450]<br>EP 2894173 A1<br>CN 104603156 A<br>KR 10-2015-0048238 A | |
| JP 2016-516083 A | 02 Jun. 2016 | US 2016/0067335 A1 claims<br>WO 2014/153636 A1<br>EP 2978450 A1<br>CN 105324128 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 1998009650 A
- WO 2013031882 A
- WO 2015050158 A
- WO 2010053140 A
- WO 2014038641 A
- WO 2010053140 A1


### Non-patent literature cited in the description


- *ACS Nano,* 2014, vol. 8, 9209-9218
- **SEIICHI NAKAHAMA et al.** Essential Polymer Science. Kodansha Ltd,